# EUROPÄISCHE PATENTSCHRIFT

(11) **EP 2 225 227 B1**
(45) Veröffentlichungstag und Bekanntmachung des Hinweises auf die Patenterteilung: **26.08.2015**
(21) Anmeldenummer: 08851085.4
(22) Anmeldetag: 21.11.2008
(51) Int. Cl.: C07D 403/14, C07D 471/04, C07D 471/10, C07D 471/20, C07D 498/10, A61K 31/517, A61K 31/527, A61K 31/537, A61P 3/10, A61P 9/00, A61P 17/00, A61P 19/00, A61P 25/06

(54) **1',3'-Dihydrospiro[imidazolidin-4,2'-inden]-2,5-dione und 1,3-dihydrospiro[inden-2,3']-pyrrole als CGRP Antagonisten**
1',3'-Dihydrospiro[imidazolidin-4,2'-inden]-2,5-diones and 1,3-dihydrospiro[inden-2,3']-pyrroles as CGRP antagonists
1',3'-Dihydrospiro[imidazolidin-4,2'-inden]-2,5-diones et 1,3-dihydrospiro[inden-2,3']-pyrroles en tant qu'antagonistes de CGRP

(30) Priorität: 22.11.2007 EP 07121350
(43) Veröffentlichungstag der Anmeldung: 08.09.2010
(60) Teilanmeldung: 10172021.7
(73) Patentinhaber: Boehringer Ingelheim International GmbH, 55216 Ingelheim am Rhein (DE)
(72) Erfinder: GOTTSCHLING, Dirk, 55216 Ingelheim am Rhein (DE); DAHMANN, Georg, 55216 Ingelheim am Rhein (DE); DOODS, Henri, 55216 Ingelheim am Rhein (DE); HEIMANN, Annekatrin, 55216 Ingelheim am Rhein (DE); MUELLER, Stephan Georg, 55216 Ingelheim am Rhein (DE); RUDOLF, Klaus, 55216 Ingelheim am Rhein (DE); SCHAENZLE, Gerhard, 55216 Ingelheim am Rhein (DE); STENKAMP, Dirk, 55216 Ingelheim am Rhein (DE)
(74) Vertreter: Simon, Elke Anna Maria
(86) Internationale Anmeldenummer: PCT/EP2008/065962
(87) Internationale Veröffentlichungsnummer: WO 2009/065920

(56) Entgegenhaltungen:
- WO-A-03/040128
- WO-A-2007/000340

## Beschreibung

Gegenstand der vorliegenden Erfindung sind neue CGRP-Antagonisten der allgemeinen Formel **I** 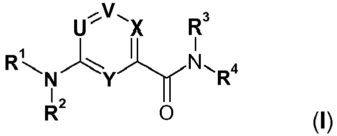 in der **U, V, X, Y, R¹, R², R³** und **R⁴** wie nachstehend erwähnt definiert sind, deren Tautomere, deren Isomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen, diese Verbindungen enthaltende Arzneimittel, deren Verwendung und Verfahren zu ihrer Herstellung.

In der obigen allgemeinen Formel **I** bedeuten in einer ersten Ausführungsform
- **R¹**: eine Gruppe der allgemeinen Formeln **IIa** oder **IIb** 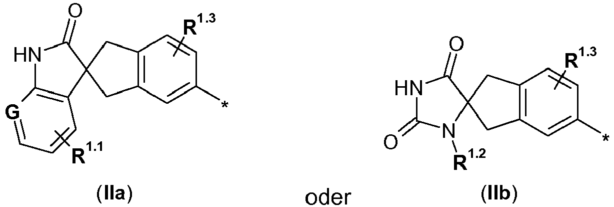 und
- **R²**: H oder C₁₋₃-Alkyl,
- **G**: C-**R^{1.1}** oder N,
- **R^{1.1}**: unabhängig voneinander
(a) H,
(b) Halogen, C₁₋₃-Alkyl, -OH, -CN, C₁₋₃-Alkyl-O-, -C(O)-O-C₁₋₃-Alkyl, C₂₋₄-Alkenyl, - C₂₋₄-Alkinyl, C₁₋₃-Alkyl-S-, Cyclopropyl, -NH₂, -COOH, -NH-C(O)-O-C₁₋₃-Alkyl, -NH-C(O)-C₁₋₃-Alkyl,
(c) eine C₁₋₃-Alkylgruppe oder C₁₋₃-Alkyl-O-gruppe, in der jede Methylengruppe mit bis zu zwei Fluoratomen und jede Methylgruppe mit bis zu drei Fluoratomen substituiert ist,
- **R^{1.2}**: unabhängig voneinander
(a) H oder
(b) C₁₋₃-Alkyl,
- **R^{1.3}**: (a) H,
(b) F, -CN, C₁₋₃-Alkyl, -CO₂-**R^{13.1}** oder
(c) eine C₁₋₃-Alkylgruppe, in der jede Methylengruppe mit bis zu zwei Fluoratomen und jede Methylgruppe mit bis zu drei Fluoratomen substituiert sein kann,
- **R^{1.3.1}**: (a) H,
(b) C₁₋₆-Alkyl,
- **R³**: (a) H,
(b) C₁₋₃-Alkyl oder
(c) eine C₁₋₃-Alkylgruppe, in der jede Methylengruppe mit bis zu zwei Fluoratomen und jede Methylgruppe mit bis zu drei Fluoratomen substituiert ist, und
- **R⁴**: H, Cyclopropyl oder eine Gruppe ausgewählt aus 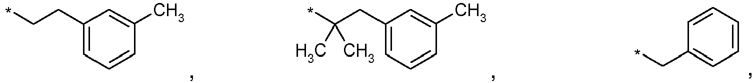 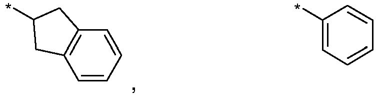 oder
**R³** und **R⁴** zusammen mit dem Stickstoffatom, an das sie gebunden sind, eine Gruppe ausgewählt aus 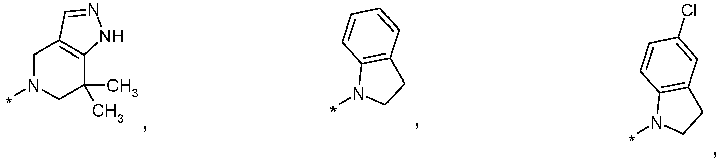 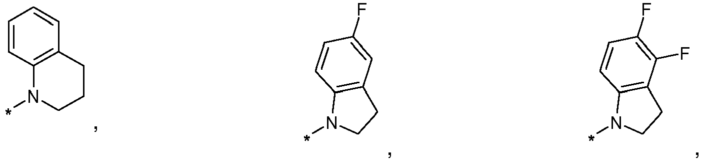 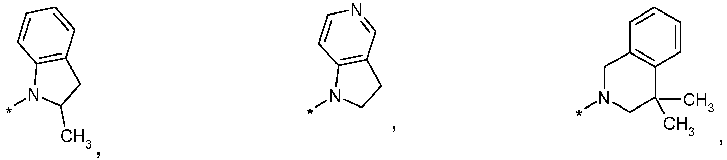 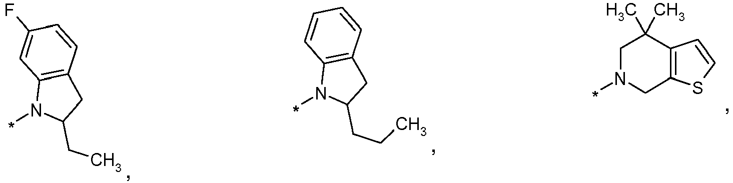 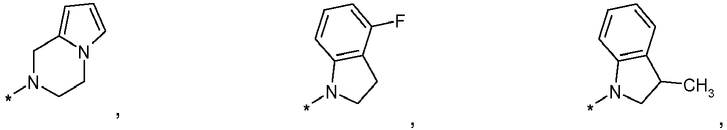 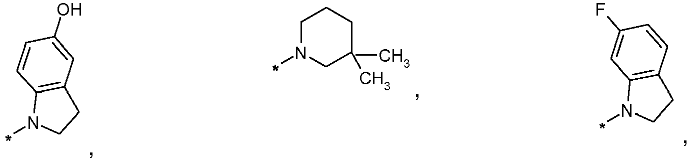 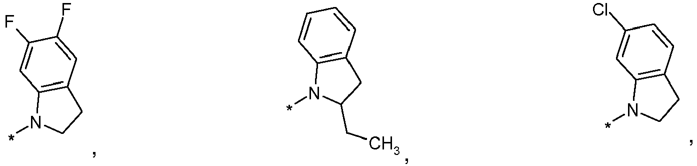 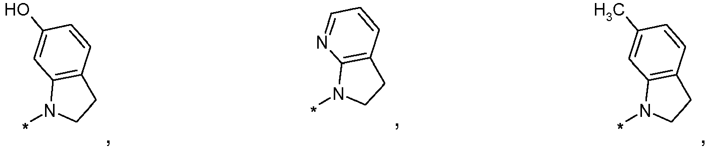 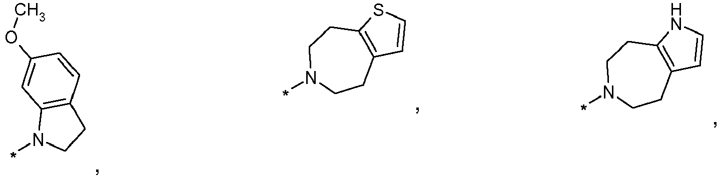 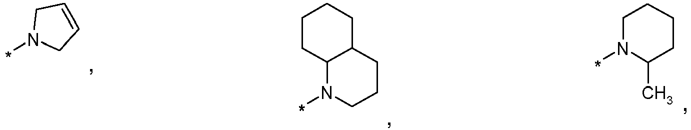 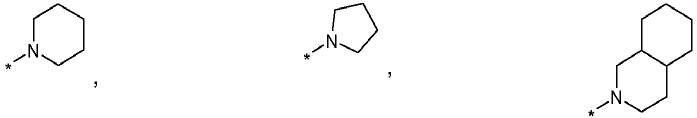 der Ring 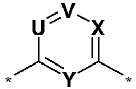 eine Gruppe ausgewählt aus 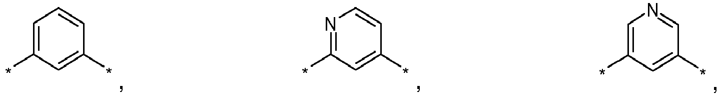 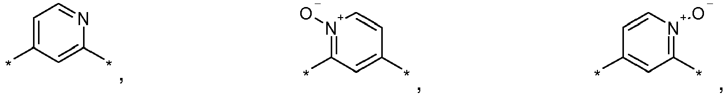 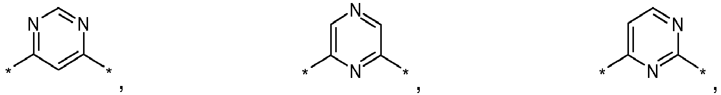 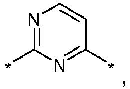 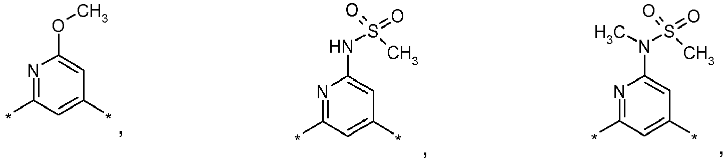 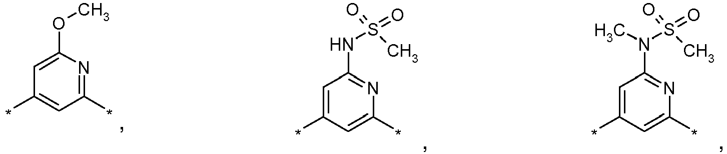 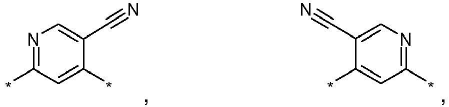 deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine zweiteAusführungsform der vorliegenden Erfindung besteht in den Verbindungen der obigen allgemeinen Formel **I**, in denen **U, V, X, Y, R³** und **R⁴** wie voranstehend unter der ersten Ausführungsform erwähnt definiert sind und
- **R¹**: eine Gruppe ausgewählt aus 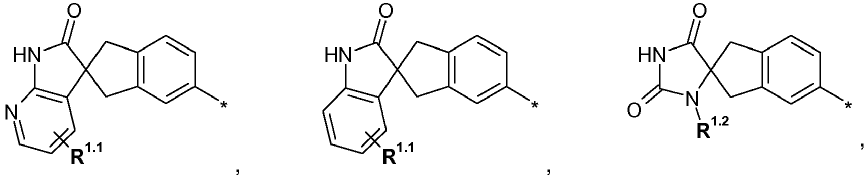
- **R^{1.1}**: (a) H,
(b) Halogen, C₁₋₃-Alkyl, -OH, -CN, -O-C₁₋₃-Alkyl, -C(o)-O-C₁₋₃-Alkyl, C₂₋₄-Alkenyl, C₂₋₄-Alkinyl, C₁₋₃-Alkyl-S-, -NH₂,
(c) eine C₁₋₃-Alkylgruppe oder C₁₋₃-Alkyl-O-gruppe, in der jede Methylengruppe mit bis zu zwei Fluoratomen und jede Methylgruppe mit bis zu drei Fluoratomen substituiert ist,
- **R^{1.2}**: H oder CH₃ und
- **R²**: H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine dritte Ausführungsform der vorliegenden Erfindung besteht in den Verbindungen der obigen allgemeinen Formel **I**, in denen **U, V, X, Y, R³** und **R⁴** wie voranstehend unter der ersten Ausführungsform erwähnt definiert sind und
- **R¹**: eine Gruppe ausgewählt aus 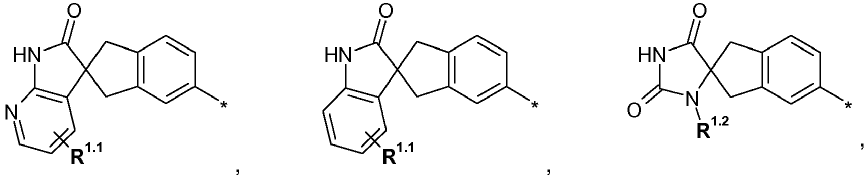
- **R^{1.1}**: (a) F, CH₃, -OH, -O-CH₃ oder
(b) CF₃ und
- **R²**: H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine vierte Ausführungsform der vorliegenden Erfindung besteht in den Verbindungen der obigen allgemeinen Formel **I**, in denen **U, V, X, Y, R³** und **R⁴** wie voranstehend unter der ersten Ausführungsform erwähnt definiert sind und
- **R¹**: eine Gruppe ausgewählt aus 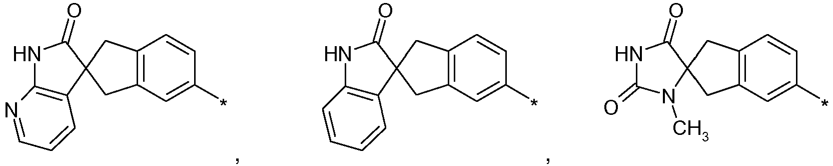 und
- **R²**: H bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Eine fünfte Ausführungsform der vorliegenden Erfindung besteht in den Verbindungen der allgemeinen Formel **I**, in der
- **R¹**: eine Gruppe ausgewählt aus 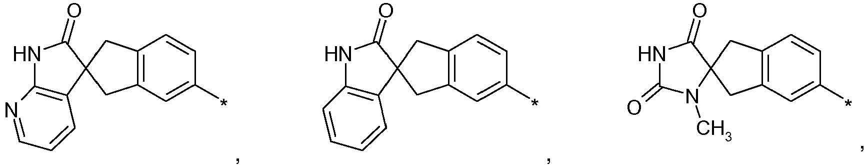
- **R²**: H,
- **R³**: (a) H,
(d) C₁₋₃-Alkyl oder
(e) eine C₁₋₃-Alkylgruppe, in der jede Methylengruppe mit bis zu zwei Fluoratomen und jede Methylgruppe mit bis zu drei Fluoratomen substituiert ist, und
- **R⁴**: H, Cyclopropyl oder eine Gruppe ausgewählt aus 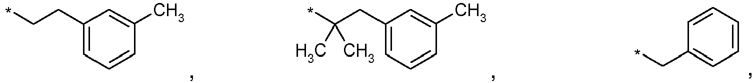 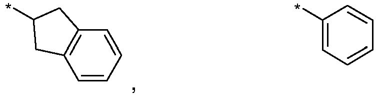
oder **R³** und **R⁴** zusammen mit dem Stickstoffatom, an das sie gebunden sind, eine Gruppe ausgewählt aus 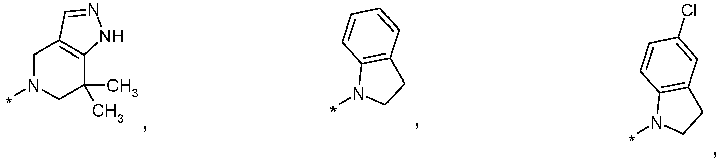 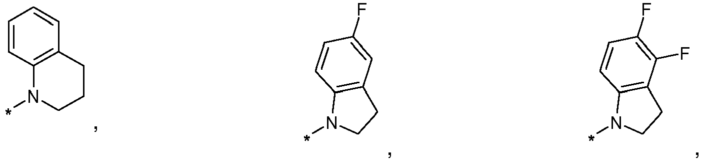 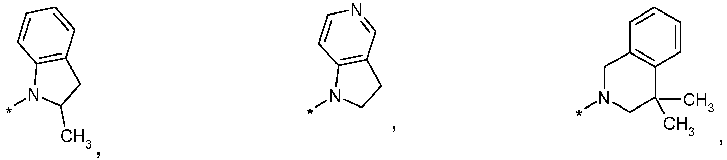 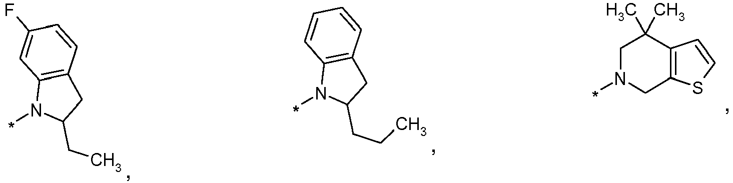 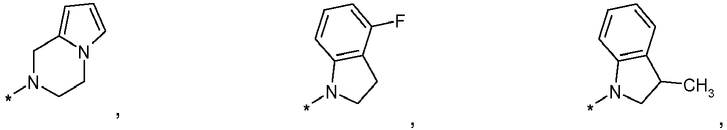 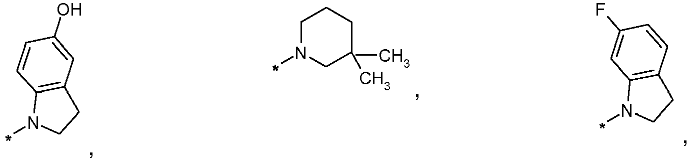 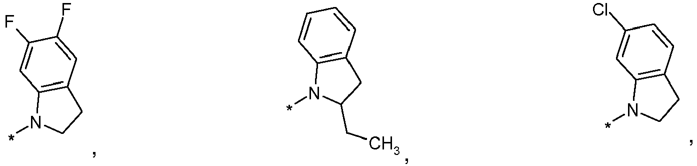 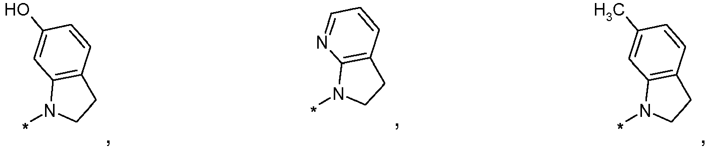 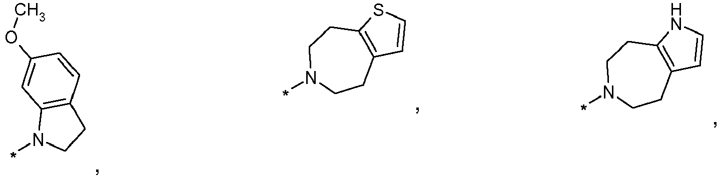 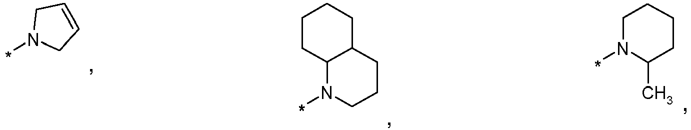 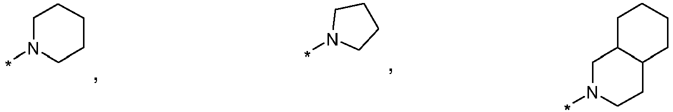 und bedeutet,
der Ring 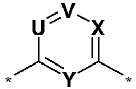 eine Gruppe ausgewählt aus 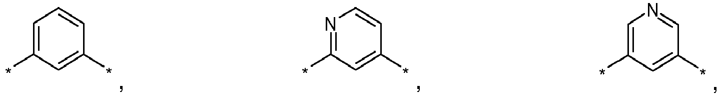 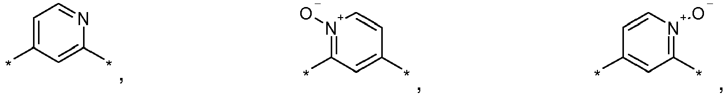 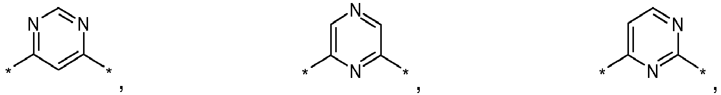 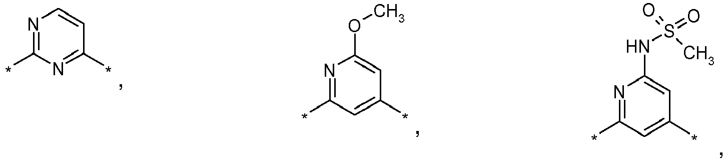 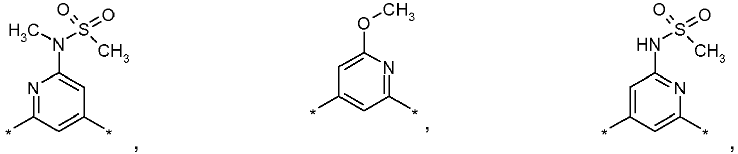 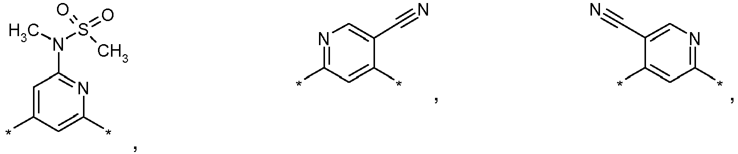 deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

Als ganz besonders bevorzugte Verbindungen der obigen allgemeinen Formel **I** seien beispielsweise folgende Verbindungen genannt:

| **Nr.** | **Struktur** |
|---|---|
| (1) | 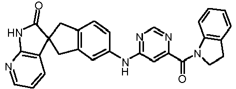 |
| | |
| | |
| | |
| (2) | 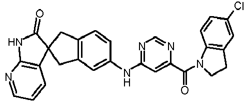 |
| (3) | 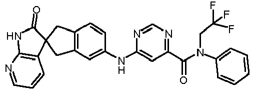 |
| (4) | 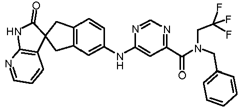 |
| (5) | 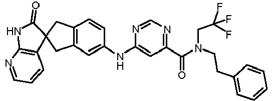 |
| (6) | 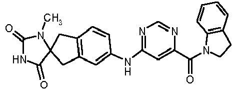 |
| (7) | 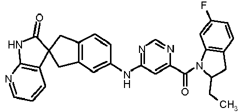 |
| (8) | 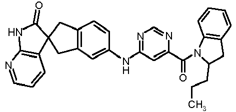 |
| (9) | 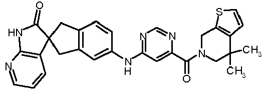 |
| (10) | 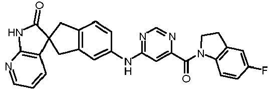 |
| (11) | 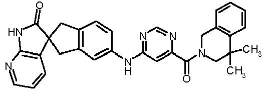 |
| (12) | 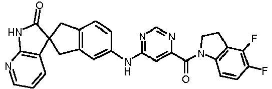 |
| (13) | 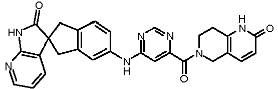 |
| (14) | 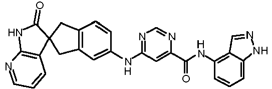 |
| (15) | 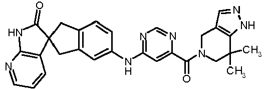 |
| (16) | 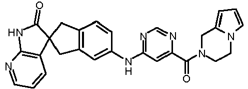 |
| (17) | 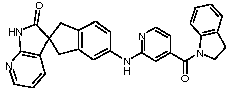 |
| (18) | 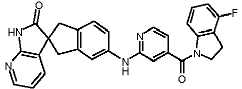 |
| (19) | 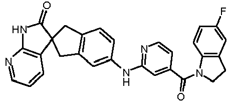 |
| (20) | 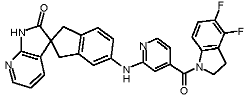 |
| (21) | 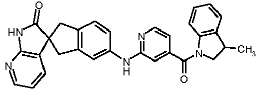 |
| (22) | 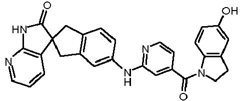 |
| (23) | 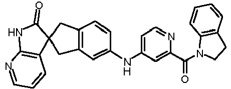 |
| (24) | 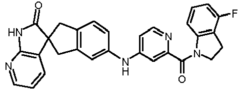 |
| (25) | 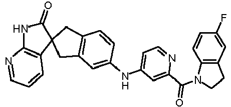 |
| (26) | 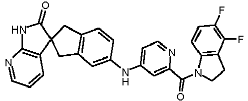 |
| (27) | 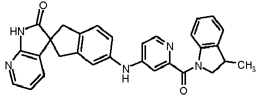 |
| (28) | 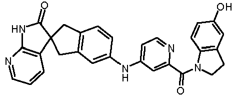 |
| (29) | 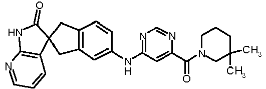 |
| (30) | 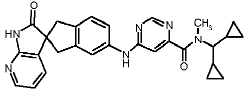 |
| (31) | 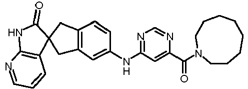 |
| (32) | 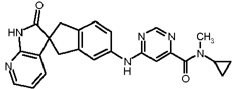 |
| (33) | 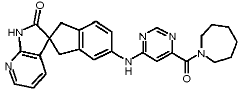 |
| | |
| (34) | 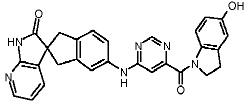 |
| (35) | 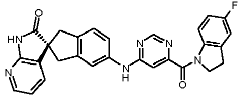 |
| (36) | 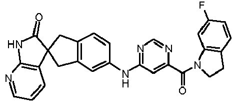 |
| (37) | 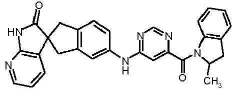 |
| | |
| (38) | 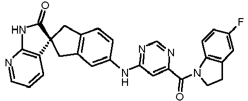 |
| (39) | 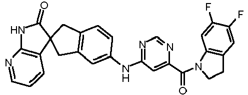 |
| (40) | 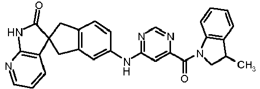 |
| (41) | 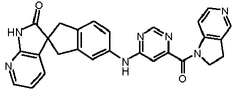 |
| (42) | 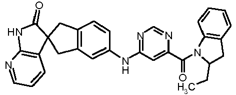 |
| (43) | 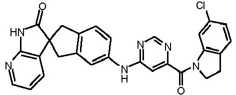 |
| (44) | 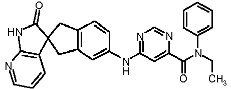 |
| (45) | 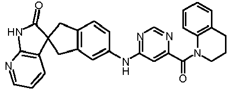 |
| (46) | 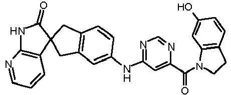 |
| (47) | 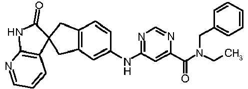 |
| (48) | 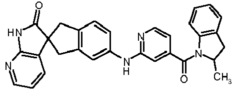 |
| (49) | 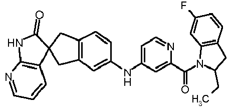 |
| (50) | 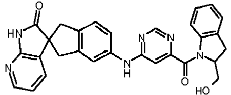 |
| (51) | 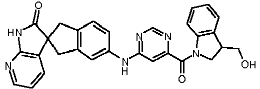 |
| (52) | 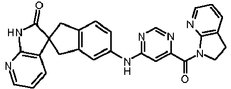 |
| (53) | 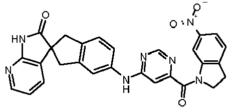 |
| (54) | 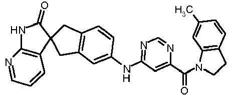 |
| (55) | 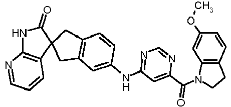 |
| (56) | 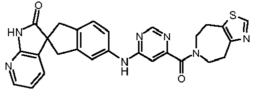 |
| (57) | 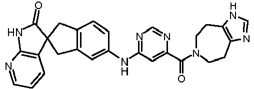 |
| (58) | 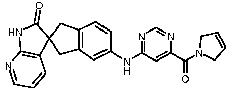 |
| (59) | 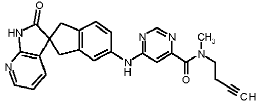 |
| (60) | 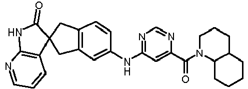 |
| (61) | 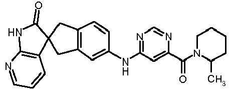 |
| (62) | 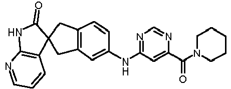 |
| (63) | 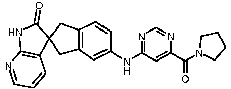 |
| | |
| (64) | 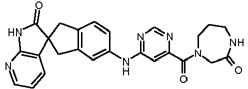 |
| (65) | 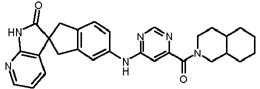 |

deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

### VERWENDETE BEGRIFFE UND DEFINITIONEN

Die vorliegende Beschreibung der Erfindung soll in Übereinstimmung mit den Gesetzmäßigkeiten und Regeln der chemischen Bindungen aufgefasst werden.

Die Verbindungen, die von dieser Erfindung umfasst sind, sind jene, die auch chemisch stabil sind.

Soweit nicht anders angegeben, sind alle Substituenten voneinander unabhängig. Sollten an einer Gruppe z. B. mehrere C₁₋₄-Alkylgruppen als Substituenten sein, so könnte, im Fall von drei Substituenten C₁₋₄-Alkyl unabhängig voneinander einmal Methyl, einmal Ethyl und einmal *n*-Propyl bedeuten.

Im Rahmen dieser Anmeldung können bei der Definition von möglichen Substituenten, diese auch in Form einer Strukturformel dargestellt werden. Dabei wird, falls vorhanden, ein Stern (*) in der Strukturformel des Substituenten als der Verknüpfungspunkt zum Rest des Moleküls verstanden. Beispielsweise wird ein Phenyl-Rest wie folgt dargestellt:

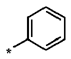

Weiterhin wird das auf den Verknüpfungspunkt folgende Atom des Substituenten als das Atom mit der Positionsnummer 1 verstanden.

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen, unter dem Begriff "C₁₋₄-Alkyl" werden verzweigte und unverzweigte Alkylgruppen mit 1 bis 4 Kohlenstoffatomen und unter dem Begriff "C₁₋₆-Alkyl" werden verzweigte und unverzweigte Alkylgruppen mit 1 bis 6 Kohlenstoffatomen verstanden. Beispielsweise werden hierfür genannt: Methyl, Ethyl, *n*-Propyl, *iso*-Propyl, *n*-Butyl, *iso*-Butyl, *sec*-Butyl, *tert*-Butyl, Pentyl, Neopentyl oder *n*-Hexyl. Gegebenenfalls werden für vorstehend genannten Gruppen auch die Abkürzungen Me, Et, *n*-Pr, *i*-Pr, *n*-Bu, *i*-Bu, *t*-Bu, etc. verwendet. Sofern nicht anders beschrieben, umfassen die Definitionen Propyl und Butyl alle denkbaren isomeren Formen der jeweiligen Reste. So umfasst beispielsweise Propyl *n*-Propyl und *iso*-Propyl, Butyl umfasst *iso*-Butyl, *sec*-Butyl und *tert*-Butyl etc.

Unter dem Begriff "C₁₋₆-Alkylen" (auch soweit sie Bestandteil anderer Reste sind) werden verzweigte und unverzweigte Alkylengruppen mit 1 bis 6 Kohlenstoffatomen und unter dem Begriff "C₁₋₃-Alkylen" werden verzweigte und unverzweigte Alkylengruppen mit 1 bis 3 Kohlenstoffatomen verstanden Beispielsweise werden hierfür genannt: Methylen, Ethylen, Propylen, 1-Methylethylen, Butylen, 1-Methylpropylen, 1,1-Dimethylethylen, 1,2-Dimethylethylen, Pentylen, 1,1-Dimethylpropylen, 2,2-Dimethylpropylen, 1,2-Dimethylpropylen, 1,3-Dimethylpropylen oder Hexylen. Sofern nicht anders beschrieben, umfasst die Definition Propylen alle denkbaren isomeren Formen der jeweiligen Reste gleicher Kohlenstoffanzahl. So umfasst beispielsweise Propyl auch 1-Methylethylen und Butylen umfasst 1-Methylpropylen, 1,1-Dimethylethylen, 1,2-Dimethylethylen. Die Definition für C₀-Alkylen bedeutet eine Bindung.

Unter dem Begriff "C₂₋₆-Alkenyl" (auch soweit sie Bestandteil anderer Reste sind) werden verzweigte und unverzweigte Alkenylgruppen mit 2 bis 6 Kohlenstoffatomen und unter dem Begriff "C₂₋₄-Alkenyl" verzweigte und unverzweigte Alkenylgruppen mit 2 bis 4 Kohlenstoffatomen verstanden, soweit sie mindestens eine Doppelbindung aufweisen. Bevorzugt sind Alkenylgruppen mit 2 bis 4 Kohlenstoffatomen. Beispielsweise werden hierfür genannt: Ethenyl oder Vinyl, Propenyl, Butenyl, Pentenyl, oder Hexenyl. Sofern nicht anders beschrieben, umfassen die Definitionen Propenyl, Butenyl, Pentenyl und Hexenyl alle denkbaren isomeren Formen der jeweiligen Reste. So umfasst beispielsweise Propenyl 1-Propenyl und 2-Propenyl, Butenyl umfasst 1-, 2- und 3-Butenyl, 1-Methyl-1-propenyl, 1-Methyl-2-propenyl etc..

Unter dem Begriff "C₂₋₆-Alkinyl" (auch soweit sie Bestandteil anderer Reste sind) werden verzweigte und unverzweigte Alkinylgruppen mit 2 bis 6 Kohlenstoffatomen und unter dem Begriff "C₂₋₄-Alkinyl" verzweigte und unverzweigte Alkinylgruppen mit 2 bis 4 Kohlenstoffatomen verstanden, soweit sie mindestens eine Dreifachbindung aufweisen. Beispielsweise werden hierfür genannt: Ethinyl, Propinyl, Butinyl, Pentinyl, oder Hexinyl. Sofern nicht anders beschrieben, umfassen die Definitionen Propinyl, Butinyl, Pentinyl und Hexinyl alle denkbaren isomeren Formen der jeweiligen Reste. So umfasst beispielsweise Propinyl 1-Propinyl und 2-Propinyl, Butinyl umfasst 1-, 2- und 3-Butinyl, 1-Methyl-1-propinyl, 1-Methyl-2-propinyl etc..

Unter dem Begriff "C₃₋₆-Cycloalkyl" (auch soweit sie Bestandteil anderer Reste sind) werden cyclische Alkylgruppen mit 3 bis 6 Kohlenstoffatomen, unter dem Begriff "C₅₋₆-Cycloalkyl" werden cyclische Alkylgruppen mit 5 bis 6 Kohlenstoffatomen und unter dem Begriff "C₅₋₇-Cycloalkyl" werden cyclische Alkylgruppen mit 5 bis 7 Kohlenstoffatomen verstanden. Beispielsweise werden hierfür genannt: Cyclopropyl, Cyclobutyl, Cyclopentyl, Cyclohexyl oder Cycloheptyl. Soweit nicht anders beschrieben, können die cyclischen Alkylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "C₅₋₆-Cycloalkenyl" (auch soweit sie Bestandteil anderer Reste sind) werden cyclische Alkenylgruppen mit 5 oder 6 Kohlenstoffatomen verstanden, die eine ungesättigte Bindung enthalten. Beispielsweise werden hierfür genannt: Cyclopentenyl oder Cyclohexenyl. Soweit nicht anders beschrieben, können die cyclischen Alkenylgruppen substituiert sein mit einem oder mehreren Resten ausgewählt aus der Gruppe bestehend aus Methyl, Ethyl, *iso*-Propyl, *tert*-Butyl, Hydroxy, Fluor, Chlor, Brom und Jod.

Unter dem Begriff "Halogen" werden Fluor-, Chlor-, Brom- oder lodatome verstanden.

Verbindungen der allgemeinen Formel **I** können Säuregruppen besitzen, hauptsächlich Carboxylgruppen, und/oder basische Gruppen wie z.B. Aminofunktionen. Verbindungen der allgemeinen Formel **I** können deshalb als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure, Zitronensäure oder als Salze mit pharmazeutisch verwendbaren Basen wie Alkali- oder Erdalkalimetallhydroxiden, beispielsweise Natriumhydroxid oder Kaliumhydroxid, oder Carbonaten, Ammoniak, Zink- oder Ammoniumhydroxiden oder organischen Aminen wie z.B. Diethylamin, Triethylamin, Ethanolamin, Diethanolamin, Triethanolamin, Cyclohexylamin, Dicyclohexylamin u.a. vorliegen.

Die erfindungsgemäßen Verbindungen können als Racemate vorliegen, sofern sie nur ein Chiralitätselement besitzen, sie können aber auch als reine Enantiomere, d.h. in (R)- oder (S)-Form gewonnen werden.

Verbindungen mit einer Kohlenstoff-Doppelbindung können sowohl in der E- als auch Z-Form vorliegen.

Falls eine Verbindung in verschiedenen tautomeren Formen vorliegen kann, so ist die dargestellte Verbindung nicht auf eine tautomere Form beschränkt, sondern umfasst alle tautomeren Formen. Dies gilt insbesondere auch bei stickstoffhaltigen Heteroarylen:

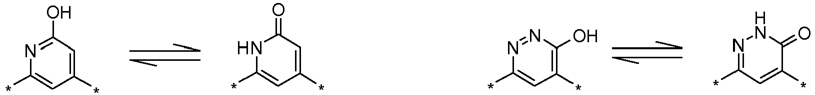

Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel **I** vorhanden ist, sowie die einzelnen optisch aktiven Enatiomeren, aus denen sich die erwähnten Racemate zusammensetzen. Gegenstand der Erfindung sind die jeweiligen Verbindungen gegebenenfalls in Form der einzelnen optischen Isomeren, Mischungen der einzelnen Enantiomeren oder Racemate, in Form der Tautomere sowie in Form der freien Basen oder der entsprechenden Säure-additionssalze mit pharmakologisch unbedenklichen.

Sogenannte Prodrugs von Verbindungen der allgemeinen Formel **I** sind ebenfalls Gegenstand dieser Erfindung. Als Prodrug wird jedes Molekül bezeichnet, das nach Applikation an Säugetieren das aktive Prinzip der allgemeinen Formel **I** in-vivo freisetzt. Das Prodrug kann per se keine oder nur geringe pharmakologische Aktivität haben, es setzt allerdings nach Verabreichung in-vivo das aktive Prinzip der allgemeinen Formel **I** frei und dieses weist die beschriebene Aktivität auf. Prodrugs für Verbindungen der allgemeinen Formel **I** können hergestellt werden, indem geeignete funktionelle Gruppen in der Verbindung der allgemeinen Formel **I** so modifiziert werden, wie es einem Fachmann in diesem Gebiet bekannt ist. (H. Bundgaard (Editor), Design of Prodrugs. (1986), Elsevier)

Diese Erfindung umfasst auch jene Metaboliten, die sich aus der Verbindungen der allgemeinen Formel **I** ableiten. Unter Metaboliten versteht man in diesem Zusammenhang solche Verbindungen, die nach Applikation in-vivo aus der Verbindung der allgemeinen Formel **I** entstehen. Als Beispiel für Metaboliten werden genannt:
- Methylgruppen der Verbindung der allgemeinen Formel **I** können in die entsprechenden Hydroxymethyl-Gruppen überführt werden. (-CH₃ -> -CH₂OH)
- Alkoxy-Gruppen der Verbindung der allgemeinen Formel **I** können in die entsprechenden Hydroxyl-Gruppen überführt werden. (-OR -> -OH)
- Sekundäre Amine der Verbindung der allgemeinen Formel **I** können in die entsprechenden primären Amine überführt werden. (-NR₁R₂ -> -NHR₁ oder -NHR₂)
- Stickstoff-Atome der Verbindung der allgemeinen Formel **I** können in die entsprechenden Stickstoff-Oxide überführt werden. (=N- -> =N⁺-(O⁻)-)

### HERSTELLVERFAHREN

Gegenstand der Erfindung ist weiterhin ein Verfahren zur Herstellung der Verbindungen der allgemeinen Formel **I**, in der die Substituenten **U, V, X, Y, R¹**, **R²**, **R³** und **R⁴** wie voranstehend erwähnt definiert sind.

Einige Methoden zur Herstellung der erfindungsgemäßen Verbindungen der allgemeinen Formel **I** 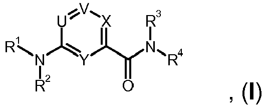 sind in den folgenden Syntheseschemata und Beispielen dargestellt.

In einigen Fällen kann die Reihenfolge bei der Durchführung der Reaktionsschemata variiert werden, um die Reaktionen zu vereinfachen oder um unerwünschte Nebenprodukte zu verhindern. Die nachfolgenden Beispiele sind genannt, um die Erfindung vollständig verständlich zu machen. Die Beispiele sollen der Anschaulichkeit der Erfindung dienen und sollen die Erfindung in keinster Weise einschränken.

Die erfindungsgemäßen Verbindungen können gemäß den dargestellten Schemata und spezifischen Beispielen oder entsprechenden Modifikationen davon hergestellt werden. Von diesen Reaktionen können auch Abwandlungen eingesetzt werden, die einem Fachmann bekannt sind, hier aber nicht detailliert beschrieben sind. Die allgemeinen Verfahren zur Herstellung der erfindungsgemäßen Verbindungen erschließen sich einem Fachmann beim Anblick der folgenden Schemata.

Ausgangsverbindungen sind kommerziell verfügbar oder werden gemäß Verfahren hergestellt, die in der Literatur beschrieben sind, in dem Fachgebiet dem Fachmann bekannt sind oder wie sie hierin beschrieben sind. Vor Durchführung der Reaktion können in den Verbindungen entsprechende funktionelle Gruppen durch übliche Schutzreste geschützt werden. Diese Schutzgruppen können auf einer geeigneten Stufe innerhalb der Reaktionssequenz nach für den Fachmann geläufigen Methoden wieder abgespalten werden.

Bei den nachstehend beschriebenen Umsetzungen können gegebenenfalls vorhandene reaktive Gruppen wie Hydroxy-, Carboxy-, Amino-, Alkylamino-, Amid- oder Iminogruppen während der Umsetzung durch übliche Schutzgruppen geschützt werden, welche nach der Umsetzung wieder abgespalten werden.
Beispielsweise kommt
- als Schutzrest für eine Hydroxygruppe die Methoxy-, Benzyloxy-,Trimethylsilyl-, Acetyl-, Benzoyl-, *tert*.-Butyl-, Trityl-, Benzyl- oder Tetrahydropyranylgruppe,
- als Schutzreste für eine Carboxylgruppe die Trimethylsilyl-, Methyl-,Ethyl-, *tert*.-Butyl-, Benzyl- oder Tetrahydropyranylgruppe, und
- als Schutzreste für eine Amidgruppe die N-Methoxymethyl- (MOM), N-Benzyloxymethyl- (BOM), N-(Trimethylsilyl)ethoxymethyl (SEM), N-*tert*-Butyldimethylsiloxy-methyl, N-*tert*-Butyldimethylsilyl- (TBDMS), N-Triisopropylsilyl- (TIPS), N-Benzyl-, N-4-Methoxybenzyl- (PMB), N-Triphenylmethyl- (Tr), N-*tert*-Butoxycarbonyl-(BOC), N-Benzyloxycarbonyl- (Cbz), N-Trimethylsilylethylsulfonyl- (SES)
- als Schutzrest für eine Amino-, Alkylamino- oder Iminogruppe die Acetyl-, Trifluor-acetyl-, Benzoyl-, Ethoxycarbonyl-, tert.-Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe,
in Betracht.

Weitere Schutzgruppen und deren Abspaltung sind in T.W. Greene, P.G.M. Wuts, "Protective Groups in Organic Synthesis", Wiley, 1991 und 1999 beschrieben.

Die gegebenenfalls anschließende Abspaltung eines verwendeten Schutzrestes erfolgt beispielsweise hydrolytisch in einem wässrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z. B. in Gegenwart von lodtrimethylsilan, bei Temperaturen zwischen 0°C und 100°C, vorzugsweise bei Temperaturen zwischen 10°C und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0°C und 50°C, vorzugsweise jedoch bei Raumtemperatur, und bei einem Wasserstoffdruck von 1 bis 7 bar, vorzugsweise jedoch von 1 bis 5 bar.

Die Abspaltung einer Methoxybenzylgruppe kann auch in Gegenwart eines Oxidationsmittels wie Cer(IV)ammoniumnitrat in einem Lösungsmittel wie Methylenchlorid, Acetonitril oder Acetonitril/Wasser bei Temperaturen zwischen 0°C und 50°C, vorzugsweise jedoch bei Raumtemperatur, erfolgen.

Die Abspaltung einer Methoxygruppe erfolgt zweckmäßigerweise in Gegenwart von Bortribromid in einem Lösungsmittel wie Methylenchlorid bei Temperaturen zwischen -35°C und -25°C.

Die Abspaltung eines 2,4-Dimethoxybenzylrestes erfolgt vorzugsweise in Trifluoressigsäure in Gegenwart von Anisol.

Die Abspaltung eines *tert*.-Butyl- oder *tert*.-Butoxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol-Wasser oder Dioxan bei Temperaturen zwischen 20°C und 50°C.

Die Abspaltung eines Methoxymethyl-Rests kann in Gegenwart einer Säure wie konzentrierter Salsäure in einem Lösungsmittel wie Dimethoxyethan durchgeführt werden. Alternativ kann eine Säure wie Trifluoressigsäure auch ohne Lösungsmittel eingesetzt werden.

Die Abspaltung eines N-(Trimethylsilyl)ethoxymethyl-Rests kann in Gegenwart von TBAF und 1,3-Dimethyl-3,4,5,6-Tetrahydro-2(1*H*)-Pyrimidon durchgeführt werden. Alternativ kann die SEM-Schutzgruppe auch mit einer Säure wie Chlorwasserstoff in einem organischen Lösungsmittel wie Dioxan oder Ethanol durchgeführt werden.

Die Abspaltung eines Allyloxycarbonylrestes erfolgt durch Behandlung mit einer katalytischen Menge Tetrakis-(triphenylphosphin)-palladium(0) vorzugsweise in einem Lösungsmittel wie Tetrahydrofuran und in Gegenwart eines Überschusses von einer Base wie Morpholin bei Temperaturen zwischen 0°C und 100°C, vorzugsweise bei Raumtemperatur und unter Inertgas, oder durch Behandlung mit einer katalytischen Menge von Tris-(tri-phenylphosphin)-rhodium(I)chlorid in einem Lösungsmittel wie wässrigem Ethanol und gegebenenfalls in Gegenwart einer Base wie 1,4-Diazabicyclo[2.2.2]octan bei Temperaturen zwischen 20°C und 70°C.

Die folgenden Verfahren zur Herstellung der erfindungsgemäßen Verbindungen der allgemeinen Formel **I** und ihrer Vorstufen haben sich besonders bewährt: 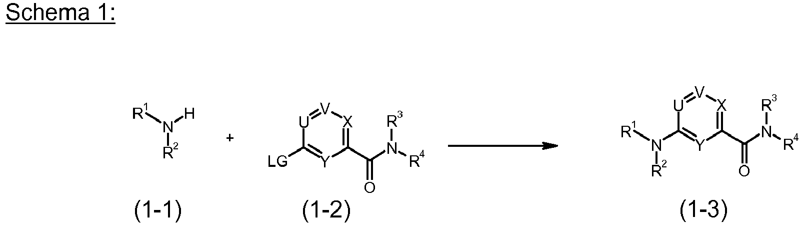 Die Herstellung einer Verbindung der allgemeinen Formel (1-3), in der **U, V, X, Y, R¹**, **R²**, **R³** und **R⁴** wie voranstehend erwähnt definiert sind, kann durch Reaktion eines Amins oder Anilins der allgemeinen Formel (1-1), in der **R¹** und **R²** wie eingangs erwähnt definiert sind, mit einer elektronenarmen Verbindung der allgemeinen Formel (1-2), in der **U, V, X, Y, R³** und **R⁴** wie eingangs erwähnt definiert sind und **LG** eine Austrittsgruppe darstellt, erfolgen. Als Austrittsgruppe **LG** können Halogenide, vorzugsweise Chloride und Bromide, -SO₂CH₃ -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe überführt werden zu können) etc. fungieren, müssen aber nicht auf diese beschränkt sein. Ganz besonders bevorzugt ist die Verwendung von Chloriden.

Die Reaktion kann über eine nucleophile aromatische Substitution in einem inerten Lösungsmittel unter Verwendung einer Hilfsbase in einem Temperaturbereich von 0°C bis zum Rückfluss des Lösungsmittels erfolgen. Nucleophile aromatische Substitutionen werden in einem geeigneten, inerten Lösungsmittel, wie Tetrahydrofuran, Toluol, Xylol, Dialkylformamide (besonders bevorzugt Dimethylformamid), cyclische Amide (besonders bevorzugt N-Methylpyrrolidon), 1,4-Dioxan, Acetonitril oder in Lösungsmittelgemischen durchgeführt. Als geeignete Hilfsbasen können tertiäre Amine wie Triethylamin oder Ethyldiisopropylamin, Alkalimetal Carbonate wie Kaliumcarbonat oder Natriumcarbonat Natriumhydrid (NaH) oder Lithiumdiisopropylamid (LDA) verwendet werden. Dabei muss das verwendete inerte Lösungsmittel kompatibel sein mit der verwendeten Base. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Alternativ können Strukturen der allgemeinen Form (1-3), wie sie in Schema 1 dargestellt sind, über Übergangsmetall-katalysierte Reaktionen synthetisiert werden. Dabei kann ein Amin oder Anilin der allgemeinen Formel (1-1), in der **R¹** und **R²** wie eingangs erwähnt definiert sind, mit einer Verbindung der allgemeinen Formel (1-2), in der **U, V, X, Y, R³** und **R⁴** wie eingangs erwähnt definiert sind und **LG** eine Austrittsgruppe darstellt, in einem inerten Lösungsmittel bei Anwesenheit eines Katalysators und einer Hilfsbase reagieren. Für den Katalysator kann zusätzlich ein geeigneter Ligand verwendet werden. Als Austrittsgruppe **LG** können Chloride, Bromide, Iodide, Trifluoracetate, Trifluormethansulfonate, Methansulfonate und Toluolsulfonate fungieren, müssen aber nicht auf diese beschränkt sein. Als inerte Lösungsmittel können Xylol, Tetrahydrofuran, Dimethylformamid, Dimethoxyethan, Toluol, Benzol, *tert*-Butanol, 1,4-Dioxan, Acetonitril oder Lösungsmittelgemische verwendet werden. Bevorzugtes Lösungsmittel ist Xylol. Geeignete Basen sind besonders Amin-Basen, wie z.B. Triethylamin oder Diisopropylethylamin, oder auch anorganische Basen wie Cäsiumcarbonat, Cäsiumacetat, Kaliumcarbonat, Kalium-*tert-*Butylat, Natriumcarbonat, Natrium-*tert*-Butylat oder Kaliumphosphat. Bevorzugte Reaktionstemperaturen sind von RT bis Rückfluss des Lösungsmittels bei Normaldruck. Typische Katalysatoren sind z.B. Übergangsmetall-Katalysatoren, wie z.B. Palladium-Katalysatoren der Art Tris(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ or Palladium(II)-chlorid. Typische Liganden sind z.B. Triphenylphosphin, Triphenylarsen, BINAP, XPhos, XantPhos, oder 2-(Di-*tert*-butylphosphino)biphenyl.

Die Herstellung einer Verbindung der allgemeinen Formel (2-3), in der **U, V, X, Y, R¹**, **R²**, **R³** und **R⁴** wie voranstehend erwähnt definiert sind, kann wie in Schema 2 gezeigt durch Kupplung einer Verbindung der allgemeinen Formel (2-2), in der **R³** und **R⁴** wie eingangs erwähnt definiert sind, mit einer Carbonsäure der allgemeinen Formel (2-1), in der **U, V, X, Y, R¹** und **R²** wie voranstehend erwähnt definiert sind, unter Zuhilfenahme von Standard-Peptid-Kupplungsreagenzien und einer Base in einem inerten Lösungsmittel erfolgen (siehe z. B. Houben-Weyl, Methoden der Organischen Chemie, Bd. 15/2). 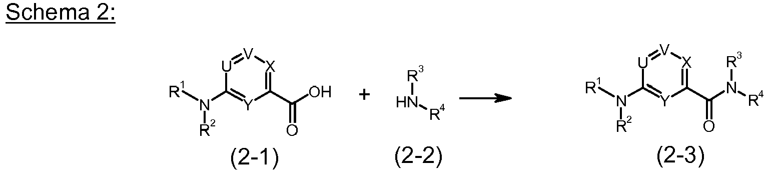 Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind besonders Amin-Basen wie z.B. Triethylamin oder Diisopropylethylamin. Als Kupplungsreagentien können beispielsweise 1*H*-Benzotriazol-1-yl-oxy-tripyrrolidino-phosphonium-hexafluorophosphat (PyBOP), Dicyclohexylcarbodiimid (DCC), Diisopropylcarbodiimid (DIC), Ethyl-(3-dimethylaminopropyl)-carbodiimid, O-(1*H*-Benzotriazol-1-yl)-N,N-N,N-tetramethyluronium-hexafluorphosphat (HBTU) oder-tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von TBTU. Die Aktivierung der Carboxylgruppe kann alternativ auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Ganz besonders bevorzugt sind Reaktionen bei Raumtemperatur. Durch Zugabe von 1-Hydroxybenzotriazol (HOBt) oder von 3-Hydroxy-4-oxo-3,4-dihydro-1,2,3-benzotriazin (HOObt) kann die Reaktionsgeschwindigkeit gesteigert werden. Auch andere Standardkupplungsbedingungen können bei der Synthese solcher Amide verwendet werden.

Die Synthese von Verbindungen der allgemeinen Formel (3-4), in der **U, V, X, Y, R¹** und **R²** wie voranstehend erwähnt definiert sind, kann entweder durch Verfahren, die dem Fachmann bekannt sind, oder durch Reaktionen, die in Schema 3 beispielhaft dargestellt sind, hergestellt werden.

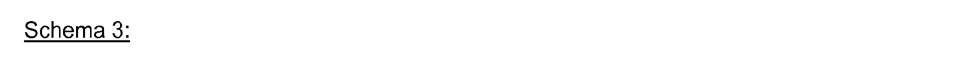

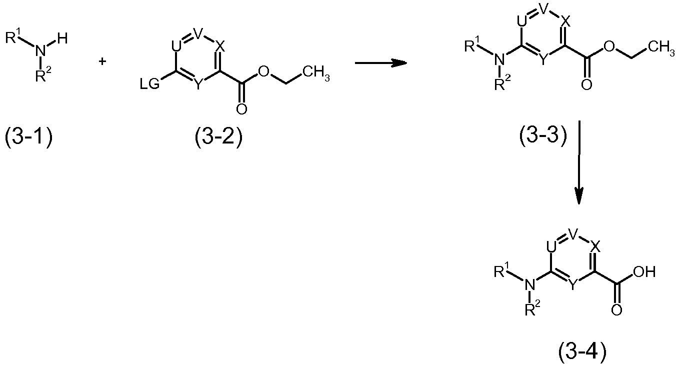

Eine Verbindung der allgemeinen Formel (3-1), in der **R¹** und **R²** wie eingangs erwähnt definiert sind, kann mit einer elektronenarmen Verbindung der allgemeinen Formel (3-2), in der **U, V, X** und **Y** wie eingangs erwähnt definiert sind und **LG** eine Austrittsgruppe darstellt, umgesetzt werden. Als Austrittsgruppe **LG** können Halogenide, bevorzugt Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe -S(O)₂-CH₃ überführt werden zu können) fungieren, müssen aber nicht auf diese beschränkt sein. Ganz besonders bevorzugt ist die Verwendung von Chloriden. Die Reaktion kann in einem inerten Lösungsmittel unter Verwendung einer Hilfsbase in einem Temperaturbereich von 0° bis Rückfluss des Lösungsmittels erfolgen. Als inertes Lösungsmittel können Tetrahydrofuran, Toluol, Xylol, Dialkylformamide (besonders bevorzugt ist Dimethylformamid), cyclische Amide (besonders ist bevorzugt N-Methyl-pyrrolidon), 1,4-Dioxan, Acetonitril oder Lösungsmittelgemische verwendet werden. Geeignete Hilfsbasen sind besonders tertiäre Amine wie Triethylamin oder Ethyldiiso-propylamin und Alkalimetal Carbonate wie Kaliumcarbonat oder Natriumcarbonat. Bevorzugt wird die Reaktion in Dimethylformamid, bei Temperaturen zwischen Raumtemperatur und Rückfluss des Lösungsmittels, in Gegenwart einer tertiären Aminbase durchgeführt.

Ester der allgemeinen Formel (3-3), in der **U, V, X, Y, R¹** und **R²** wie voranstehend erwähnt definiert sind, können durch basische oder saure Hydrolyse (J. March, Advanced Organic Chemistry (New York: J. Wiley and Sons, 1985) oder durch Umsetzen mit Alkalimetallsalzen (bevorzugt ist LiJ oder NaCN) in einem inerten Lösungsmittel in die Säure der allgemeinen Formel (3-4) überführt werden. Inerte Lösungsmittel können Dialkylformamide (besonders bevorzugt ist N,N-Dimethylformamid), Dialkylacetamide (besonders bevorzugt ist N,N-Dimethylacetamid), cyclische Amide (besonders bevorzugt ist N-Methylpyrrolidon) sein. Besonders bevorzugt ist eine alkalische Verseifung mit Alkalimetall Hydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

Die Verbindungen der allgemeinen Formel (4-3), in der **U, V, X, Y, R³** und **R⁴** wie voranstehend erwähnt definiert sind und **LG** eine Austrittsgruppe darstellt, können analog Schema 4 synthetisiert werden.

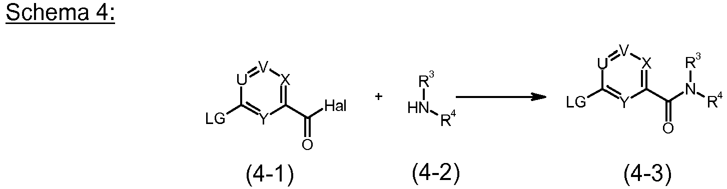

Carbonsäurehalogenide der allgemeinen Formel (4-1), in der **U, V, X** und **Y** wie voranstehend erwähnt definiert sind, und **LG** eine Austrittsgruppe, beispielsweise ein Chlorid oder Bromid, darstellt und **Hal** ein Halogenid, beispielsweise ein Chlorid oder Bromid, bedeutet, können mit Verbindungen der allgemeinen Formel (4-2), in der **R³** und **R⁴** wie eingangs erwähnt definiert sind, umgesetzt werden. Dabei kann die Reaktion in einem inerten Lösungsmittel oder auch ohne Lösungsmittel durchgeführt werden. Ebenso kann die Reaktion mit oder ohne Base durchgeführt werden. Als inerte Lösungsmittel können halogenhaltige Kohlenwasserstoffe (bevorzugt ist die Verwendung von Dichlormethan oder Dichlorethan), Dialkylether (bevorzugt ist Diethylether), cyclische Ether (bevorzugt ist 1,4-Dioxan oder Tetrahydrofuran) und aromatische Kohlenwasserstoffe verwendet werden. Als Basen können tertiäre Amine (bevorzugt ist Triethylamin oder Diisopropylethylamin) und aromatische Amine (bevorzugt ist Pyridin) verwendet werden.

Die Verbindungen der allgemeinen Formel (5-3), in der **U, V, X, Y, R³** und **R⁴** wie voranstehend erwähnt definiert sind und **LG** eine Austrittsgruppe darstellt, können analog Schema 5 synthetisiert werden.

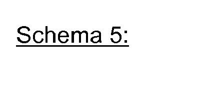

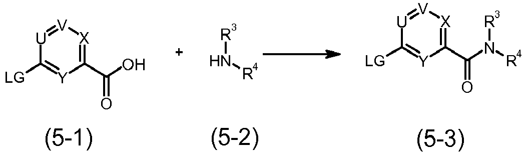

Carbonsäuren der allgemeinen Formel (5-1), in der **U, V, X** und **Y** wie voranstehend erwähnt definiert sind und **LG** eine Austrittsgruppe darstellt können mit Verbindungen der allgemeinen Formel (5-2), in der **R³** und **R⁴** wie eingangs erwähnt definiert sind, unter Zuhilfenahme von Standard-Peptid-Kupplungsreagenzien und einer Base in einem inerten Lösungsmittel zu Amiden der allgemeinen Formel (5-3) umgesetzt werden (siehe z. B. Houben-Weyl, Methoden der Organischen Chemie, Bd. 15/2). Als Austrittsgruppe **LG** können Halogenide, bevorzugt Chloride und Bromide, -SO₂CH₃, -OSO₂CH₃, -OSO₂C₆H₄-CH₃ oder -S-CH₃ (-S-CH₃ erfordert die weitere Umsetzung mit einem organischen Peroxid um in die eigentliche Austrittsgruppe -S(O)₂-CH₃ überführt werden zu können) fungieren, müssen aber nicht auf diese beschränkt sein. Als inerte Lösungsmittel können Dimethylformamid, N-Methylpyrrolidon, Dimethoxyethan, Dichlormethan, Acetonitril oder Lösungsmittelgemische verwendet werden. Bevorzugtes Lösungsmittel ist Dimethylformamid. Geeignete Basen sind Amin-Basen wie z.B. Triethylamin oder Diisopropylethylamin. Als Kupplungsreagenzien können beispielsweise 1*H*-Benzotriazol-1-yl-oxy-tripyrrolidino-phosphonium-hexafluorophosphat (PyBOP), Dicyclohexylcarbodiimid (DCC), Diisopropyl-carbodiimid (DIC), Ethyl-(3-dimethylamino-propyl)-carbodümid, O-(1*H*-Benzotriazol-1-yl)-N,N-N,N-tetramethyl-uronium-hexafluorphosphat (HBTU) oder-tetrafluorborat (TBTU) oder 1*H*-Benzotriazol-1-yl-oxy-tris-(dimethylamino)-phosphonium-hexafluorphosphat (BOP) verwendet werden. Besonders bevorzugt ist die Verwendung von TBTU. Die Aktivierung der Carboxylgruppe kann alternativ auch über ein entsprechendes Säureanhydrid oder Säurechlorid erfolgen. Die Umsetzung erfolgt im Allgemeinen in einem Temperaturbereich von -20°C bis zum Rückfluss des Lösungsmittels bei Normaldruck. Besonders bevorzugt ist die Verwendung von Diisopropylethylamin als Base und Dimethylformamid als Lösungsmittel.

Verbindungen der allgemeinen Formel (6-3), in der **U, V, X, Y, R¹** und **R²** wie voranstehend erwähnt definiert sind, lassen sich analog zu Schema 6 herstellen.

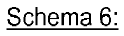

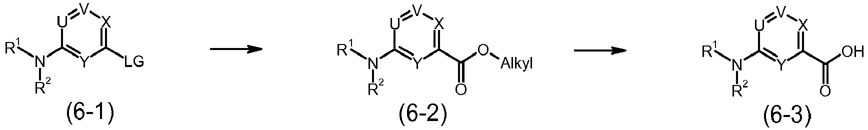

Dabei kann eine Verbindung der allgemeinen Formel (6-1), in der **U, V, X, Y, R¹** und **R²** wie voranstehend erwähnt definiert sind und **LG** eine Austrittsgruppe darstellt, in Anwesenheit eines Katalysators und einer Hilfsbase mit einem Alkohol und Kohlenmonoxid reagieren. Für den Katalysator kann zusätzlich ein geeigneter Ligand verwendet werden. Als Austrittsgruppe **LG** können Chloride, Bromide, lodide, Trifluoracetate, Trifluormethansulfonate, Methansulfonate und Toluolsulfonate fungieren, müssen aber nicht auf diese beschränkt sein. Als Alkohole können bevorzugt Methanol und Ethanol verwendet werden, müssen aber nicht auf diese beschränkt sein. Geeignete Basen sind besonders Amin-Basen wie z.B. Triethylamin oder Diisopropylethylamin oder auch anorganische Basen wie Cäsiumcarbonat, Cäsiumacetat, Kaliumcarbonat, Kalium-*tert-*Butylat, Natriumcarbonat, Natriumacetat, Natrium-*tert*-Butylat oder Kaliumphosphat. Typische Katalysatoren sind Übergangsmetall-Katalysatoren, wie z.B. Palladium-Katalysatoren wie Tris(dibenzylidenaceton)-dipalladium(0), Tetrakis-(triphenylphosphin)-palladium(0), Palladium-(II)-acetat, Pd(PPh₃)₂Cl₂, Pd(CH₃CN)₂Cl₂, Pd(dppf)Cl₂ oder Palladium(II)-chlorid. Typische Liganden sind z.B. Triphenylphosphin, Tricyclohexylphosphin, Tri-(*tert*-Butyl)phosphin, 1,4-Bis(diphenylphosphino)butan (dppb), 1,1'-Bis(diphenylphosphino)ferrocen (dppf), 1,3-Bis(diisopropylphosphino)-propan, 1,3-Bis(diphenylphosphino)propan (dppp), 1,4-bis(Dicyclohexylphosphino)butan, 1,1'-Bis(Dicyclohexylphosphino)ferrocen. Der Druck von Kohlenmonoxid im Reaktionsgefäß beträgt von 1 bar bis 100 bar, bevorzugt sind erhöhte Kohlenmonoxid-Drücke von 10 bis 30 bar. Die Reaktionen können in einem Temperaturbereich von RT bis 200°C durchgeführt werden. Besonders bevorzugt ist ein Temperaturbereich von 100°C bis 150°C (M. Beller, W. Magerlein, A.F. Indolese, Ch. Fischer, Synthesis (2001) 7, 1098-1109 und darin zitierte Literatur.).

Ester der allgemeinen Formel (6-2), in denen **U, V, X, Y, R¹** und **R²** wie voranstehend erwähnt definiert sind und **Alkyl** eine C₁₋₆-Alkylgruppe darstellt, können durch basische oder saure Hydrolyse (J. March, Advanced Organic Chemistry (New York: J. Wiley and Sons, 1985) oder durch Umsetzen mit Alkalimetal Salze (bevorzugt ist LiJ oder NaCN) in einem inerten Lösungsmittel in die Säure der allgemeinen Formel (6-3) überführt werden. Inerte Lösungsmittel können Dialkylformamide (bevorzugt ist N,N-Dimethylformamid), Dialkylacetamide (bevorzugt ist N,N-Dimethylacetamid), cyclische Amide (bevorzugt ist N-Methylpyrrolidon) sein. Besonders bevorzugt ist eine alkalische Verseifung mit Alkalimetallhydroxiden wie Natriumhydroxid oder Lithiumhydroxid in inerten Lösungsmitteln. Als inerte Lösungsmittel eignen sich Wasser und cyclische Ether wie 1,4-Dioxan oder Tetrahydrofuran sowie auch Lösungsmittelgemische.

In einigen Fällen kann das Endprodukt weiter derivatisiert werden, z.B. durch Manipulation der Substituenten. Diese Manipulationen können unter anderem diejenigen sein, die dem Fachmann allgemein bekannt sind wie Oxidation, Reduktion, Alkylierung, Acylierung und Hydrolyse, müssen allerdings nicht auf diese beschränkt sein.

Die erfindungsgemäßen neuen Verbindungen der allgemeinen Formel **I** können ein oder mehrere Chiralitätszentren enthalten. Sind beispielsweise zwei Chiralitätszentren vorhanden, dann können die Verbindungen in Form zweier diastereomerer Antipodenpaare auftreten. Die Erfindung umfasst die einzelnen Isomere ebenso wie ihre Gemische.

Die Trennung der jeweiligen Diastereomeren gelingt auf Grund ihrer unterschiedlichen physikochemischen Eigenschaften, z.B. durch fraktionierte Kristallisation aus geeigneten Lösemitteln, durch Hochdruckflüssigkeits- oder Säulenchromatographie unter Verwendung chiraler oder bevorzugt achiraler stationärer Phasen.

Die Trennung von unter die allgemeine Formel **I** fallenden Racematen gelingt beispielsweise durch HPLC an geeigneten chiralen stationären Phasen (z. B. Chiral AGP, Chiralpak AD). Racemate, die eine basische oder saure Funktion enthalten, lassen sich auch über die diastereomeren, optisch aktiven Salze trennen, die bei Umsetzung mit einer optisch aktiven Säure, beispielsweise (+)- oder (-)-Weinsäure, (+)- oder (-)-Diacetylweinsäure, (+)- oder (-)-Monomethyltartrat oder (+)- oder (-)-Camphersulfonsäure, bzw. einer optisch aktiven Base, beispielsweise mit (R)-(+)-1-Phenylethylamin, (S)-(-)-1-Phenylethyl-amin oder (S)-Brucin, entstehen.

Nach einem üblichen Verfahren zur Isomerentrennung wird das Racemat einer Verbindung der allgemeinen Formel **I** mit einer der vorstehend angegebenen optisch aktiven Säuren bzw. Basen in äquimolarer Menge in einem Lösemittel umgesetzt und die erhaltenen kristallinen, diastereomeren, optisch aktiven Salze unter Ausnutzung ihrer verschiedenen Löslichkeit getrennt. Diese Umsetzung kann in jeder Art von Lösemitteln durchgeführt werden, solange sie einen ausreichenden Unterschied hinsichtlich der Löslichkeit der Salze aufweisen. Vorzugsweise werden Methanol, Ethanol oder deren Gemische, beispielsweise im Volumenverhältnis 50:50, verwendet. Sodann wird jedes der optisch aktiven Salze in Wasser gelöst, mit einer Base, wie Natriumcarbonat oder Kaliumcarbonat, oder mit einer geeigneten Säure, beispielsweise mit verdünnter Salzsäure oder wässeriger Methansulfonsäure, vorsichtig neutralisiert und dadurch die entsprechende freie Verbindung in der (+)- oder (-)-Form erhalten.

Jeweils nur das (R)- oder (S)-Enantiomer bzw. ein Gemisch zweier optisch aktiver, unter die allgemeine Formel **I** fallender, diastereomerer Verbindungen wird auch dadurch erhalten, dass man die oben beschriebenen Synthesen mit jeweils einer geeigneten (R)-bzw. (S)-konfigurierten Reaktionskomponente durchführt.

Die neuen Verbindungen der allgemeinen Formel **I** und deren physiologisch verträgliche Salze weisen wertvolle pharmakologische Eigenschaften auf, die auf ihre selektiven CGRP-antagonistischen Eigenschaften zurück gehen. Ein weiterer Gegenstand der Erfindung sind diese Verbindungen enthaltende Arzneimittel, deren Verwendung und deren Herstellung.

Zum Nachweis der Affinität der voranstehend genannten Verbindungen zu humanen CGRP-Rezeptoren und ihrer antagonistischen Eigenschaften wurden die folgenden Versuche durchgeführt:

### A. Bindungsstudien mit (den humanen CGRP-Rezeptor exprimierenden) SK-N-MC-Zellen

SK-N-MC Membranen (~ 20 µg Proteinmenge) werden für 180 Minuten bei Raumtemperatur mit 50 pM ¹²⁵I-Iodotyrosyl-Calcitonin-Gene-Related Peptide und ansteigenden Konzentrationen der Testsubstanzen in einem Gesamtvolumen von 250 µl inkubiert (Assay Puffer: 10 mM Tris, 50 mM NaCl, 5 mM MgCl₂, 1mM EDTA, pH=7.4). Die Inkubation wird durch rasche Filtration durch mit Polyethylenimin (0.1%) behandelte GF/B-Glasfaserfilter mittels eines Zellharvesters beendet. Die an Protein gebundene Radioaktivität wird mit Hilfe eines Gammacounters bestimmt. Als nichtspezifische Bindung wird die gebundene Radioaktivität nach Gegenwart von 1 µM BIBN4096BS während der Inkubation definiert.

Die Analyse der Konzentrations-Bindungskurven erfolgt mit Hilfe einer computergestützten nichtlinearen Kurvenanpassung.

Die eingangs erwähnten Verbindungen zeigen in dem beschriebenen Test Kᵢ-Werte ≤ 50 µM.

### B. CGRP-Antagonismus in SK-N-MC-Zellen

SK-N-MC Zellen (-1000 Zellen pro well) werden in Anwesenheit von steigenden Konzentrationen von CGRP und verschiedenen Konzentrationen der Testsubstanz für 30 Minuten inkubiert.

Die cAMP-Gehalte der Proben werden mittels AlphaScreen cAMP assay kit (Perkin Elmer) bestimmt und die pA₂-Werte antagonistisch wirkender Substanzen graphisch ermittelt.

Die erfindungsgemäßen Verbindungen zeigen in dem beschriebenen *in vitro*-Testmodell CGRP-antagonistische Eigenschaften in einem Dosisbereich zwischen 10⁻¹² bis 10⁻⁴ M.

Um zu zeigen, dass die Verbindungen der allgemeinen Formel **I** mit unterschiedlichen Strukturelementen gute bis sehr gute CGRP-antagonistische Aktivitäten zeigen, werden in der folgenden Tabelle die Kᵢ-Werte angegeben, die gemäß dem voranstehenden Versuchsprotokoll erhalten wurden. Dazu wird angemerkt, dass die Verbindungen im Hinblick auf ihre unterschiedlichen strukturellen Elemente ausgewählt wurden und nicht, um spezifische Verbindungen hervorzuheben:

| **Beispiel** | **Kᵢ [nM]** |
|---|---|
| (1) | 15 |
| (3) | 120 |
| (6) | 132 |

### INDIKATIONSGEBIETE

Aufgrund ihrer pharmakologischen Eigenschaften eignen sich die erfindungsgemäßen Verbindungen und deren Salze mit physiologisch verträglichen Säuren somit zur akuten und prophylaktischen Behandlung von Kopfschmerzen, insbesondere Migräne-, Cluster-Kopfschmerz sowie Spannungskopfschmerzen. Weiterhin beeinflussen die erfindungsgemäßen Verbindungen auch die folgenden Erkrankungen positiv: Nicht-insulinabhängigen Diabetes mellitus ("NIDDM"), cardiovaskuläre Erkrankungen, Morphintoleranz, Clostridiumtoxin-bedingte Durchfallerkrankungen, Erkrankungen der Haut, insbesondere thermische und strahlenbedingte Hautschäden inklusive Sonnenbrand, Lichen, Prurigo, pruriginöse Toxidermien sowie schwerer Juckreiz, entzündliche Erkrankungen, z.B. entzündliche Gelenkerkrankungen (Osteoarthritis, rheumatoide Arthritis, neurogene Arthritis), generalisierter Weichteilrheumatismus (Fibromyalgie), neurogene Entzündungen der oralen Mucosa, entzündliche Lungenerkrankungen, allergische Rhinitis, Asthma, COPD, Erkrankungen, die mit einer überschießenden Gefäßerweiterung und dadurch bedingter verringerter Gewebedurchblutung einhergehen, z.B. Schock und Sepsis, chronische Schmerzerkrankungen, wie z.B. diabetische Neuropathien, durch Chemotherapien induzierte Neuropathien, HIV-induzierte Neuropathien, postherpetische Neuropathien durch Gewebetrauma induzierte Neuropathien, trigeminale Neuralgien, temporomandibuläre Dysfunktionen, CRPS (complex regional pain syndrome), Rückenschmerzen, und viszerale Erkrankungen, wie z.B. irritable bowel syndrome (IBS), inflammatory bowel syndrome. Darüber hinaus zeigen die erfindungsgemäßen Verbindungen eine lindernde Wirkung auf Schmerzzustände im allgemeinen. Die Symptomatik menopausaler, durch Gefäßerweiterung und erhöhten Blutfluss verursachter Hitzewallungen östrogendefizienter Frauen sowie hormonbehandelter Prostatakarzinompatienten und Kastraten wird durch die CGRP-Antagonisten der vorliegenden Anwendung präventiv und akut-therapeutisch günstig beeinflusst, wobei sich dieser Therapieansatz vor der Hormonsubstitution durch Nebenwirkungsarmut auszeichnet. Vorzugsweise eignen sich die erfindungsgemäßen Verbindungen zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

Die zur Erzielung einer entsprechenden Wirkung erforderliche Dosierung beträgt zweckmäßigerweise bei intravenöser oder subkutaner Gabe 0.0001 bis 3 mg/kg Körpergewicht, vorzugsweise 0.01 bis 1 mg/kg Körpergewicht, und bei oraler, nasaler oder inhalativer Gabe 0.01 bis 10 mg/kg Körpergewicht, vorzugsweise 0.1 bis 10 mg/kg Körpergewicht, jeweils ein- bis dreimal täglich.

Sofern die Behandlung mit CGRP-Antagonisten oder/und CGRP-Release-Hemmern in Ergänzung zu einer üblichen Hormonsubstitution erfolgt, empfiehlt sich eine Verringerung der vorstehend angegebenen Dosierungen, wobei die Dosierung dann 1/5 der vorstehend angegebenen Untergrenzen bis zu 1/1 der vorstehend angegebenen Obergrenzen betragen kann.

Ein weiterer Gegenstand der Erfindung ist die Verwendung der erfindungsgemäßen Verbindungen als wertvolle Hilfsmittel zur Erzeugung und Reinigung (Affinitätschromatographie) von Antikörpern sowie, nach geeigneter radioaktiver Markierung, beispielsweise durch Tritiierung geeigneter Vorstufen, beispielsweise durch katalytische Hydrierung mit Trithium oder Ersatz von Halogenatomen durch Tritium, in RIA- und ELISA-Assays und als diagnostische bzw. analytische Hilfsmittel in der Neurotransmitter-Forschung.

### KOMBINATIONEN

Als Kombinationspartner denkbare Wirkstoffklassen sind z.B. Antiemetica, Prokinetica, Neuroleptica, Antidepressiva, Neurokinin-Antagonisten, Anticonvulsiva, Histamin-H1-Rezeptorantagonisten, β-Blocker, α-Agonisten und α-Antagonisten, Ergotalkaloiden, schwachen Analgetica, nichtsteroidalen Antiphlogistica, Corticosteroiden, Calcium-Antagonisten, 5-HT_{1B/1D}-Agonisten oder andere Antimigränemitteln, die zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/- Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden können.

Für die oben erwähnten Kombinationen kommen somit als weitere Wirksubstanzen beispielsweise die nicht-steroidalen Antiphlogistika Aceclofenac, Acemetacin, Acetyl-salicylsäure, Acetaminophen (Paracetamol), Azathioprin, Diclofenac, Diflunisal, Fenbufen, Fenoprofen, Flurbiprofen, Ibuprofen, Indometacin, Ketoprofen, Leflunomid, Lornoxicam, Mefenaminsäure, Naproxen, Phenylbutazon, Piroxicam, Sulfasalazin, Zomepirac oder deren pharmazeutisch verträgliche Salze sowie Meloxicam und andere selektive COX2-Inhibitoren, wie beispielsweise Rofecoxib, Valdecoxib, Parecoxib, Etoricoxib und Celecoxib, in Betracht sowie Substanzen, die frühere oder spätere Schritte in der Prostaglandin-Synthese inhibieren oder Prostaglandinrezeptor Antagonisten wie z.B. EP2-Rezeptor Antagonisten und IP-Rezeptor Antagonisten.

Weiterhin können Ergotamin, Dihydroergotamin, Metoclopramid, Domperidon, Diphenhydramin, Cyclizin, Promethazin, Chlorpromazin, Vigabatrin, Timolol, Isomethepten, Pizotifen, Botox, Gabapentin, Pregabalin, Duloxetin, Topiramat, Riboflavin, Montelukast, Lisinopril, Micardis, Prochlorperazin, Dexamethason, Flunarizin, Dextropropoxyphen, Meperidin, Metoprolol, Propranolol, Nadolol, Atenolol, Clonidin, Indoramin, Carbamazepin, Phenytoin, Valproat, Amitryptilin, Imipramine, Venlafaxine, Lidocain oder Diltiazem und andere 5-HT_{1B/1D}-Agonisten wie z.B. Almotriptan, Avitriptan, Eletriptan, Frovatriptan, Naratriptan, Rizatriptan, Sumatriptan und Zolmitriptan verwendet werden.

Außerdem können CGRP-Antagonisten mit Vanilloid-Rezeptor Antagonisten, wie z.B. VR-1 Antagonisten, Glutamatrezeptor Antagonisten, wie z.B. mGlu5-Rezeptor Antagonisten, mGlu1-Rezeptor Antagonisten, iGlu5-Rezeptor Antagonisten, AMPA-Rezeptor Antagonisten, Purinrezeptor Blockern, wie z.B. P2X3 Antagonisten, NO-Synthase Inhibitoren, wie z.B. iNOS Inhibitoren, Calciumkanal-Blockern, wie z.B. PQ-typ Blockern, N-typ Blockern, Kaliumkanalöffnern, wie z.B. KCNQ Kanalöffnern, Natriumkanal Blockern, wie z.B. PN3 Kanal Blockern, NMDA-Rezeptor Antagonisten, Acid-sensing Ionenkanal Antagonisten, wie z.B. ASIC3 Antagonisten, Bradykinin Rezeptor Antagonisten wie z.B. B1-Rezeptor Antagonisten, Cannabinoid-Rezeptor Agonisten, wie z.B. CB2 Agonisten, CB1 Agonisten, Somatostatin-Rezeptor Agonisten, wie z.B. sst2 Rezeptor Agonisten gegeben werden.

Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 20 bis 100 mg Sumatriptan.

### DARREICHUNGSFORMEN

Die erfindungsgemäß hergestellten Verbindungen können entweder alleine oder gegebenenfalls in Kombination mit anderen Wirksubstanzen zur Behandlung von Migräne intravenös, subkutan, intramuskulär, intraartikulär, intrarektal, intranasal, durch Inhalation, topisch, transdermal oder oral erfolgen, wobei zur Inhalation insbesondere Aerosol-formulierungen geeignet sind. Die Kombinationen können entweder simultan oder sequentiell verabreicht werden.

Geeignete Anwendungsformen sind beispielsweise Tabletten, Kapseln, Lösungen, Säfte, Emulsionen oder Inhalationspulver oder -aerosole. Hierbei soll der Anteil der pharmazeutisch wirksamen Verbindung(en) jeweils im Bereich von 0.1 bis 90 Gew.-%, bevorzugt 0.5 bis 50 Gew.-% der Gesamtzusammensetzung liegen, d.h. in Mengen die ausreichend sind, um den voranstehend angegebenen Dosierungsbereich zu erreichen.

Die orale Gabe kann in Form einer Tablette, als Pulver, als Pulver in einer Kapsel (z.B. Hartgelatinekapsel), als Lösung oder Suspension erfolgen. Im Fall einer inhalativen Gabe kann die Wirkstoffkombination als Pulver, als wässrige oder wässrig-ethanolische Lösung oder mittels einer Treibgasformulierung erfolgen.

Bevorzugt sind deshalb pharmazeutische Formulierungen gekennzeichnet durch den Gehalt an einer oder mehrerer Verbindungen der Formel **I** gemäß der obigen bevorzugten Ausführungsformen.

Besonders bevorzugt ist es, wenn die Verbindungen der Formel **I** oral verabreicht werden, ganz besonders bevorzugt ist es, wenn die Verabreichung ein oder zweimal täglich erfolgt. Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxymethyl-cellulose, Celluloseacetatphthalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein Geschmack verbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie *p*-Hydroxybenzoate, enthalten.

Die eine oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt. Geeignete Zäpfchen lassen sich beispielsweise durch Vermischen mit dafür vorgesehenen Trägermitteln, wie Neutralfetten oder Polyäthylenglykol beziehungsweise dessen Derivaten, herstellen.

Als Hilfsstoffe seien beispielsweise Wasser, pharmazeutisch unbedenkliche organische Lösemittel, wie Paraffine (z.B. Erdölfraktionen), Öle pflanzlichen Ursprungs (z.B. Erdnuss- oder Sesamöl), mono- oder polyfunktionelle Alkohole (z.B. Ethanol oder Glycerin), Trägerstoffe wie z.B. natürliche Gesteinsmehle (z.B. Kaoline, Tonerden, Talkum, Kreide) synthetische Gesteinsmehle (z.B. hochdisperse Kieselsäure und Silikate), Zucker (z.B. Rohr-, Milch- und Traubenzucker) Emulgiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat) erwähnt.

Im Falle der oralen Anwendung können die Tabletten selbstverständlich außer den genannten Trägerstoffen auch Zusätze, wie z.B. Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mit verwendet werden. Im Falle wässriger Suspensionen können die Wirkstoffe außer den oben genannten Hilfsstoffen mit verschiedenen Geschmacksaufbesserern oder Farbstoffen versetzt werden.

Ebenfalls bevorzugt ist es, wenn die Verbindungen der Formel **I** inhalativ verabreicht werden, besonders bevorzugt ist es, wenn die Verabreichung ein oder zweimal täglich erfolgt. Hierzu müssen die Verbindungen der Formel **I** in inhalierbaren Darreichungsformen bereitgestellt werden. Als inhalierbare Darreichungsformen kommen Inhalations-pulver, treibgashaltige Dosieraerosole oder treibgasfreie Inhalationslösungen in Betracht, die gegebenenfalls im Gemisch mit gebräuchlichen physiologisch verträglichen Hilfsstoffen vorliegen.

Im Rahmen der vorliegenden Erfindung sind von dem Begriff treibgasfreie Inhalationslösungen auch Konzentrate oder sterile, gebrauchsfertige Inhalationslösungen umfasst. Die im Rahmen der vorliegenden Erfindung einsetzbaren Darreichungsformen werden im nachfolgenden Teil der Beschreibung detailliert beschrieben.

### EXPERIMENTELLER TEIL

Für die hergestellten Verbindungen liegen in der Regel ¹H-NMR und/oder Massenspektren vor. Wenn nicht anders angegeben, werden R_{f}-Werte unter Verwendung von DC-Fertigplatten Kieselgel 60 F254 (E. Merck, Darmstadt, Artikel-Nr. 1.05714) ohne Kammersättigung bestimmt.

Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser (NH₄OH). Verwendete Laufmittelsysteme für Dünnschicht-Chromatographie:
- Laufmittel A: Dichlormethan/Cyclohexan/Methanol/NH₄OH = 70/15/15/2
- Laufmittel B: Petrolether/Ethylacetat = 2/1

Soweit nicht anders vermerkt sind die bei den Aufarbeitungen der Reaktionslösungen verwendeten Säure-, Basen- und Salzlösungen wässrige Systeme der angegebenen Konzentrationen. Zu chromatographischen Reinigungen wird Kieselgel der Firma Millipore (MATREX^{™}, 35-70 µm) verwendet.

Die angegebenen HPLC-Daten werden unter nachstehend angeführten Parametern und unter Verwendung der aufgeführten Säulen erhoben:

### Verwendete Säulen:

**(Säulentemperatur: 30°C; Injektionsvolumen: 5 µL; Detektion bei 254 nm)**

| | |
|---|---|
| S1 | Zorbax-Säule (Agilent Technologies), |
| | SB (Stable Bond) C18; 3.5 µm; 4.6 x 75 mm |
| S2 | Waters Sunfire, |
| | SB (Stable Bond) C18; 3.5 µm; 4.6 x 75 mm |
| S3 | Agilent Bonus C18; 5 µm, 4.6 x 75 mm |
| S4 | Zorbax-Säule (Agilent Technologies), |
| | SB (Stable Bond) C18; 1.8 µm; 3.0 x 30 mm |
| S5 | Zorbax-Säule (Agilent Technologies), |
| | SB (Stable Bond) C18; 5 µm; 4.6 x 75 mm |
| S6 | Waters Symmetry C18; 3.5 µm; 4.6 x 75 mm |
| S7 | Waters XBridge C18; 3.5 µm; 4.6 x 75 mm (basische Säule) |
| S8 | Waters XBridge C18; 3.0 x 30mm, 2.5 µm (basische Säule) |
| S9 | Waters Sunfire C18; 4.6 x 50mm, 3.5 µm (Säulentemperatur 40°C) |

### Verwendete Lösungsmittel:

- Für die Säulen S1 bis S6 (saure Bedingungen) wurden als Lösungsmittel verwendet:
   Lösungsmittel A: Wasser (mit 0.1% Ameisensäure)
   Lösungsmittel B: Acetonitril (mit 0.1% Ameisensäure)
- Für die Säule S7 und S8 (basische Bedingungen) wurden folgende Lösungsmittel verwendet:
   Lösungsmittel A: Wasser (mit 0.1% NH₄OH)
   Lösungsmittel B: Acetonitril (mit 0.1% NH₄OH)
- Für die Säule S9 (saure Bedingngen) wurden als Lösungsmittel verwendet:
   Lösungsmittel A: Wasser (mit 0.1% Trifluoressigsäure)
   Lösungsmittel B: Acetonitril (mit 0.1% Trifluoressigsäure)
(die prozentualen Angaben beziehen sich auf das Gesamtvolumen)

**Gradienten:**

| Gradient (Fluss) | Zeit [min] | %A | %B |
|---|---|---|---|
| **G1** (0.8 mL/min) | 0.0 | 95 | 5 |
| | 8.0 | 50 | 50 |
| | 9.0 | 10 | 90 |
| | 10.0 | 10 | 90 |
| | 11.0 | 95 | 5 |

| Gradient | Zeit [min] | %A | %B |
|---|---|---|---|
| **G2** (1.6 mL/min) | 0.00 | 95 | 5 |
| | 0.10 | 95 | 5 |
| | 1.75 | 5 | 95 |
| | 1.90 | 5 | 95 |
| | 1.95 | 95 | 5 |
| | 2.00 | 95 | 5 |
| **G3** (1.6 mL/min) | 0.00 | 95 | 5 |
| | 4.50 | 10 | 90 |
| | 5.00 | 10 | 90 |
| | 5.50 | 95 | 5 |
| **G4** (1.6 mL/min) | 0.00 | 95 | 5 |
| | 4.00 | 50 | 50 |
| | 4.50 | 10 | 90 |
| | 5.00 | 10 | 90 |
| | 5.50 | 95 | 5 |
| **G5** (1.6 mL/min) | 0.00 | 90 | 10 |
| | 4.50 | 10 | 90 |
| | 5.50 | 10 | 90 |
| **G6** (1.4 mL/min) | 0.00 | 95 | 5 |
| | 1.80 | 10 | 90 |
| | 2.00 | 10 | 90 |
| | 2.20 | 95 | 5 |
| **G7** (1.6 mL/min) | 0.00 | 95 | 5 |
| | 2.00 | 50 | 50 |
| **G8** (1.5 mL/min) | 2.25 | 10 | 90 |
| | 2.50 | 10 | 90 |
| | 2.75 | 95 | 5 |
| | 0.00 | 95 | 5 |
| | 2.00 | 00 | 100 |
| | 2.50 | 00 | 100 |
| | 2.60 | 95 | 5 |
| | 2.90 | 95 | 5 |

**Methoden:**

| Methode | Säule | Gradient |
|---|---|---|
| Methode A | S1 | G4 |
| Methode B | S2 | G4 |
| Methode C | S4 | G2 |
| Methode D | S6 | G4 |
| Methode E | S1 | G3 |
| Methode F | S3 | G3 |
| Methode G | S5 | G4 |
| Methode H | S1 | G5 |
| Methode K | S2 | G3 |
| Methode L | S1 | G2 |
| Methode M | S7 | G3 |
| Methode N | S2 | G1 |
| Methode O | S8 | G6 |
| Methode P | S4 | G7 |
| Methode Q | S9 | G8 |

Bei präparativen HPLC-Reinigungen erfolgt die Sammlung der Produkte entweder massengesteuert oder über UV-Detektion. Die Produkt enthaltenden Fraktionen werden vereinigt und gefriergetrocknet. Für preparative HPLC-Trennungen können folgende Säulen verwendet werden:

| | |
|---|---|
| S8 | Agilent Zorbax SB C18, 50 x 150 mm, 5 µm |
| S9 | Agilent Zorbax Stable Bond, 50 x 140 mm, 7 µm |
| S10 | Waters Sunfire C18, 30 x 100 mm, 5 µm |
| S11 | Waters Symmetry 50 x 140 mm, 7 µm |
| S12 | Agilent Zorbax Stable Bond C18, 30 x 100 mm, 5 µm, |

Falls nähere Angaben zur Konfiguration fehlen, bleibt offen, ob es sich um reine Enantiomere handelt oder ob partielle oder gar völlige Racemisierung eingetreten ist.

In den Versuchsbeschreibungen werden die folgenden Abkürzungen verwendet:
- AAV: Allgemeine Arbeitsvorschrift
- AcOH: Essigsäure
- BINAP: 2,2'-Bis-(diphenylphosphino)-1,1'-binaphtyl
- BOC: *tert*.-Butyloxycarbonyl
- CDI: 1,1'-Carbonyldiimidazol
- Cyc: Cyclohexan
- DCM: Dichlormethan
- DIPE: Diisopropylether
- DIPEA: Diisopropylethylamin
- DMF: *N*,*N*-Dimethylformamid
- dppf: 1,1'-Bis-(diphenylphosphino)ferrocen
- d.Th.: der Theorie
- EI: Elektronenstoß-Ionisation (bei MS)
- eq: Äquivalente
- ESI: Elektrospray-Ionisation (bei MS)
- EtOAc: Essigsäureethylester
- EtOH: Ethanol
- E-Wasser: entionisiertes Wasser
- HATU: *O*-(7-Azabenztriazol-1-yl)-*N*,*N*,*N*',*N*'-tetramethyluronium-hexafluorphosphat
- HCl: Chlorwasserstoff
- HPLC: High Performance Liquid Chromatography
- HPLC-MS: HPLC gekoppelte Massenspektrometrie
- i.vac.: in vacuo (im Vakuum)
- konz.: konzentriert
- MeOH: Methanol
- MS: Massenspektrometrie
- MW: Molekulargewicht [g/mol]
- NaOAc: Natriumacetat
- NaOH: Natriumhydroxid
- NH₄OH: Ammoniumhydroxid (wässrige Ammoniak-Lösung, 30%)
- NMP: N-Methyl-2-Pyrrolidin
- Pd/C: Palladium auf Kohle
- PdCl₂(PPh₃)₂: Bis(Triphenylphosphine)palladium (II) chlorid
- Pd₂dba₃: Bis(dibenzylidenaceton) palladium (0)
- PE: Petrolether
- R_{f}: Retentionsindex (bei DC)
- Rₜ: Retentionszeit (bei HPLC)
- RT: Raumtemperatur
- TBTU: O-(Benzotriazol-1-yl)-*N*,*N*,*N*',*N*'-tetramethyluronium-tetrafluoroborat
- TEA: Triethylamin
- TFA: Trifluoressigsäure
- THF: Tetrahydrofuran
- TS: Trockenschrank
- ULTS: Umluft-Trockenschrank
- XantPhos: 4,5-Bis(Diphenylphosphino)-9,9-dimethylxanthen
- XPhos: 2-Dicyclohexylphosphino-2',4',6'-triisopropylbiphenyl

### Herstellung der Ausgangsverbindungen:

### Intermediat 1:

### 1'H-Spiro[piperidin-4,2'-chinazolin]-4'(3'H)-on

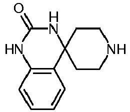

wurde wie in der Offenlegungsschrift WO 2003/104236 beschrieben dargestellt.

| | |
|---|---|
| Ausbeute: | 5.20 g (97% d.Th.) |
| ESI-MS: | m/z = 218 (M+H)⁺ |
| R_{f} (Kieselgel): | 0.08 (Laufmittel A) |

### Intermediat 2:

### 6-Chlorpyrimidin-4-carbonsäurechlorid

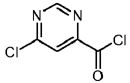

### Stufe 1: 6-Hydroxypyrimidin-4-carbonsäure

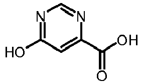

Zu 24.1 g (0.6 mol) NaOH in 3.6 L Wasser wurden 63.5 g (0.3 mol) Natriumdiethyloxalacetat und 30.2 g (0.3 mol) Formamidin-acetat zugegeben. Der Ansatz wurde über Nacht bei RT gerührt. Anschließend fügte man Aktivkohle hinzu und kochte den Ansatz für 1 h unter Rückfluss. Es wurde heiß abfiltriert und nach dem Erkalten mit einer Salzsäure-Lösung angesäuert. Die Lösung wurde bis zur Trockne einrotiert. Der Rückstand enthielt das gewünschte Produkt und wurde ohne weitere Aufreinigung in die nächste Stufe eingesetzt.

| | |
|---|---|
| Ausbeute: | 83.0 g |

### Stufe 2: 6-Chlorpyrimidin-4-carbonsäurechlorid

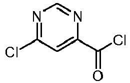

50.0 g (0.35 mol) 6-Hydroxypyrimidin-4-carbonsäure wurde vorgelegt und 500 mL Phosphoroxychlorid zugegeben. Anschließend wurde unter Rühren 150 g (0.72 mol) Phosphorpentachlorid portionsweise zugegeben. Der Reaktionsansatz wurde 5 h unter Rückfluss gekocht. Das Phosphoroxychlorid wurde abdestilliert und der Rückstand durch Vakuumdestillation über eine Kolonne aufgereinigt.

| | |
|---|---|
| Ausbeute: | 51.9 g (83% d.Th.) |
| MS: | m/z = 176 / 178 / 180 (M)⁺ |

### Intermediat 3:

### 6-Chlorpyrimidin-4-carbonsäureethylester

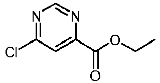

1.0 g (5.65 mmol) 6-Chlorpyrimidin-4-carbonsäurechlorid und 0.4 mL (6.94 mmol) Ethanol wurden in 30 mL Dichlormethan zusammengegeben und über Nacht bei RT gerührt. Das Lösungsmittel wurde i.vac. entfernt.

| | |
|---|---|
| Ausbeute: | 1.0 g (95% d.Th.) |
| ESI-MS: | m/z = 187 / 189 (M+H)⁺ |
| R_{f} (Kieselgel): | 0.85 (EtOAc) |

### Intermediat 4:

### 6-(3-Methyl-2,5-dioxo-1',3'-dihydrospiro[imidazolidin-4,2'-inden]-5'-ylamino)pyrimidin-4-carbonsäureethylester

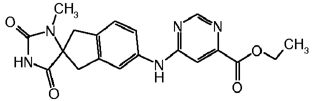

Zu 400 mg (2.14 mmol) 6-Chlorpyrimidin-4-carbonsäureethylester in 10 mL DMF wurden 500 mg (2.16 mmol) 5'-Amino-3-methyl-1',3'-dihydrospiro-[imidazolidin-4,2'-inden]-2,5-dion (Bioorg. Med. Chem. Lett.; 2006; 16; 24; 6165-6169) und 400 µL (2.33 mmol) DIPEA hinzugefügt und der Reaktionsansatz 8 h bei 100°C gerührt. Das Reaktionsgemisch wurde mit 60 mL Wasser verdünnt und 30 min gerührt. Der Niederschlag wurde abgesaugt und das Filtrat mit Dichlormethan (3 x 40 mL) extrahiert. Die organischen Phasen wurden vereinigt, über Magnesiumsulfat getrocknet, filtriert und i.vac. eingeengt. Der Rückstand wurde in DMF gelöst und mittels präparativer HPLC aufgereinigt. Die Produktfraktionen wurden vereinigt und gefriergetrocknet.

| | |
|---|---|
| Ausbeute: | 140 mg (17% d.Th.) |
| ESI-MS: | m/z = 382 (M+H)⁺ |
| R_{f} (Kieselgel): | 0.49 (Laufmittel A) |

### Intermediat 5:

### 6-(3-Methyl-2,5-dioxo-1',3'-dihydrospiro[imidazolidin-4,2'-inden]-5'-ylamino)pyrimidin-4-carbonsäure

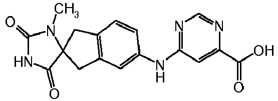

140 mg (0.37 mmol) 6-(3-Methyl-2,5-dioxo-1',3'-dihydrospiro[imidazolidin-4,2'-inden]-5'-ylamino)pyrimidin-4-carbonsäureethylester, 5 mL Ethanol und 1 mL (1.0 mmol) einer 1 M NaOH-Lösung wurden 2 h bei RT gerührt. Der Reaktionsansatz wurde mit 1 mL einer 1 M Salzsäure-Lösung versetzt und das Ethanol i.vac. entfernt. Der wässrige Rückstand wurde gekühlt und das ausgefallene Produkt abgesaugt, mit Wasser gewaschen und getrocknet.

| | |
|---|---|
| Ausbeute: | 80 mg (62% d.Th.) |
| ESI-MS: | m/z = 354 (M+H)⁺ |

### Intermediat 6:

### (6-Chlorpyrimidin-4-yl)-(2,3-dihydroindol-1-yl)-methanon

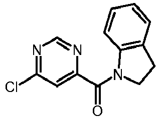

0.50 g (2.83 mmol) 6-Chlorpyrimidin-4-carbonsäurechlorid in 20 mL Dichlormethan wurden mit einem Eis/Ethanol-Bad gekühlt und mit 0.30 mL (2.68 mmol) 2,3-Dihydro-1*H-*indol versetzt. Es wurden 2.7 mL (2.7 mmol) einer 1 M Natronlauge zugetropft. Der Reaktionsansatz wurde 2 h unter Kühlung und 1 h bei RT gerührt. Anschließend gab man 50 mL einer wässrigen gesättigten Natriumhydrogencarbonat-Lösung hinzu. Nach 10 min Rühren wurde die organische Phase abgetrennt und mit Wasser und1 M Salzsäure gewaschen. Die organische Phase wurde über Magnesiumsulfat getrocknet, filtriert und i.vac. eingeengt.

| | |
|---|---|
| Ausbeute: | 570 mg (78% d.Th.) |
| ESI-MS: | m/z = 260/262 (M+H)⁺ |
| R_{f} (Kieselgel): | 0.59 (Laufmittel B) |

### Intermediat 7:

### (5-Chlor-2,3-dihydro-indol-1-yl)-(6-chlor-pyrimidin-4-yl)-methanon

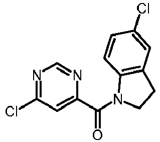

1.5 g (8.48 mmol) 6-Chlorpyrimidin-4-carbonsäurechlorid in 50 mL Dichlormethan wurden mit einem Eis/Ethanol-Bad gekühlt und mit 1.2 g (7.81 mmol) 5-Chlor-2,3-dihydro-1*H*-indol versetzt. Es wurden 7.9 mL (7.9 mmol) einer 1 M Natronlauge zugetropft. Der Reaktionsansatz wurde 2 h unter Kühlung und 1 h bei RT gerührt. Anschließend gab man 50 mL einer wässrigen gesättigten Natriumhydrogencarbonat-Lösung hinzu. Nach 10 min Rühren wurde die organische Phase abgetrennt und mit Wasser und 1 M Salzsäure gewaschen. Die organische Phase wurde über Magnesiumsulfat getrocknet, filtriert und i.vac. eingeengt.

| | |
|---|---|
| Ausbeute: | 2.0 g (80% d.Th.) |
| ESI-MS: | m/z = 294/296/298 (M+H)⁺ |
| R_{f} (Kieselgel): | 0.65 (Laufmittel B) |

### Intermediat 8:

### 6-Chlor-N-phenyl-N-(2,2,2-trifluorethyl)pyrimidin-4-carboxamid

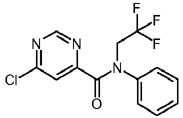

### Stufe 1: N-(2,2,2-Trifluorethyl)anilin

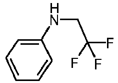

Zu 2.0 g (21.5 mmol) Anilin in 50 mL Xylol wurden 2.5 g (10.7 mmol) 2,2,2-Trifluorethyltrifluormethansulfonat hinzugefügt und der Reaktionsansatz wurde 2 Tage unter Rückfluss gekocht. Nach dem Abkülen wurde das Reaktionsgemisch filtriert, mit DIPE nachgewaschen und das Filtrat i.vac. eingeengt. Der Rückstand wurde mittels Flash-Chromatographie aufgereinigt. Die Produktfraktionen wurden vereinigt und i.vac. eingeengt.

| | |
|---|---|
| Ausbeute: | 220 mg (6% d.Th.) |
| R_{f} (Kieselgel): | 0.85 (Laufmittel B) |

### Stufe 2: 6-Chlor-N-phenyl-N-(2,2,2-trifluorethyl)pyrimidin-4-carboxamid

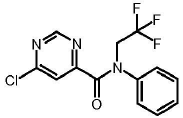

Zu 212 mg (1.2 mmol) 6-Chlorpyrimidin-4-carbonsäurechlorid in 20 mL Dichlormethan wurden unter Eis/Ethanol-Bad-Kühlung 200 mg (1.14 mmol) *N*-(2,2,2-Trifluorethyl)anilin und 1.14 mL (1.14 mmol) einer 1 M Natronlauge zugetropft. Der Ansatz wurde zunächst

2 h unter Kühlung und anschließend 1 h bei RT nachgerührt. Es wurden 50 mL einer gesättigten Natriumhydrogencarbonat-Lösung zugegeben und 10 min gerührt. Die organische Phase wurde abgetrennt, mit Wasser und 1 M Salzsäure gewaschen, über Natriumsulfat getrocknet, filtriert und i.vac. einrotiert.

| | |
|---|---|
| Ausbeute: | 280 mg (74% d.Th.) |
| ESI-MS: | m/z = 316/318 [M+H]⁺ |
| R_{f} (Kieselgel): | 0.83 (Laufmittel B) |

### Intermediat 9:

### N-Benzyl-6-chlor-N-(2,2,2-trifluorethyl)pyrimidin-4-carboxamid

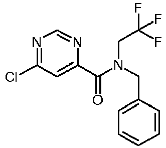

### Stufe 1: Benzyl-(2,2,2-trifluorethyl)-amin

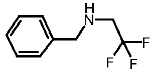

Zu 2.0 g (18.7 mmol) Benzylamin in 50 mL Xylol wurden 2.2 g (9.4 mmol) 2,2,2-Trifluorethyltrifluormethansulfonat hinzugefügt und der Reaktionsansatz über Nacht unter Rückfluss gekocht. Nach dem Abkülen wurde der ausgefallene Niederschlag abgesaugt, mit DIPE nachgewaschen und das Filtrat am Rotationsverdampfer eingeengt. Der Rückstand wurde mittels Flash-Chromatographie aufgereinigt. Die Produktfraktionen wurden vereinigt und i.vac. eingeengt.

| | |
|---|---|
| Ausbeute: | 2.3 g (65% d.Th.) |
| ESI-MS: | m/z = 190.0 [M+H]⁺ |

### Stufe 2: N-Benzyl-6-chlor-N-(2,2,2-trifluorethyl)pyrimidin-4-carboxamid

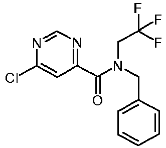

Zu 1.0 g (5.65 mmol) 6-Chlorpyrimidin-4-carbonsäurechlorid in 20 mL Dichlormethan wurden unter Eis/Ethanol-Bad-Kühlung 1.04 g (5.5 mmol) Benzyl-(2,2,2-trifluorethyl)-amin und 5.5 mL (5.5 mmol) einer 1 M Natronlauge zugetropft. Der Ansatz wurde zunächst 2 h unter Kühlung und anschließend 1 h bei RT nachgerührt. Es wurden 50 mL einer gesättigten Natriumhydrogencarbonat-Lösung zugegeben und 10 min gerührt. Die organische Phase wurde abgetrennt, mit Wasser und 1 M Salzsäure gewaschen, über Natriumsulfat getrocknet, filtriert und i.vac. eingeengt.

| | |
|---|---|
| Ausbeute: | 1.2 g (64% d.Th.) |
| ESI-MS: | m/z = 330/332 [M+H]⁺ |
| Rₜ (HPLC-MS): | 1.56 min (Methode C) |

### Intermediat 10:

### 6-Chlor-N-phenethyl-N-(2,2,2-trifluorethyl)pyrimidin-4-carboxamid

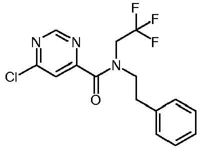

### Stufe 1: Phenethyl-(2,2,2-trifluorethyl)-amin

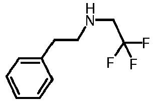

Zu 2.0 g (16.5 mmol) Phenethylamin in 50 mL Xylol wurden 1.9 g (8.3 mmol) 2,2,2-Trifluorethyltrifluormethansulfonat hinzugefügt und der Reaktionsansatz über Nacht unter Rückfluss gekocht. Nach dem Abkülen wurde der ausgefallene Niederschlag abgesaugt, mit DIPE nachgewaschen und das Filtrat i.vac. eingeengt. Der Rückstand wurde mittels Flash-Chromatographie aufgereinigt. Die Produktfraktionen wurden vereinigt und i.vac. eingeengt.

| | |
|---|---|
| Ausbeute: | 0.8 g (24% d.Th.) |
| ESI-MS: | m/z = 204 [M+H]⁺ |
| Rₜ (HPLC-MS): | 1.61 min (Methode C) |

### Stufe 2: 6-Chlor-N-phenethyl-N-(2,2,2-trifluorethyl)pyrimidin-4-carboxamid

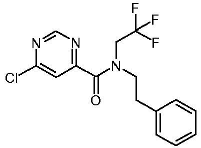

Zu 0.77 g (4.33 mmol) 6-Chlorpyrimidin-4-carbonsäurechlorid in 20 mL Dichlormethan wurden unter Eis/Ethanol-Bad-Kühlung 0.80 g (3.94 mmol) Phenethyl-(2,2,2-trifluorethyl)-amin und 3.9 mL (3.9 mmol) einer 1 M Natronlauge zugetropft. Der Ansatz wurde zunächst 2 h unter Kühlung und anschließend 1 h bei RT nachgerührt. Es wurden 50 mL einer gesättigten Natriumhydrogencarbonat-Lösung zugegeben und 10 min gerührt. Die organische Phase wurde abgetrennt, mit Wasser und 1 M Salzsäure gewaschen, über Natriumsulfat getrocknet, filtriert und i.vac. einrotiert.

| | |
|---|---|
| Ausbeute: | 1.0 g (67% d.Th.) |
| ESI-MS: | m/z = 344/346 [M+H]⁺ |
| Rₜ (HPLC-MS): | 1.58 min (Methode C) |

### Intermediat 11:

### 6-(2'-Oxo-1,1',2',3-tetrahydrospiro[inden-2,3'-pyrrolo[2,3-b]pyridin]-5-ylamino)pyrimidin-4-carbonsäure-Hydrochlorid

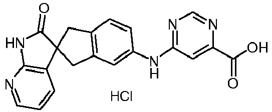

### Stufe 1: 6-(2'-Oxo-1,1',2',3-tetrahydrospiro[inden-2,3'-pyrrolo[2,3-b]pyridin]-5-yl-amino)pyrimidin-4-carbonsäureethylester

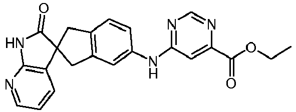

Zu 0.16 g (0.60 mmol) 5-Amino-1,3-dihydrospiro[inden-2,3'-pyrrolo[2,3-b]pyridin]-2'(1'H)-on in 1.0 mL 2-Propanol wurden 0.10 g (0.55 mmol) 6-Chlorpyrimidin-4-carbonsäure-ethylester und 13 µL (50 µmol) 4M HCl gegeben. Das Reaktionsgemisch wurde 2 h unter Rückfluss gekocht, dann auf RT abgekühlt und der entstandene Feststoff abfiltriert und getrocknet.

| | |
|---|---|
| Ausbeute: | 165 mg (54% d.Th.) |
| ESI-MS: | m/z = 402 (M+H)⁺ |

### Stufe 2: 6-(2'-oxo-1,1',2',3-tetrahydrospiro[inden-2,3'-pyrrolo[2,3-b]pyridin]-5-yl-amino)pyrimidin-4-carbonsäure-Hydrochlorid

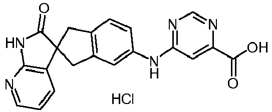

Zu 1.80 g (4.48 mmol) 6-(2'-Oxo-1,1',2',3-tetrahydrospiro[inden-2,3'-pyrrolo[2,3-b]pyridin]-5-ylamino)pyrimidin-4-carbonsäure ethyl ester in 5.0 mL Ethanol wurden 5.0 mL (5.0 mmol) einer 1 M wässrigen Lithiumhydroxid Lösung gegeben und über Nacht bei RT gerührt.Das Reaktionsgemisch wurde mit 1.3 mL einer 4M wässrigen HCl Lösung angesäuert und der entstandene Niederschlag wurde abgesaugt und getrocknet.

| | |
|---|---|
| Ausbeute: | 1.5 g (83% d.Th.) |
| ESI-MS: | m/z = 372 [M-H]⁻ |
| Rₜ (HPLC-MS): | 1.85 min (Methode E) |

### Intermediat 12:

### 4-(2'-Oxo-1,1',2',3-tetrahydrospiro[inden-2,3'-pyrrolo[2,3-b]pyridin]-5-ylamino)-pyridin-2-carbonsäure

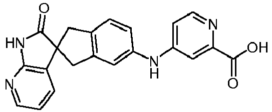

Zu 0.20 g (0.78 mmol) 5-Amino-1,3-dihydrospiro[inden-2,3'-pyrrolo[2,3-b]pyridin]-2'(1'H)-on in 5.0 mL 2-Propanol wurden 0.17 g (0.79 mmol) 4-Brom-pyridin-2-carbonsäuremethyl ester und 17 µL (70 µmol) einer 4M wässrigen Salzsäure Lösung gegeben. Das Reaktionsgemisch wurde 2h unter Rückfluss gekocht, dann auf RT abgekühlt und eingeengt. Der Rückstand wurde in THF aufgenommen und mit 0.50 mL (2.0 mmol) einer 4M wässriger NaOH Lösung versetzt und über Nacht bei RT gerührt. Das Reaktionsgemisch wurde mit Wasser verdünnt, mit 0.5 mL konz. HCl versetzt und das Lösungsmittel abgezogen. Das ausgefallene Produkt wurde abgesaugt und getrocknet.

| | |
|---|---|
| Ausbeute: | 0.23 g (80% d.Th.) |
| ESI-MS: | m/z = 373 [M+H]⁺ |
| Rₜ (HPLC-MS): | 0.96 min (Methode C) |

### Intermediat 13:

### 4-(2'-Oxo-1,1',2',3-tetrahydrospiro[inden-2,3'-pyrrolo[2,3-b]pyridin1-5-ylamino)-pyridin-3-carbonsäure

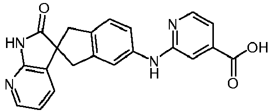

Zu 0.20 g (0.78 mmol) 5-Amino-1,3-dihydrospiro[inden-2,3'-pyrrolo[2,3-b]pyridin]-2'(1'H)-on in 5.0 mL 2-Propanol wurden0.13 g (0.92 mmol) 2-Fluorpyridin-3-carbonsäure und 20 µL (0.08 mmol) einer 4M wässrigen HCl Lösung gegeben. Das Reaktionsgemisch wurde 2 h unter Rückfluss gekocht, dann auf RT abgekühlt und der entstandene Feststoff abfiltriert und getrocknet.

| | |
|---|---|
| Ausbeute: | 110 mg (38% d.Th.) |
| ESI-MS: | m/z = 373 [M+H]⁺ |
| Rₜ (HPLC-MS): | 1.01 min (Methode C) |

### Intermediat 14:

### 4,4-Dimethyl-4,5,6,7-tetrahydro-thieno[2,3-c]pyridin

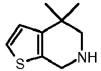

### Stufe 1: Methylen-(2-methyl-2-thiophen-3-yl-propyl)-amin

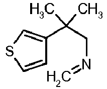

5.8 g (37 mmol) 2-Methyl-2-thiophen-3-yl-propylamin und 3.6 mL (44 mmol) Formaldehyd wurden zusammen mit 2.0 g Molsieb (4Ǻ Pulver) über Nacht bei RT gerührt. Das Reaktionsgemisch wurde filtriert und das Filtrat wurde zur Trockene einrotiert.

| | |
|---|---|
| Ausbeute: | 6.0 g (97% d. Th) |
| ESI-MS: | m/z = 168 (M+H)⁺ |

### Stufe 2: 4,4-Dimethyl-4,5,6,7-tetrahydro-thieno[2,3-c]pyridin

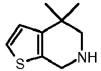

6.0 g (36 mmol) Methylen-(2-methyl-2-thiophen-3-yl-propyl)-amin, 11 mL (45 mmol) 4M HCl und 12 mL (0.14 mol) konz. HCl wurden über das Wochenende bei RT gerührt. Das Reaktionsgemisch wurde mit einer 4M Natriumhydroxid-Lösung alkalisch gestellt. Der ausgefallene Niederschlag wurde abgesaugt, mit Wasser gewaschen und getrocknet. Die Substanz wurde über Alox gereinigt. Die Produkt enthaltenden Fraktionen wurden vereint und zur Trockene einrotiert.

| | |
|---|---|
| Ausbeute: | 0.74 g (12% d. Th) |
| Rₜ (HPLC-MS): | 1.24 min (Methode K) |

### Intermediat 15:

### 7,7-Dimethyl-4,5,6,7-tetrahydro-1H-pyrazolo[4,3-c]pyridin Dihydrochlorid

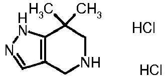

Zu 1.6 g (6.1 mmol) 7,7-Dimethyl-1,4,6,7-tetrahydro-pyrazolo[4,3-c]pyridin-5-carbonsäure-tert-butylester in 15 mL Dichlormethan wurden bei 0°C 5 mL Trifluoressigsäure gegeben und 2 h bei RT gerührt. Das Reaktionsgemisch wurde eingeengt. Der Rückstand wurde in Ethanol aufgenommen und mit 12 mL (15 mmol) 1.25 M ethanolischer HCl versetzt und dann eingeengt. Der Rückstand wurde mit Ethanol verrieben und der Feststofff abgesaugt und getrocknet.

| | |
|---|---|
| Ausbeute: | 1.2 g (92% d.Th.) |
| ESI-MS: | m/z = 152 [M+H]⁺ |

### Intermediate 16:

### (6-Chlor-pyrimidin-4-yl)-(3-ethyl-6-fluor-2,3-dihydro-indol-1-yl)-methanon

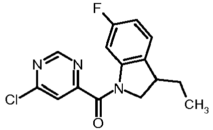

### Stufe 1: 2-But-1-ynyl-5-fluor-phenylamin

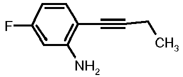

Es wurden 160 mL THF im Kolben vorgelegt und mit Argon entgast. Dann wurden 10 g (45 mmol) 5-Fluor-2-iod-anilin, 1.5 g (2.1 mmol) PdCl₂(PPh₃)₂ und 0.40 g (2.1 mmol) Kupferiodid zugegeben und der Kolben mit Argon gespült. Dann wurden 18 mL (0.13 mol) Triethylamin zugegeben und 3.0 g (55 mmol) 1-Butin (gasförmig) durch die Lösung geleitet. Das Reaktionsgemisch wurde 3h bei RT gerührt, anschließend mit Diethylether verdünnt, über Kieselgur filtriert und eingeengt.

| | |
|---|---|
| Ausbeute: | 6.90 g (quantitativ) |

### Stufe 2: 2-Ethyl-6-fluor-1H-indol

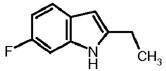

Die Reaktion wurde unter Argon-Atmosphere durchgeführt.Zu 30 g (0.27 mol) Kalium-tert.butylat in 120 mL N-Methyl-2-pyrrolidinon wurden 21 g (0.13 mol) 2-But-1-ynyl-5-fluor-phenylamin zugegeben und 4h bei RT gerührt. Das Reaktionsgemisch wurde mit Wasser versetzt und mit Diethylether extrahiert. Die organische Phase wurde getrocknet und eingeengt. Der Rückstand wurde über Kieselgel gereinigt.

| | |
|---|---|
| Ausbeute: | 16.25 g (78% d.Th.) |
| ESI-MS: | m/z = 164 [M+H]⁺ |

### Stufe 3: 2-Ethyl-6-fluor-2,3-dihydro-1H-indol

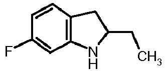

Zu 1.6 g (10 mmol) 2-Ethyl-6-fluor-1H-indol in 20 mL konz. Essigsäure wurden 2.7 g (40 mmol) Natriumcyanoborhydrid gegeben und 1h bei RT gerührt. Das Reaktionsgemisch wurde eingeengt. Der Rückstand wurde mit 20 mL einer 4N HCl-Lösung versetzt und 1 h bei RT gerührt.Dann wurde unter Eiskühlung langsam 45 mL einer 4N Natriumhydroxid-Lösung zugegeben und mit Essigester extrahiert. Die organische Phase wurde über Natriumsulfat getrocknet und eingeengt.

| | |
|---|---|
| Ausbeute: | 2.5 g (quantitativ) |
| ESI-MS: | m/z = 166 [M+H]⁺ |
| Rₜ (HPLC-MS): | 1.32 min (Methode C) |

### Stufe 4: (6-Chlor-pyrimidin-4-yl)-(2-ethyl-6-fluor-2,3-dihydro-indol-1-yl)-methanon

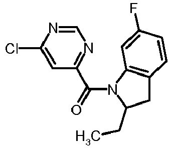

Zu 1.8 g (10 mmol) 6-Chlor-pyrimidin-4-carbonsäurechlorid in 25 mL Dichlormethan wurden bei 0°C ein Gemisch aus 2.5 g (10 mmol) 2-Ethyl-6-fluor-2,3-dihydro-1H-indol in 25 mL Dichlormethan zugegeben und danach 10 mL (10 mmol) einer 1 M Natriumhydroxid-Lösung zugetropft. Das Reaktionsgemisch wurde 30 min bei 0°C gerührt und dann 1 h bei RT. Die organische Phase wurde abgetrennt und eingeengt. Der Rückstand wurde über Kieselgel gereinigt.

| | |
|---|---|
| Ausbeute: | 2.5 g (82% d.Th.) |
| ESI-MS: | m/z = 306 [M+H]⁺ |
| Rₜ (HPLC-MS): | 1.66 min (Methode C) |

### Intermediate 17:

### (6-Chlor-pyrimidin-4-yl)-(2-propyl-2,3-dihydro-indol-1-yl)-methanon

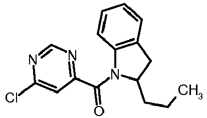

### Stufe 1: 2-Pent-1-ynyl-phenylamin

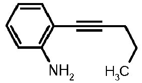

Es wurden 20 mL THF im Kolben vorgelegt und mit Argon entgast. Anschließend wurden 2.3 g (10 mmol) 2-lod-anilin, 0.72 g (1.0 mmol) PdCl₂(PPh₃)₂ und 0.20 g (1.0 mmol) Kupferiodid zugegeben und der Kolben mit Argon gespült. Dann wurden 4.2 mL (30 mmol) Triethylamin und 1.5 mL (15 mmol) 1-Pentin zugegeben. Das Reaktionsgemisch wurde 3 h bei RT gerührt, mit Diethylether verdünnt, über Kieselgur filtriert und eingeengt.

| | |
|---|---|
| Ausbeute: | 2.7 g (quantitativ) |

### Stufe 2: 2-Propyl-1H-indol

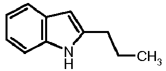

Die Reaktion wurde unter Argon-Atmosphere durchgeführt.Zu 2.3 g (20 mmol) Kalium-tert.butylat in 50 mL N-Methyl-2-pyrrolidinon wurden 2.7 g (10 mmol) 2-Pent-1-ynyl-phenylamin zugegeben und über Nacht bei RT gerührt. Das Reaktionsgemisch wurde mit Wasser versetzt und mit Essigester extrahiert. Die organische Phase wurde getrocknet und eingeengt. Der Rückstand wurde über Kieselgel gereinigt.

| | |
|---|---|
| Ausbeute: | 1.2 g (75% d.Th.) |
| ESI-MS: | m/z = 160 [M+H]⁺ |
| Rₜ (HPLC-MS): | 1.60 min (Methode C) |

### Stufe 3: 2-Propyl-2,3-dihydro-1H-indol

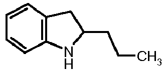

Zu 0.75 g (4.7 mmol) 2-Propyl-1H-indol in 10 mL konz. Essigsäure wurden 1.3 g (20 mmol) Natriumcyanoborhydrid gegeben und 5 h bei RT gerührt.Das Reaktionsgemisch wurde eingeengt. Der Rückstand wurde mit 20 mL einer 4N HCl-Lösung versetzt und 1 h bei RT gerührt.Dann wurde unter Eiskühlung langsam 45 mL einer 4N Natriumhydroxid-Lösung zugegeben und mit Essigester extrahiert. Die organische Phase wurde über Natriumsulfat getrocknet und eingeengt.

| | |
|---|---|
| Ausbeute: | 0.76 g (quantitativ) |
| ESI-MS: | m/z = 162 [M+H]⁺ |
| Rₜ (HPLC-MS): | 1.50 min (Methode C) |

### Stufe 4: (6-Chlor-pyrimidin-4-yl)-(2-propyl-2,3-dihydro-indol-1-yl)-methanon

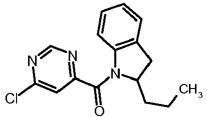

Zu 0.88 g (5.0 mmol) 6-Chlor-pyrimidin-4-carbonsäurechlorid in 15 mL Dichlormethan wurden bei 0°C ein Gemisch aus 0.76 g (4.7 mmol) 2-Propyl-2,3-dihydro-1H-indol in 15 mL Dichlormethan zugegeben und danach 4.7 mL (4.7 mmol) einer 1 M Natriumhydroxid-Lösung zugetropft. Das Reaktionsgemisch wurde 30 min bei 0°C gerührt und dann 1 h bei RT. Die organische Phase wurde abgetrennt und eingeengt. Der Rückstand wurde über Kieselgel gereinigt.

| | |
|---|---|
| Ausbeute: | 0.40 g (26% d.Th.) |

| | |
|---|---|
| ESI-MS: | m/z = 302 [M+H]⁺ |
| Rₜ (HPLC-MS): | 1.71 min (Methode C) |

### Intermediat 18:

### Spiro[piperidin-4,4'-pyrido[2,3-d][1,3]oxazin]-2'(1'H)-on Hydrochlorid

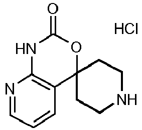

### Stufe 1: (6-Chlor-pyridin-2-yl)-carbaminsäure-tert-butylester

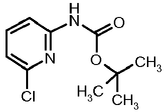

Unter einer Stickstoffatmosphäre wurden zu 17.4 g (0.14 mol) 6-Chlor-pyridin-2-ylamin und 300 mL (0.30 mol) einer 1 molaren Natriumhexamethyldisilazidlösung in THF in 200 mL THF eine Lösung aus 32.7 g (0.15 mol) BOC Anhydrid in 100 mL THF bei RT zugetropft. Der Reaktionsansatz wurde über Nacht bei RT gerührt und im Anschluß eingeengt. Der Rückstand wurde zwischen EtOAc und 1 N wässriger Salzsäurelösung verrührt. Die organische Phase wurde abgetrennt und die wässrige Phase wurde nochmals mit EtOAc extrahiert. Die vereinten organischen Phasen wurden mit 300 mL gesättigter Natriumhydrogencarbonatlösung gewaschen, getrocknet und eingeengt. Der Rückstand wurde aus EtOH umkristallisiert, der Feststoff abgesaugt und getrocknet.

| | |
|---|---|
| Ausbeute: | 29.2 g (95% d. Th.) |
| ESI-MS: | m/z = 228 (M+) |
| Rₜ(HPLC): | 1.70 min (Methode C) |

### Stufe 2: Benzyl 7'-chlor-2'-oxo-1',2'-dihydrospiro[piperidin-4,4'-pyrido[2,3d][1,3]-oxazin]-1-carboxylat

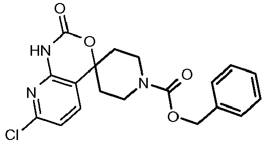

Unter einer Stickstoffatmosphäre wurden 26 mL (1734 mmol) N,N,N,N-Tetramethylenethylendiamin in 180 mL THF auf -20°C gekühlt und mit 70 mL (175 mmol) einer 2.5 molaren Butyllithiumlösung innerhalb von 10 min versetzt. Nach 30 minütigem Rühren wurde der Reaktionsansatz auf -78°C gekühlt und bei dieser Temperatur wurden 17.8 g (78.0 mmol) (6-Chlor-pyridin-2-yl)-carbaminsäure-tert-butylester in 120 mL THF innerhalb von 20 min zugetropft. Der Reaktionsansatz wurde 2.5 h bei -78°C nachgerührt und anschließend mit 27.2 g (116.7 mmol) 4-Oxo-piperidin-1-carbonsäurebenzyl ester in 60 mL THF innerhalb von 10 min versetzt. Nach einer weiteren Stunde Rühren bei -78°C wurde der Reaktionsansatz zunächst auf RT erwärmt und dann 18 h bei 40°C gerührt. Dann wurde durch Zutropfen von 150 mL gesättigter Natriumhydrogencarbonatlösung der Reaktionsansatz zersetzt. Anschließend wurde der Reaktionsansatz mehrmals mit DCM extrahiert. Die vereinten organischen Phasen wurden mit Wasser gewaschen, getrocknet und eingeengt. Der Rückstand wurde mit PE/EtOAc 1/1 verrieben, der entstandene Niederschlag abgesaugt, mit PE/ETOAc 1/1 gewaschen und getrocknet.

| | |
|---|---|
| Ausbeute: | 16.4 g (54% d. Th.) |
| ESI-MS: | m/z = 388 (M+H)⁺ |
| Rₜ(HPLC): | 1.57 min (Methode C) |

### Stufe 3: Spiro[piperidin-4,4'-pyrido[2,3-d][1,3]oxazin]-2'(1'H)-on Hydrochlorid

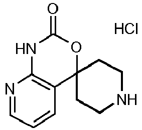

16.4 g (42.4 mmol) Benzyl 7'-chlor-2'-oxo-1',2'-dihydrospiro[piperidin-4,4'-pyrido[2,3d][1,3]-oxazin]-1-carboxylat und 2.00 g Palladium(Pd/C 10%) in 500 mL EtOH wurden 6 h bei RT in einer Wasserstoff-Atmosphäre hydriert. Dann wurden 1.0 g Palladium (Pd/C 10%) zusätzlich zugefügt und weitere 3 h bei RT in einer Wasserstoff-Atmosphäre hydriert. Nach Filtration des Reaktionsgemisches wurde das Lösungsmittel im Vakuum entfernt. Der Rückstand wurde mit EtOH verrieben, der ausgefallene Niederschlag abgesaugt, mit EtOH nachgewaschen und getrocknet.

| | |
|---|---|
| Ausbeute: | 5.40 g (50% d. Th.) |
| ESI-MS: | m/z = 220 (M+H)⁺ |
| Rₜ(HPLC): | 0.90 min (Methode C) |

### Intermediat 19:

### (6-Chlor-pyrimidin-4-yl)-(5-fluor-2,3-dihydro-indol-1-yl)-methanon

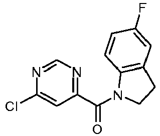

0.92 g (4.9 mmol) 6-Chlor-pyrimidin-4-carbonsäurechlorid in 40 mL DCM wurden in einem Eis/Aceton-Bad abgekühlt und mit 0.67 g (4.9 mmol) 5-Fluor-2,3-dihydro-1H-indol versetzt. Weiterhin wurden noch 5 mL (5.0 mmol) einer 1 N wäßrigen Natronlaugelösung zugetropft und 1 h unter Kühlen gerührt. Dann wurden 50 mL einer gesättigten Natriumhydrogencarbonatlösung zugegeben und 10 min weitergerührt. Die organische Phase wurde abgetrennt, mit 1 N wäßriger Salzsäurelösung und mit Wasser ausgeschüttelt, getrocknet und eingeengt.

| | |
|---|---|
| Ausbeute: | 0.81 mg (60% d. Th.) |
| ESI-MS: | m/z = 278 (M+H)⁺ |
| Rₜ (HPLC-MS): | 1.50 min (Methode C) |

### Intermediat 20:

### 2,3-Dihydro-1H-indol-5-ol

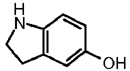

Unter einer Stickstoffatmosphäre wurden zu 0.60 g (4.5 mmol) 5-Hydroxyindol in 5.0 mL Eisessig 0.34 g Natriumcyanoborhydrid portionsweise zugegeben und 60 min bei RT gerührt. Dann wurde der Reaktionsansatz auf eine 4N wässrige Natronlaugelösung gegossen und mit EtOAc extrahiert. Die vereinigten organischen Phasen wurden mehrmals mit gesättigter Natriumhydrogencarbonatlösung gewaschen, über Natriumsulfat getrocknet und eingeengt.

| | |
|---|---|
| Ausbeute: | 265 mg (35% d. Th.) |
| ESI-MS: | m/z = 136 (M+H)⁺ |
| Rₜ (HPLC-MS): | 0.38 min (Methode C) |

### Intermediat 21:

### 6-Fluor-2,3-dihydro-1H-indol

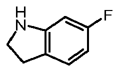

Unter einer Stickstoffatmosphäre wurden zu 0.54 g (4.0 mmol) 6-Fluorindol in 5.0 mL Eisessig 0.29 g (4.6 mmol) Natriumcyanoborhydrid portionsweise zugegeben und 30 min bei RT gerührt. Anschließend wurde der Reaktionsansatz auf eine 4N wässrige Natron-laugelösung gegossen und mit EtOAc extrahiert. Die vereinigten organischen Phasen wurden mehrmals mit gesättigter Natriumhydrogencarbonatlösung gewaschen, über Natriumsulfat getrocknet und eingeengt.

| | |
|---|---|
| Ausbeute: | 0.56 g (97% d. Th.) |
| ESI-MS: | m/z = 138 (M+H)⁺ |
| Rₜ (HPLC-MS): | 0.74 min (Methode C) |

### Intermediat 22:

### (2-Chlor-pyridin-4-yl)-(4,5-difluor-2,3-dihydro-indol-1-yl)-methanon

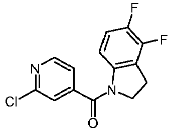

0.50 g (3.2 mmol) 2-Chlorisonicotinsäure, 0.63 mg (3.3 mmol) 4,5-Difluorindolin-Hydrochlorid, 0.91 mL (6.5 mmol) TEA und 1.1 g (3.4 mmol) TBTU in 10 mL DMF wurden über Nacht bei RT gerührt. Anschließend wurde der Reaktionsansatz auf 200 mL 15%ige Kaliumcarbonatlösung gegossen, der ausgefallene Niederschlag abgesaugt, mit Wasser nachgewaschen und getrocknet.

| | |
|---|---|
| Ausbeute: | 0.90 g (96% d.Th.) |
| ESI-MS: | m/z = 295 (M+H)⁺ |
| Rₜ (HPLC-MS): | 3.80 min (Methode E) |

### Intermediat 23:

### 5,6-Difluor-2,3-dihydro-1H-indol

>

Unter einer Argonatmosphäre wurden 0.30 g (1.8 mmol) 5,6-Difluoroxindol in 10 mL THF gelöst und 3.0 mL einer 1 molaren Boranlösung in THF zugetropft. Anschließend wurde der Reaktionsansatz 2 h auf 70°C erwärmt und dann abgekühlt. Nach dem Versetzen mit 3 mL MeOH wurden noch 5 mL einer 4N wässrigen Salzsäurelösung zugefügt und 1 h unter Rückfluss gekocht. Die organische Phase wurde eingeengt, die wässrige Phase wurde mit DCM gewaschen und anschließend mit einer 4N wässrigen Natronlaugelösung alkalisch gestellt und mehrmals mit EtOAc extrahiert. Die vereinigten organischen Phasen wurden mit Natriumsulfat getrocknet, filtriert und eingeengt.

| | |
|---|---|
| Ausbeute: | 160 mg (47% d.Th.) |
| ESI-MS: | m/z = 156 (M+H)⁺ |
| Rₜ (HPLC-MS): | 0.73 min (Methode C) |

### Intermediat 24:

### 3-Methyl-2,3-dihydro-1H-indol

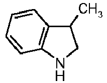

Unter einer Stickstoffatmosphäre wurden zu 1.0 g (7.6 mmol) 3-Methylindol in 5.0 mL Eisessig 0.58 g (9.2 mmol) Natriumcyanoborhydrid portionsweise zugegeben und 60 min bei RT gerührt. Dann wurde der Reaktionsansatz auf eine 4N wässrige Natronlaugelösung gegossen und mit EtOAc extrahiert. Die vereinigten organischen Phasen wurden mehrmals mit gesättigter Natriumhydrogencarbonatlösung gewaschen, über Natriumsulfat getrocknet und eingeengt.

| | |
|---|---|
| Ausbeute: | 1.2 g (83% d. Th.) |
| ESI-MS: | m/z = 134 (M+H)⁺ |
| Rₜ (HPLC-MS): | 0.81 min (Methode C) |

### Intermediat 25:

### 2,3-Dihydro-1H-pyrrol[3,2-c]pyridin

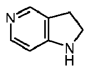

### Stufe 1: (E)-3-(2-(Dimethylamino)vinyl)-4-nitropyridin-1-oxid

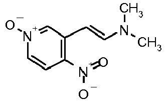

7.7 g (50 mmol) 3-Methyl-4-nitropyridin-N-oxid und 8.0 mL (60 mmol) Dimethylformamiddimethylacetal in 1.0 mL DMF wurden bei 120°C für 4 h gerührt. Anschließend wurde der Reaktionsansatz einrotiert, der Rückstand mit EtOH verrieben und aus Diethylether ausgefällt. Der ausgefallene Niederschlag wurde abgesaugt, mit Diethylether nachgewaschen und getrocknet.

| | |
|---|---|
| Ausbeute: | 8.6 g (82 % d. Th.) |
| ESI-MS: | m/z = 441 (2 M+Na)⁺ |
| R_{f}: | 0.4 (Kieselgel, EtOAc/EtOH 8:2) |

### Stufe 2: 1H-Pyrrol[3,2-c]pyridin

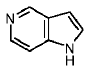

2.1 g (10 mmol) (E)-3-(2-(Dimethylamino)vinyl)-4-nitropyridin-1-oxid in 25 mL EtOH wurden mit 3.2 g Raney-Nickel (50%in Wasser) versetzt und in einer 1 bar Wasserstoff-atmosphäre zunächst 3 h 15 min bei RT und anschließend 2 h bei 40°C hydriert. Der Katalysator wurde abgesaugt und das Filtrat wurde mit Aktivkohle gereinigt. Eine weitere Reinigung erfolgte mittels Flash-Chromatographie.

| | |
|---|---|
| Ausbeute: | 0.90 g (76% d. Th.) |
| EI-MS: | m/z = 118 (M)⁺ |
| R_{f}: | 0.5 (Kieselgel, DCM/MeOH 2:3) |

### Stufe 3: 2,3-Dihydro-1H-pyrro[3,2-c]pyridin

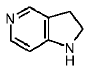

1.5 g (12.7 mmol) 1 H-Pyrrol[3,2-c]pyridin in 70 mL EtOH wurden mit 0.75 g Raney-Nickel versetzt und in einer 3 bar Wasserstoffatmosphäre 3 Tage bei 70°C hydriert. Der Katalysator wurde abgesaugt und das Filtrat wurde eingeengt. Der Rückstand wurde mittels Flash-Chromatographie gereinigt. Die Produkt enthaltenden Fraktionen wurden vereinigt und eingeengt.

| | |
|---|---|
| Ausbeute: | 0.62 g (41% d. Th.) |
| ESI-MS: | m/z = 121 (M+H)⁺ |
| R_{f}: | 0.12 (Kieselgel, DCM/MeOH/NH₄OH 80:20:2) |

### Intermediat 26:

### (6-Chlor-pyrimidin-4-yl)-(2-ethyl-2,3-dihydro-indol-1-yl)-methanon

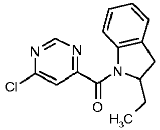

### Stufe 1: 1-Benzolsulfonyl-1H-indol

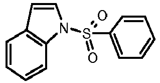

Zu 2.0 g (17.1 mmol) Indol in 30 mL THF wurden unter Eisbadkühlung 0.89 g (22.2 mmol) Natriumhydrid (60%ig) zugefügt und 15 min bei dieser Temperatur gerührt. Anschließend wurden 2.2 mL (17.0 mmol) Benzolsulfonsäurechlorid zugegeben und bei RT über Nacht gerührt. Das Reaktionsgemisch wurde mit Wasser und EtOAc versetzt und mehrmals mit EtOAc extrahiert. Die vereinten organischen Phasen wurden über Natriumsulfat getrocknet und eingeengt.

| | |
|---|---|
| Ausbeute: | 4.6 g (quantitativ) |
| ESI-MS: | m/z = 275 (M+H)⁺ |

### Stufe 2: 1-Benzolsulfonyl-2-ethyl-1H-indol

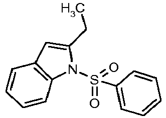

Unter einer Argonatmosphäre wurden zu 2.8 g (11 mmol) 1-Benzolsulfonyl-1H-indol in 25 mL THF bei -78°C langsam 6.7 mL (12 mmol) einer 1.8 molaren Lithiumdiisopropylamidlösung in THF zugetropft. Danach wurde die Kühlung entfernt, der Reaktionsansatz auf RT erwärmt und eine weitere Stunde bei RT nachgerührt. Der Reaktionsansatz wurde wieder auf -78°C abgekühlt und mit 1.0 mL (12 mmol) Jodethan versetzt. Anschließend wurde der Reaktionsansatz wieder auf RT erwärmt und über Nacht nachgerührt. Da die Umsetzung unvollständig war, wurde der Reaktionsansatz nochmals auf -78°C abgekühlt, mit 3.3 mL (6.0 mmol) einer 1.8 molaren Lithiumdiisopropylamidlösung in THF versetzt und nach beendeter Zugabe auf RT erwärmt. Anschließend wurde der Reaktionsansatz auf Eiswasser gegossen und mit EtOAc extrahiert. Die organische Phase wurde über Natriumsulfat getrocknet und eingeengt. Der Rückstand wurde mittels Flash-Chromatographie gereinigt. Die Produkt enthaltenden Fraktionen wurden vereinigt, eingeengt und im HV getrocknet.

| | |
|---|---|
| Ausbeute: | 0.75 g (24% d. Th.) |
| R_{f}: | 0.61 (Kieselgel, PE/EtOAc 3/1) |

### Stufe 3: 2-Ethyl-1H-indol

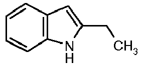

1.2 g (4.2 mmol) 1-Benzolsulfonyl-2-ethyl-1H-indol in 10 mL EtOH wurden mit 5 mL einer (20 mmol) 4 N wässrigen Natronlauge versetzt und 8h unter Rückfluss gekocht. Anschließend wurde das Lösungsmittel am Rotationsverdampfer entfernt und der Rückstand mit Eiswasser verdünnt. Nach dem Ansäuern mit halbkonzentrierter wässriger Salzsäure wurde die ausgefallene Schmiere mit Ethylacetat extrahiert. Die organische Phase wurde über Natriumsulfat getrocknet, abfiltriert, eingeengt und getrocknet.

| | |
|---|---|
| Ausbeute: | 0.66 g (quantitativ) |
| ESI-MS: | m/z = 146 (M+H)⁺ |

### Stufe 4: 2-Ethyl-2,3-dihydro-1H-indol

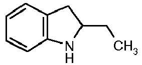

0.66 g (4.2 mmol) 2-Ethyl-1 H-indol in 10 mL Essigsäure wurden mit 1.3 g (20 mmol) Natriumcyanoborhydrid versetzt und einen Tag bei RT gerührt. Der Reaktionsansatz wurde am Rotationsverdampfer eingeengt, mit 20 mL wässriger 4N Salzsäure versetzt und 1 h bei RT gerührt. Unter Eiskühlung wurden anschließend 45 mL einer wässrigen 4N Natriumhydroxid-Lösung hinzu gefügt und mit Ethylacetat extrahiert. Die organische Phase wurde über Natriumsulfat getrocknet, filtriert, eingeengt und der Rückstand im Vakuum getrocknet.

| | |
|---|---|
| Ausbeute: | 0.80 g (quantitativ) |

### Stufe 5: (6-Chlor-pyrimidin-4-yl)-(2-ethyl-2,3-dihydro-indol-1-yl)-methanon

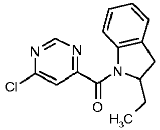

0.80 g (4.5 mmol) 6-Chlorpyrimidin-4-carbonsäurechlorid in 30 mL DCM wurden in einem Eis/Ethanoll-Bad abgekühlt und mit 0.62 g (4.2 mmol) 2-Ethyl-2,3-dihydro-1H-indol in DCM und 4.7 mL (4.7 mmol) einer 1 molaren wässrigen Natronlaugelösung versetzt. Anschließend wurde 30 min unter Kühlen und 1h bei RT nachgerührt. Nach Zugabe von 50 mL einer gesättigten Natriumhydrogencarbonat-Lösung wurden weitere 10 min gerührt. Die organische Phase wurde abgetrennt, mit Wasser gewaschen und eingeengt. Der Rückstand wurde mittels Flash-Chromatographie gereinigt. Die Produkt enthaltenden Fraktionen wurden vereinigt, eingeengt und getrocknet.

| | |
|---|---|
| Ausbeute: | 0.25 g (19% d. Th.) |
| R_{f}: | 0.54 (Kieselgel, PE/EtOAc 4/1) |

### Intermediat 27:

### 6-Chlor-2,3-dihydro-1H-indol

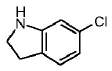

Unter einer Stickstoffatmosphäre wurden zu 1.0 g (6.6 mmol) 5-Hydroxyindol in 5.0 mL Eisessig 0.50 g (7.9 mmol) Natriumcyanoborhydrid portionsweise zugegeben und 60 min bei RT gerührt. Dann wurde der Reaktionsansatz auf eine 4N wässrige Natronlauge gegossen und mit EtOAc extrahiert. Die vereinigten organischen Phasen wurden mehrmals mit gesättigter Natriumhydrogencarbonat-Lösung gewaschen, über Natrium-sulfat getrocknet und eingeengt.

| | |
|---|---|
| Ausbeute: | 1.1 g (87% d. Th.) |
| Rₜ (HPLC-MS): | 1.24 min (Methode C) |

### Intermediat 28:

### 2,3-Dihydro-1H-indol-6-ol

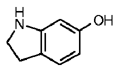

0.25 g (1.7 mmol) 6-Methoxy-2,3-dihydro-1H-indol und 2.0 g (16.8 mmol) Pyridin-Hydrochlorid wurden zusammengegeben und ca. 20 min auf 350°C erhitzt. Dann wurde der Reaktionsansatz in Acetonitril/DMF gelöst und mittels präparativer HPLC-MS gereinigt. Die Produkt enthaltenden Fraktionen wurden vereinigt und gefriergetrocknet.

| | |
|---|---|
| Ausbeute: | 50 mg (22% d. Th.) |
| Rₜ (HPLC-MS): | 0.94 min (Methode O) |

### Intermediat 29:

### (S)-2-(3,5-difluorphenyl)-5,5-dimethylpiperidin

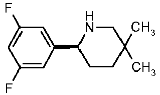

Zu 0.48 g (2.0 mmol) (S)-6-(3,5-Difluorphenyl)-3,3-dimethylpiperidin-2-on in 10 mL THF wurden unter Eiskühlung 7.0 mL (7.0 mmol) einer 1 molaren Di-isobutyl-aluminiumhdyrid-Lösung in Toluol zugefügt und 20 h bei RT nachgerührt. Anschließend wurde der Reaktionsansatz 8 h unter Rückfluss gekocht. Es wurden noch zweimal eine 1 M Diisobutylaluminiumhdyrid-Lösung in Toluol zugefügt und jeweils 8 h und 24 h unter Rückfluß gekocht. Nach Hydrolyse des Reaktionsansatzes wurde der entstandene Niederschlag abgesaugt und mit THF nachgewaschen. Das Filtrat wurde eingeengt und der Rückstand mittels Flash-Chromatographie (Aluminiumoxid) gereinigt.

| | |
|---|---|
| Ausbeute: | 0.40 g (53% d. Th.) |
| ESI-MS: | m/z = 226 (M+H)⁺ und m/z = 240 (M₂+H)⁺ |
| Rₜ (HPLC-MS): | 1.55 min (Methode C) |

### Intermediat 30:

### (2,3-Dihydro-1H-indol-3-yl)-methanol

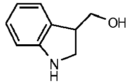

### Stufe 1: 2,3-Dihydro-1H-indol-3-carbonsäureethylester Hydrochlorid

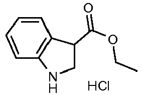

Diese Verbindung wurde wie in der WO2007 054453 beschrieben synthetisiert.

### Stufe 2: (2,3-Dihydro-1H-indol-3-yl)-methanol

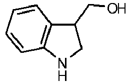

Zu 7.8 mL (7.8 mmol) einer 1 molaren Lithiumaluminiumhydrid-THF-Lösung in 40 mL THF wurden 0.79 g (3.5 mmol) 2,3-Dihydro-1H-indol-3-carbonsäureethylester portionsweise bei RT zugegeben und 1 h unter Rückfluss gekocht. Anschließend wurde der Reaktionsansatz unter Kühlen mit Wasser zersetzt, der entstandene Niederschlag abfiltriert und das Filtrat eingeengt.

| | |
|---|---|
| Ausbeute: | 52 mg (95% d. Th.) |
| ESI-MS: | m/z = 150 (M+H)⁺ |
| Rₜ (HPLC-MS): | 0.31 min (Methode P) |

### Intermediat 31:

### 5,6,7,8-Tetrahydro-4H-thiazol[4,5-d]azepin Hydrochlorid

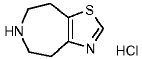

### Stufe 1: 3-Benzylamino-propionsäureethylester

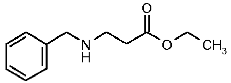

25 g (0.23 mol) Benzylamin und 21 g (0.21 mol) Ethylacrylat in 125 mL EtOH wurden 15 h bei RT gerührt. Anschließend wurde das Lösungsmittel eingeengt und das Rohprodukt ohne weitere Reinigung in die nächste Stufe eingesetzt.

| | |
|---|---|
| Ausbeute: | 30 g (62% d. Th.) |
| ESI-MS: | m/z = 208 (M+H)⁺ |
| R_{f}: | 0.5 (Kieselgel, EtOAc/PE 50%) |

### Stufe 2: 4-[Benzyl-(2-ethoxycarbonyl-ethyl)-amino]-propancarbonsäureethylester

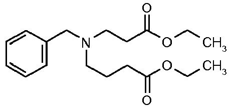

Zu 50 g (0.24 mol) 3-Benzylamino-propionsäureethylester und 83 g (0.60 mol) Kalium-carbonat in 1.0 L Acetonitril wurden langsam 70.6 g (0.36 mol) 4-Brombuttersäureethylester bei RT zugetropft. Anschließend wurde der Reaktionsansatz bei 90°C 12 h gerührt. Nach dem Abkühlen wurde der Reaktionsansatz mit EtOAc verdünnt und die organische Phase abgetrennt. Diese wurde mit Wasser und gesättigter Natriumchlorid-Lösung gewaschen und anschließend über Natriumsulfat getrocknet. Nach dem Filtrieren wurde das Filtrat eingeengt und der Rückstand mittels Flash-Chromatographie (über Aluminiumoxid) gereinigt.

| | |
|---|---|
| Ausbeute: | 55.00 g (68% d. Th.) |
| R_{f}: | 0.7 (Kieselgel, EtOAc/PE 2%) |

### Stufe 3: 1-Benzyl-azepan-4-on

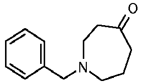

Unter einer Argonatmosphäre wurden 1.0 L Xylol mit einer Dean-Stark-Apparatur bei 145°C 1 bis 2 h erhitzt. Dann wurde das Lösungsmittel auf 65°C abgekühlt, mit 20.8 g (0.19 mol) Kalium-tert-butylat versetzt und für weitere 1 bis 2 h auf 145°C erhitzt. Anschließend wurden 30 g (0.093 mol) 4-[Benzyl-(2-ethoxycarbonyl-ethyl)-amino]-buttersäureethylester in Xylol tropfenweise über einen Zeitraum von 1 h zu dem Reaktionsansatz gegeben und bei 145°C 2 bis 3 h gerührt. Nach dem Abkühlen auf 0°C wurde der Reaktionsansatz mit 0.45 L einer 6N wässrigen Salzsäurelösung versetzt, die wäßrige Phase abgetrennt und für 2 h unter Rückfluss gekocht. Anschließend wurde wieder auf 0°C abgekühlt, der Reaktionsansatz mit wäßriger Natriumhydroxid-Lösung alkalisch gestellt und mit EtOAc extrahiert. Die vereinigten organischen Phasen wurden über Natriumsulfat getrocknet, filtriert und eingeengt. Der Rückstand wurde mittels Flash-Chromatographie (Aluminiumoxid) gereinigt. Ausbeute: 5.50 g (29% d. Th.) ESI-MS: m/z = 204 (M+H)⁺ R_{f}: 0.4 (Kieselgel, EtOAc/PE 30%)

### Stufe 4: 1-Benzyl-5-brom-azepan-4-on Hydrobromid

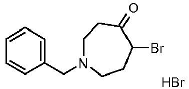

Zu 10 g (49 mmol) 1-Benzyl-azepan-4-on in 28 mL Essigsäure wurden 5.7 mL HBr in Essigsäure (33%) bei RT zugetropft. Anschließend wurden noch 9.5g (60 mmol) Brom bei RT zugegeben und 1.5 h bei RT gerührt. Nach dem Einengen des Reaktionsansatzes unter 35°C wurde der Rückstand in EtOAc gegeben und ca. 1 h unter Rückfluss gekocht. Es wurde die überstehende organische Phase von dem ausgefallenen Feststoff abdekantiert, anschließend nochmals mit EtOAc versetzt und ca. 1 h unter Rückfluss gekocht. Der ausgefallene Feststoff wurde filtriert, mit EtOAc gewaschen und getrocknet.

| | |
|---|---|
| Ausbeute: | 6.0 g (34% d. Th.) |
| R_{f}: | 0.6 (Kieselgel, EtOAc/PE 30%) |

### Stufe 5: 6-Benzyl-5,6,7,8-tetrahydro-4H-thiazol[4,5-d]azepin Hydrochlorid

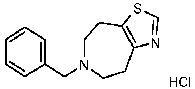

2.1 g (9.7 mmol) Phosphorpentasulfid und 1.9 g (41 mmol) Formamid in Dioxan wurden insgesamt 2.5 h bei 100°C gerührt. Nach dem Abkühlen auf RT wurden 10 g (28 mmol) 1-Benzyl-5-brom-azepan-4-on Hydrobromid zugefügt und 5 h bei 100°C gerührt. Anschließend wurde das Lösungsmittel eingeengt, der Rückstand in gesättigte Natriumbicarbonatlösung gegeben und mit EtOAc extrahiert. Die vereinigten organischen Phasen wurden mit Wasser, wässriger Natriumbicarbonatlösung und gesättigter Natriumchloridlösung gewaschen. Dann wurde die organische Phase über Natriumsulfat getrocknet, filtriert und eingeengt. Der Rückstand wurde mittels Flash-Chromatographie gereinigt. Die Produkt enthaltenden Fraktionen wurden vereinigt und eingeengt. Die freie Base wurde mit methanolischer Salzsäurelösung versetzt. Der entstandene Niederschlag wurde abfiltriert.

| | |
|---|---|
| Ausbeute: | 3.50 g (45% d. Th.) |
| ESI-MS: | m/z = 245 (M+H)⁺ |
| R_{f}: | 0.5 (Kieselgel, MeOH/Chloroform 10%) |

### Stufe 6: 4,5,7,8-Tetrahydro-thiazol[4,5-d]azepin-6-carbonsäureethylester

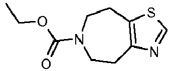

Zu 2.0 g (8.2 mmol) 6-Benzyl-5,6,7,8-tetrahydro-4H-thiazol[4,5-d]azepin in 100 mL DCM wurden 1.4 g (9.8 mmol) 1-Chlorethylchloroformiat bei -20°C zugetropft und 30 min nachgerührt. Dann wurde das organische Lösungsmittel eingeengt und der Rückstand wurde ohne weitere Reinigung weiter umgesetzt.

| | |
|---|---|
| Ausbeute: | 1.5 g (81% d. Th.) |
| R_{f}: | 0.6 (Kieselgel, EtOAc/PE 20%) |

### Stufe 7: 5,6,7,8-Tetrahydro-4H-thiazol[4,5-d]azepin Hydrochlorid

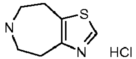

1.5 g (6.6 mmol) 4,5,7,8-Tetrahydro-thiazol[4,5-d]azepin-6-carbonsäureethyl ester in 50 mL MeOH wurden 3 h unter Rückfluss gekocht. Nach dem Einengen des organischen Lösungsmittels wurde der Rückstand mittels Flash-Chromatographie gereinigt. Die Produkt enthaltenden Fraktionen wurden vereinigt und eingeengt. Die freie Base wurde mit 5.0 mL (12.5 mmol) einer 2.5 molaren methanolischen Salzsäurelösung versetzt und das überschüssige Lösungsmittel eingeengt.

| | |
|---|---|
| Ausbeute: | 0.70 g (55% d. Th.) |
| ESI-MS: | m/z = 155 (M+H)⁺ |
| R_{f}: | 0.2 (Kieselgel, MeOH/Chloroform 20%) |

### Intermediat 32:

### 1,4,5,6,7,8-Hexahydro-imidazo[4,5-d]azepin

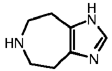

### Stufe 1: 6-Benzyl-1,4,5,6,7,8-hexahydro-imidazo[4,5-d]azepin

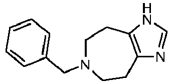

10 g (28 mmol) 1-Benzyl-5-brom-azepan-4-on Hydrobromid, 11 g (83 mmol) Kalium-carbonat und 6.7 g (83 mmol) Formamidin Hydrochlorid in 0.10 L MeOH wurden 5 h unter Rückfluss gekocht. Nach dem Abkühlen auf RT wurde das Lösungsmittel eingeengt und der Rückstand in DCM gelöst. Die organische Phase wurde mit Wasser gewaschen, über Natriumsulfat getrocknet, filtriert und eingeengt. Der Rückstand wurde mittels Flash-Chromatographie gereinigt.

| | |
|---|---|
| Ausbeute: | 1.5 g (24%d.Th.) |
| ESI-MS: | m/z = 228 (M+H)⁺ |
| R_{f}: | 0.2 (Kieselgel, MeOH/DCM/NH₃ 2:8:0.1) |

### Stufe 2: 1,4,5,6,7,8-Hexahydro-imidazo[4,5-d]azepin

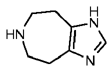

1.0 g (4.4 mmol) 6-Benzyl-1,4,5,6,7,8-hexahydro-imidazo[4,5-d]azepin in EtOH wurden mit 1.0 g Palladium auf Kohle (10%) versetzt und 15 h in einer 75 psi Wasserstoff-atmosphäre hydriert. Der Katalysator wurde abgesaugt und das Filtrat wurde eingeengt. Der Rückstand wurde mit Acetonitril gewaschen und anschließend getrocknet.

| | |
|---|---|
| Ausbeute: | 0.35 g (58 % d. Th.) |
| ESI-MS: | m/z = 138 (M+H)⁺ |
| R_{f}: | 0.1 (Kieselgel, MeOH/DCM/NH₃ 2:8:0.1) |

### Intermediat 33:

### But-3-ynyl-methyl-amin

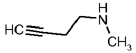

9.0 mL (51 mmol) 3-Butyn-p-Toluolsulfonat in 24 mL (0.31 mol) einer 40%igen wässriger Methylaminlösung wurden für 10 min in der Mikrowelle erhitzt. Das Reaktionsgemisch wurde mit Wasser/Kaliumcarbonatlösung und DCM ausgeschüttelt. Die organische Phase wurde getrocknet und eingeengt.

| | |
|---|---|
| Ausbeute: | 2.70 g (64% d. Th.) |

### Herstellung der Endverbindungen:

### Beispiel 1:

### 5-(6-(Indolin-1-carbonyl)pyrimidin-4-ylamino)-1,3-dihydrospiro[inden-2,3'-pyrrolo[2,3-b]pyridin]-2'(1'H)-on

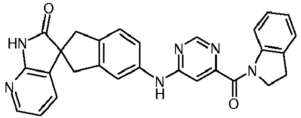

Zu 100 mg (0.39 mmol) (6-Chlor-pyrimidin-4-yl)-(2,3-dihydro-indol-1-yl)-methanon und 100 µL (0.58 mmol) DIPEA in 10 mL DMF wurden 100 mg (0.40 mmol) 5-Amino-1,3-di-hydrospiro[inden-2,3'-pyrrolo[2,3-b]pyridin]-2'(1'H)-on zugegeben. Der Reaktionsansatz wurde über das Wochenende bei 100°C gerührt. Der Reaktionsansatz wurde am Rotationsverdampfer einrotiert. Der Rückstand wurde mit Wasser versetzt und das ausgefallene Produkt abgesaugt. Der Niederschlag wurde in DMF gelöst und mittels präparativer HPLC aufgereinigt. Die Produktfraktionen wurden vereinigt und lyophilisiert.

| | |
|---|---|
| Ausbeute: | 2.0 mg (1% d.Th.) |
| ESI-MS: | m/z = 475 (M+H)⁺ |
| R_{f} (Kieselgel): | 0.46 (Laufmittel A) |

### Beispiel 2:

### 5-(6-(5-Chlorindolin-1-carbonyl)pyrimidin-4-ylamino)-1,3-dihydrospiro[inden-2,3'-pyrrolo[2,3-b]pyridin]-2'(1'H)-on

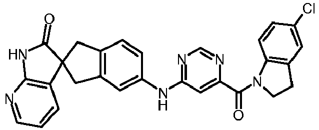

Zu 100 mg (0.34 mmol) (5-Chlor-2,3-dihydro-indol-1-yl)-(6-chlor-pyrimidin-4-yl)-methanon und 100 µL (0.58 mmol) DIPEA in 10 mL DMF wurden 90 mg (0.36 mmol) 5-Amino-1,3-dihydrospiro[inden-2,3'-pyrrolo[2,3-b]pyridin]-2'(1'H)-on zugegeben. Der Reaktionsansatz wurde über das Wochenende bei 100°C gerührt. Der Reaktionsansatz wurde am Rotationsverdampfer eingeengt und der Rückstand mit Wasser versetzt. Das ausgefallene Produkt wurde abgesaugt und in DMF gelöst. Die Reinigung erfolgte mittels präparativer HPLC. Die Produktfraktionen wurden vereinigt und lyophilisiert.

| | |
|---|---|
| Ausbeute: | 2.0 mg (1 % d.Th.) |
| ESI-MS: | m/z = 509/511 (M+H)⁺ |
| R_{f} (Kieselgel): | 0.50 (Laufmittel A) |

### Allgemeine Arbeitsvorschrift 1 (AAV1) zur Umsetzung von 6-Chlor-N-(2,2,2-trifluorethyl)-pyrimidin-4-carboxamid-Derivaten mit 5-Amino-1,3-dihydrospiro[inden-2,3'-pyrrolo[2,3-b]-pyridin]-2'(1'H)-on:

Zu 50 mg des 6-Chlor-N-(2,2,2-trifluorethyl)pyrimidin-4-carboxamid -Derivats und 45.2 mg 5-Amino-1,3-dihydrospiro[inden-2,3'-pyrrolo[2,3-b]pyridin]-2'(1'H)-on in 5 mL 2-Pentanol wurden 5.1 mg (0.032 mmol) Benzolsulfonsäure zugegeben. Der Reaktionsansatz wurde über Nacht unter Rückfluss gekocht. Der Reaktionsansatz wurde am Rotationsverdampfer eingeengt und der Rückstand in DMF aufgenommen. Die Reinigung erfolgte mittels präparativer HPLC. Die Produktfraktionen wurden vereinigt und lyophilisiert.

| Beispiel Methode | Struktur | Carboxamid [Menge Carboxamid-Derivat] Ausbeute | Analytische Daten |
|---|---|---|---|
| Beispiel 3: AAV1 | 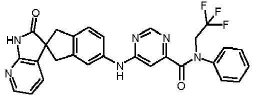 | 6-Chlor-N-phenyl-N-(2,2,2-trifluorethyl)-pyrimidin-4-carboxamid | ESI-MS: m/z = 531 [M+H]⁺ |
| | 6-(2'-Oxo-1,1',2',3-tetrahydro-spiro[inden-2,3'-pyrrolo[2,3-b]-pyridin]-5-ylamino)-N-phenyl-N-(2,2,2-trifluorethyl)pyrimidin-4-carboxamid | 50 mg (0.16 mmol) | Rₜ: 1.34 min (Methode C) |
| | | 13 mg (16% d.Th.) | |
| Beispiel 4: AAV1 | 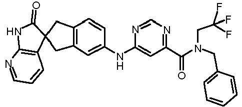 | N-Benzyl-6-chlor-N-(2,2,2-trifluorethyl)-pyrimidin-4-carboxamid | ESI-MS: m/z = 545[M+H]⁺ |
| | N-Benzyl-6-(2'-oxo-1,1',2',3-tetrahydrospiro[inden-2,3'-pyrrolo[2,3-b]pyridin]-5-yl-amino)-N-(2,2,2-trifluoroethyl)-pyrimidin-4-carboxamid | 50 mg (0.15 mmol) | Rₜ: 3.81 min (Methode E) |
| | | 7 mg (9% d.Th.) | |
| Beispiel 5: AAV1 | 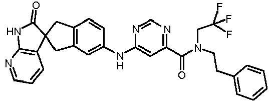 | 6-Chlor-N-phenethyl-N-(2,2,2-trifluorethyl)-pyrimidin-4-carboxamid | ESI-MS: m/z = 559 [M+H]⁺ |
| | 6-(2'-Oxo-1,1',2',3-tetrahydro-spiro[inden-2,3'-pyrrolo[2,3-b]-pyridin]-5-ylamino)-N-phenethyl-N-(2,2,2-trifluorethyl)pyrimidin-4-carboxamid | 50 mg (0.15 mmol) | Rₜ: 3.92 min (Methode K) |
| | | 41 mg (51% d.Th.) | |

### Beispiel 6:

### 5'-(6-(Indolin-1-carbonyl)pyrimidin-4-ylamino)-3-methyl-1',3'-dihydrospiro[imidazolidin-4,2'-inden]-2,5-dion

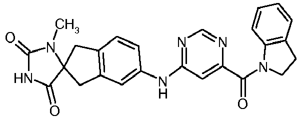

80.0 mg (0.23 mmol) 6-(3-Methyl-2,5-dioxo-1',3'-dihydrospiro[imidazolidin-4,2'-inden]-5'-ylamino)pyrimidin-4-carbonsäure , 30 µL (0.27 mmol) 2,3-Dihydro-1H-indol, 70 µL (0.50 mmol) Triethylamin und 80.0 mg (0.25 mmol) TBTU in 1.5 mL DMF wurden 4 h bei RT gerührt. Der Reaktionsansatz wurde über einen Spritzenfilter filtriert und mittels präparativer HPLC aufgereinigt. Die Produktfraktionen wurden vereinigt, einrotiert und getrocknet.

| | |
|---|---|
| Ausbeute: | 35 mg (34% d.Th.) |
| ESI-MS: | m/z = 455 (M+H)⁺ |
| R_{f} (Kieselgel): | 0.30 (Laufmittel A ) |

### Beispiel 7:

### 5-(6-(2-Ethyl-6-fluorindolin-1-carbonyl)pyrimidin-4-ylamino)-1,3-dihydrospiro[inden-2,3'-pyrrolo[2,3-b]pyridin]-2'(1'H)-on

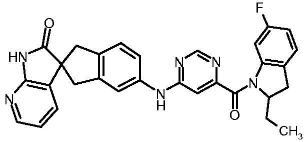

Zu 516 mg (2.00 mmol) (6-Chlor-pyrimidin-4-yl)-(2-ethyl-6-fluor-2,3-dihydro-indol-1-yl)-methanon und 611 mg (2.00 mmol) 5-Amino-1,3-dihydrospiro[inden-2,3'-pyrrolo[2,3-b]-pyridin]-2'(1'H)-on in 10 mL 2-Pentanol wurden 63 µL (0.25 mmol) wässriger 4M Salz-säure gegeben. Das Reaktionsgemisch wurde 6 h unter Rückfluss etrhitzt. Der Reaktionsansatz wurde am Rotationsverdampfer eingeengt und der Rückstand in DMF aufgenommen. Die Reinigung erfolgte mittels präparativer HPLC. Die Produktfraktionen wurden vereinigt und lyophilisiert.

| | |
|---|---|
| Ausbeute: | 650 mg (62% d.Th.) |
| ESI-MS: | m/z = 521 [M+H]⁺ |
| Rₜ (HPLC-MS): | 1.60 min (Methode C) |

### Beispiel 8:

### 5-(6-(2-Propylindolin-1-carbonyl)pyrimidin-4-ylamino)-1,3-dihydrospiro[inden-2,3'-pyrrolo[2,3-b]pyridin]-2'(1'H)-on

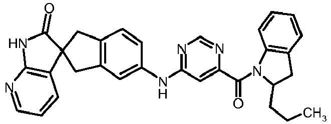

Zu 103 mg (0.40 mmol) (6-Chlor-pyrimidin-4-yl)-(2-propyl-2,3-dihydro-indol-1-yl)-methanon und 108 mg (0.36 mmol) 5-Amino-1,3-dihydrospiro[inden-2,3'-pyrrolo[2,3-b]pyridin]-2'(1'H)-on in 2 mL 2-Pentanol wurden 12.5 µL (50 µmol) wässriger 4M Salzsäure gegeben. Der Reaktionsansatz wurde 2 h unter Rückfluss gekocht. Das Reaktionsgemisch wurde am Rotationsverdampfer eingeengt und der Rückstand in DMF aufgenommen. Die Reinigung erfolgte mittels präparativer HPLC. Die Produktfraktionen wurden vereinigt und lyophilisiert.

| | |
|---|---|
| Ausbeute: | 90 mg (43% d.Th.) |
| ESI-MS: | m/z = 517 [M+H]⁺ |
| Rₜ (HPLC-MS): | 1.55 min (Methode C) |

### Beispiel 9:

### 5-(6-(4,4-Dimethyl-4,5,6,7-tetrahydrothieno[2,3-c]pyridin-6-carbonyl)pyrimidin-4-ylamino)-1,3-dihydrospiro[inden-2,3'-pyrrolo[2,3-b]pyridin]-2'(1'H)-on

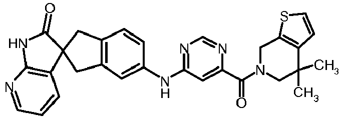

86 mg (0.21 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 40 mg (0.24 mmol) 4,4-Dimethyl-4,5,6,7-tetrahydro-thieno[2,3-c]pyridin, 68 µL (0.48 mmol) Triethylamin und 77 mg (0.24 mmol) TBTU in 1.0 mL DMF wurden 4 h bei RT gerührt.Der Reaktionsansatz wurde mittels präparativer HPLC aufgereinigt. Die Produktfraktionen wurden vereinigt, eingeengt, abgesaugt und anschließend mit Wasser gewaschen. Das Produkt wurde getrocknet..

| | |
|---|---|
| Ausbeute: | 39 mg (35% d.Th.) |
| ESI-MS: | m/z = 523 [M+H]⁺ |
| Rₜ (HPLC-MS): | 3.49 min (Methode E) |

### Allgemeine Arbeitsvorschrift 2 (AAV2) zur Umsetzung von 6-,2'-Oxo-1,1',2',3-tetrahydrospiro[inden-2,3'-pyrrolo[2,3-b]pyridin]-5-ylamino)pyrimidin-4-carbonsäure Hydrochlorid mit Amin-Derivaten:

Zu 86 mg (0.21 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid in 1 mL DMF wurden 1.1 Äquivalente (0.24 mmol) des Amin-Derivats, 2 bis 5 Äquivalente Triethylamin und 1.1 Äquivalente (0.24 mmol) TBTU gegeben und 4 h bei RT gerührt. Die Reinigung erfolgte mittels präparativer HPLC. Die Produktfraktionen wurden vereinigt und lyophilisiert. Analog dieser Arbeitsvorschrift konnten folgende Verbindungen synthetisiert werden:

| Beispiel Methode | Struktur | Amin-Derivat [Menge Amin-Derivat] Ausbeute | Analytische Daten |
|---|---|---|---|
| Beispiel 10: AAV2 | 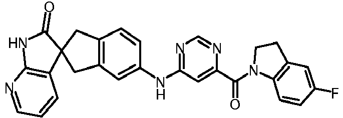 | 5-Fluor-2,3-dihydro-1H-indol | ESI-MS: m/z =493 [M+H]⁺ |
| | 5-(6-(5-Fluorindolin-1-carbonyl)-pyrimidin-4-ylamino)-1,3-dihydro-spiro[inden-2,3'-pyrrolo[2,3-b]pyridin]-2'(1'H)-on | 33 mg (0.24 mmol) | |
| | | 12 mg (12% d.Th.) | Rₜ: 3.46 min (Methode E) |
| Beispiel 11: AAV2 | 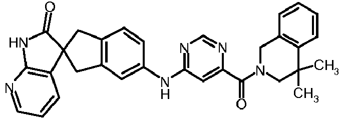 | 4,4-Dimethyl-1,2,3,4-tetrahydro-iso-chinolin Hydrochlorid | ESI-MS: m/z =517 [M+H]⁺ |
| | 5-(6-(4,4-Dimethyl-1,2,3,4-tetrahydroisochinolin-2-carbonyl)pyrimidin-4-ylamino)-1,3-dihydrospiro-[inden-2,3'-pyrrolo[2,3-b]pyridin]-2'(1'H)-on | 47 mg (0.24 mmol) | |
| | | 11 mg (10% d.Th.) | Rₜ: 3.51 min (Methode E) |
| Beispiel 12: AAV2 | 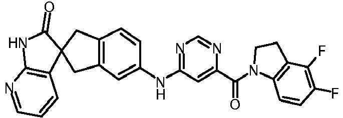 | 4,5-Difluor-2,3-dihydro-1H-indol Hydrochlorid | ESI-MS: m/z =511 [M+H]⁺ |
| | 5-(6-(4,5-Difluorindolin-1-carbonyl)-pyrimidin-4-ylamino)-1,3-dihydro-spiro[inden-2,3'-pyrrolo[2,3-b]pyridin]-2'(1'H)-on | 45.8 mg (0.24 mmol) | |
| | | 55 mg (51% d.Th.) | Rₜ: 3.68 min (Methode E) |
| Beispiel 13: AAV2 | 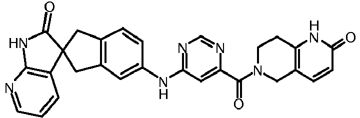 | 5,6,7,8-Tetrahydro-1 H-[1,6]naphthyridin-2-on | ESI-MS: m/z =506 [M+H]⁺ |
| | 5-(6-(2-Oxo-1,2,5,6,7,8-hexahydro-1,6-naphthyridin-6-carbonyl)pyrimidin-4-ylamino)-1,3-dihydrospiro-[inden-2,3'-pyrrolo[2,3-b]pyridin]-2'(1'H)-on | 40 mg (0.27 mmol) | Rₜ: 2.12 min (Methode E) |
| | | 25 mg (24% d.Th.) | |
| Beispiel 14: AAV2 | 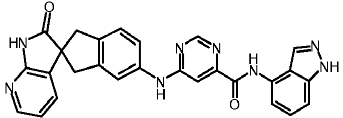 | 1H-Indazol-4-ylamin | ESI-MS: m/z =489 [M+H]⁺ |
| | | 32 mg (0.24 mmol) | |
| | N-(1 H-lindazol-4-yl)-6-(2'-oxo-1,1',2',3-tetrahydrospiro[inden-2,3'-pyrrolo[2,3-b]pyridin]-5-ylamino)-pyrimidin-4-carboxamid | 10 mg (8% d.Th.) | Rₜ:1.24 min (Methode C) |
| Beispiel 15: AAV2 | 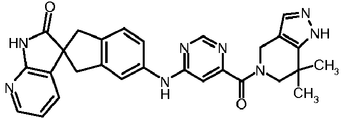 | 7,7-Dimethyl-4,5,6,7-tetrahydro-1H-pyrazolo[4,3-c]pyridin Dihydrochlorid | ESI-MS: m/z =507 [M+H]⁺ |
| | 5-(6-(7,7-Dimethyl-4,5,6,7-tetrahydro-1H-pyrazolo[4,3-c]pyridin-5-carbonyl)pyrimidin-4-ylamino)-1,3-dihydrospiro[inden-2,3'-pyrrolo[2,3-b]pyridin]-2'(1'H)-on | 54 mg (0.24 mmol) | Rₜ: 2.43 min (Methode E) |
| | | 6 mg (6% d.Th.) | |
| Beispiel 16: AAV2 Ansatz wurde in 2mL DMF durchgeführt | 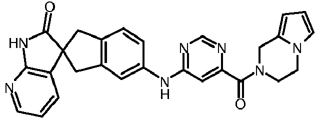 | 1,2,3,4-Tetrahydropyrrolo[1,2-a]-pyrazin | ESI-MS: m/z =478 [M+H]⁺ |
| | 5-(6-(1,2,3,4-Tetrahydropyrrolo-[1,2-a]pyrazin-2-carbonyl)pyrimidin-4-ylamino)-1,3-dihydrospiro-[inden-2,3'-pyrrolo[2,3-b]pyridin]-2'(1'H)-on | 37 mg (0.24 mmol) | |
| | | 59 mg (59% d.Th | Rₜ: 1.29 min (Methode C) |

### Allgemeine Arbeitsvorschrift 3 (AAV3) zur Umsetzung von 2-(2'-Oxo-1,1',2',3-tetrahydrospiro[inden-2,3'-pyrrolo[2,3-b]pyridin]-5-ylamino)isonicotinsäure mit Amin-Derivaten:

Zu 0.10 g (0.27 mmol) 2-(2'-Oxo-1,1',2',3-tetrahydrospiro[inden-2,3'-pyrrolo[2,3-b]pyridin]-5-ylamino)isonicotinsäure in 3.0 mL DMF wurden 1.1 Äquivalente (0.30 mmol) des Amin-Derivates, 1.3 bis 5 Äquivalente Triethylamin und 1 Äquivalent (0.28 mmol) TBTU gegeben und 4 h bei RT gerührt. Die Reinigung erfolgte mittels präparativer HPLC. Die Produktfraktionen wurden vereinigt und lyophilisiert. Analog dieser Arbeitsvorschrift konnten folgende Verbindungen synthetisiert werden:

| Beispiel Methode | Struktur | Amin-Derivat [Menge Amin-Derivat] Ausbeute | Analytische Daten |
|---|---|---|---|
| Beispiel 17: AAV3 | 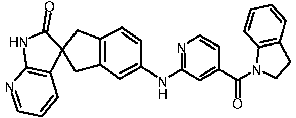 | 2,3-Dihydro-1H-indol | ESI-MS: m/z =474 [M+H]⁺ |
| | 5-(4-(Indolin-1-carbonyl)pyridin-2-ylamino)-1,3-dihydrospiro-[inden-2,3'-pyrrolo[2,3-b]pyridin]-2'(1'H)-on | 34 µL (0.30 mmol) | |
| | | 50 mg (39% d.Th.) | Rₜ: 1.27 min (Methode C) |
| Beispiel 18: AAV3 | 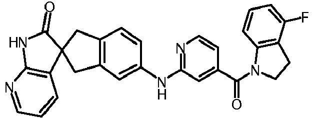 | 4-Fluor-2,3-dihydro-1H-indol | ESI-MS: m/z =492 [M+H]⁺ |
| | 5-(4-(4-Fluorindolin-1-carbonyl)-pyridin-2-ylamino)-1,3-dihydro-spiro[inden-2,3'-pyrrolo[2,3-b]-pyridin]-2'(1'H)-on | 40 mg (0.29 mmol) | |
| | | 50 mg (38% d.Th.) | Rₜ: 1.31 min (Methode C) |
| Beispiel 19: AAV3 | 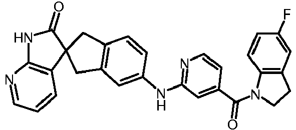 | 5-Fluor-2,3-dihydro-1H-indol | ESI-MS: m/z =492 [M+H]⁺ |
| | | 40 mg (0.29 mmol) | |
| | 5-(4-(5-Fluorindolin-1-carbonyl)-pyridin-2-ylamino)-1,3-dihydrospiro[inden-2,3'-pyrrolo[2,3-b]-pyridin]-2'(1'H)-on | 45 mg (34% d.Th.) | Rₜ: 1.28 min (Methode C) |
| Beispiel 20: AAV3 | 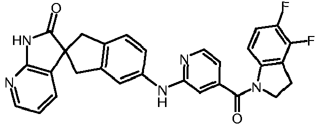 | 4,5-Difluor-2,3-dihydro-1H-indol Hydrochlorid | ESI-MS: m/z =510 [M+H]⁺ |
| | 5-(4-(4,5-Difluorindolin-1-carbonyl)pyridin-2-ylamino)-1,3-di-hydrospiro[inden-2,3'-pyrrolo[2,3-b]pyridin]-2'(1'H)-on | 55 mg (0.29 mmol) | |
| | | 50 mg (37% d.Th.) | Rₜ: 1.35 min (Methode C) |
| Beispiel 21: AAV3 | 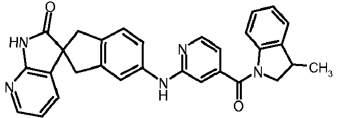 | 3-Methyl-2,3-dihydro-1H-indol | ESI-MS: m/z =488 [M+H]⁺ |
| | 5-(4-(3-Methylindolin-1-carbonyl)pyridin-2-ylamino)-1,3-di-hydrospiro[inden-2,3'-pyrrolo-[2,3-b]pyridin]-2'(1'H)-on | 40 mg (0.30 mmol) | |
| | | 45 mg (34% d.Th.) | Rₜ: 1.33 min (Methode C) |
| Beispiel 22: AAV3 | 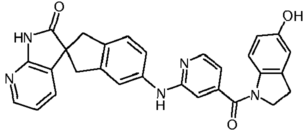 | 2,3-Dihydro-1H-indol-5-ol | ESI-MS: m/z =490 [M+H]⁺ |
| | 5-(4-(5-Hydroxyindolin-1-carbonyl)pyridin-2-ylamino)-1,3-di-hydrospiro[inden-2,3'-pyrrolo[2,3-b]pyridin]-2'(1'H)-on | 50µL (0.36 mmol) | |
| | | 65 mg (49% d.Th.) | Rₜ: 1.15 min (Methode C) |

### Allgemeine Arbeitsvorschrift 4 (AAV4) zur Umsetzung von 4-(2'-Oxo-1,1',2',3-tetrahydrospiro[inden-2,3'-pyrrolo[2,3-b]pyridin]-5-ylamico)picolinsäure mit Amin-Derivaten:

Zu 0.10 mg (0.27 mmol) 4-(2'-Oxo-1,1',2',3-tetrahydrospiro[inden-2,3'-pyrrolo[2,3-b]pyridin]-5-ylamino)picolinsäure in 3.0 mL DMF wurden 1.1 Äquivalente (0.30 mmol) des Amin-Derivats, 1.3 bis 5 Äquivalente Triethylamin und 1 Äquivalent (0.28 mmol) TBTU gegeben und 4 h bei RT gerührt. Die Reinigung erfolgte mittels präparativer HPLC. Die Produktfraktionen wurden vereinigt und lyophilisiert. Analog dieser Arbeitsvorschrift konnten folgende Verbindungen synthetisiert werden:

| Beispiel Methode | Struktur | Amin-Derivat [Menge Amin-Derivat] Ausbeute | Analytische Daten |
|---|---|---|---|
| Beispiel 23: AAV4 | 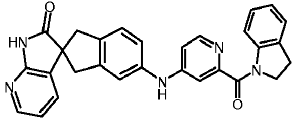 | 2,3-Dihydro-1H-indol | ESI-MS: m/z =474 [M+H]⁺ |
| | 5-(2-(Indolin-1-carbonyl)pyridin-4-ylamino)-1,3-dihydrospiro-[inden-2,3'-pyrrolo[2,3-b]pyridin]-2'(1'H)-on | 34 µL (0.30 mmol) | |
| | | 80 mg (63% d.Th.) | Rₜ: 1.10 min (Methode C) |
| Beispiel 24: AAV4 | 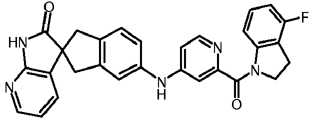 | 4-Fluor-2,3-dihydro-1H-indol | ESI-MS: m/z =492 |
| | 5-(2-(4-Fluorindolin-1-carbonyl)-pyridin-4-ylamino)-1,3-dihydrospiro[inden-2,3'-pyrrolo[2,3-b]-pyridin]-2'(1'H)-on | 40 mg (0.29 mmol) | [M+H]⁺ |
| | | 65 mg (49% d.Th.) | Rₜ: 1.14 min (Methode C) |
| Beispiel 25: AAV4 | 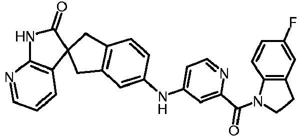 | 5-Fluor-2,3-dihydro-1H-indol | ESI-MS: m/z =492 [M+H]⁺ |
| | 5-(2-(5-Fluorindolin-1-carbonyl)-pyridin-4-ylamino)-1,3-dihydrospiro[inden-2,3'-pyrrolo[2,3-b]-pyridin]-2'(1'H)-on | 40 mg (0.29 mmol) | |
| | | 55 mg (42% d.Th.) | Rₜ: 1.13 min (Methode C) |
| Beispiel 26: AAV4 | 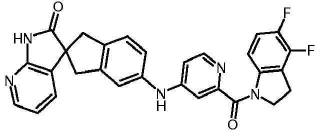 | 4,5-Difluor-2,3-dihydro-1H-indol Hydrochlorid | ESI-MS: m/z =510 [M+H]⁺ |
| | 5-(2-(4,5-Difluorindolin-1-carbonyl)pyridin-4-ylamino)-1,3-dihydrospiro[inden-2,3'-pyrrolo[2,3-b]pyridin]-2'(1'H)-on | 55 mg (0.29 mmol) | |
| | | 30 mg (22% d.Th.) | Rₜ: 1.17 min (Methode C) |
| Beispiel 27: AAV4 | 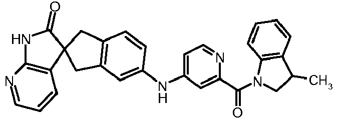 | 3-Methyl-2,3-dihydro-1H-indol | ESI-MS: m/z =488 [M+H]⁺ |
| | | 40 mg (0.30 mmol) | |
| | 5-(2-(3-Methylindolin-1-carbonyl)pyridin-4-ylamino)-1,3-dihydrospiro[inden-2,3'-pyrrolo[2,3-b]pyridin]-2'(1'H)-on | 55 mg (42% d.Th.) | Rₜ: 1.15 min (Methode C) |
| Beispiel 28: AAV4 | 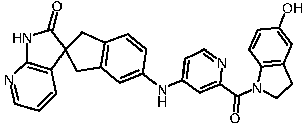 | 2,3-Dihydro-1H-indol-5-ol | ESI-MS: m/z =490 [M+H]⁺ |
| | 5-(2-(5-Hydroxyindolin-1-carbonyl)pyridin-4-ylamino)-1,3-dihydrospiro[inden-2,3'-pyrrolo[2,3-b]pyridin]-2'(1'H)-on | 40 mg (0.30 mmol) | |
| | | 65 mg (49% d.Th.) | Rₜ: 1.08 min (Methode C) |

### Beispiel 29:

### 5-(6-(3,3-Dimethylpiperidin-1-carbonyl)pyrimidin-4-ylamino)-1,3-dihydrospiro[inden-2,3'-pyrrolo[2,3-b]pyridin]-2'(1'H)-on

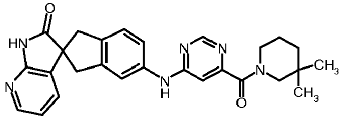

82 mg (0.20 mmol) 6-(2'-Ooxo-1,1',2',3-tetrahydrospiro[inden-2,3'-pyrrolo[2,3-b]pyridin]-5-ylamino)pyrimidin-4-carbonsäure Hydrochlorid, 32 mg (0.27 mmol) 3,3-Dimethyl-piperidin, 80 µL (0.57 mmol) Triethylamin und 70 mg (0.22 mmol) TBTU in 0.80 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 21 mg (22% d.Th.) |
| ESI-MS: | m/z = 469 [M+H]⁺ |
| Rₜ (HPLC-MS): | 1.30 min (Methode C) |

### Beispiel 30:

### N-(Dicyclopropylmethyl)-N-methyl-6-(2'-oxo-1,1',2',3-tetrahydrospiro[inden-2,3'-pyrrolo-[2,3-b]pyridin]-5-ylamino)pyrimidin-4-carboxamid

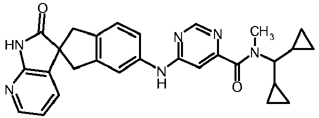

82 mg (0.20 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 35 mg (0.27 mmol) Dicyclopropylmethyl-methyl-amin, 80 µL (0.57 mmol) Triethylamin und 70 mg (0.22 mmol) TBTU in 0.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 25 mg (26% d.Th.) |
| ESI-MS: | m/z = 481 [M+H]⁺ |
| Rₜ (HPLC-MS): | 1.33 min (Methode C) |

### Allgemeine Arbeitsvorschrift 5 (AAV5) zur Umsetzung von 6-(2'-Oxo-1,1',2',3-tetrahydrospiro[inden-2,3'-pyrrolo[2,3-b]pyridin]-5-ylamino)pyrimidin-4-carbonsäure Hydrochlorid mit Amin-Derivaten:

Zu 4.1 mg (10 µmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid in 0,32 mL DMF wurden 1.2 Äquivalente (12 µmol) des Amin-Derivats, 3.4 Äquivalente DIPEA (34 µmol) und 1.20 Äquivalente (12 µmol) TBTU gegeben und über Nacht bei RT geschüttelt. Der Reaktionsansatz wurde in der Vakuumzentrifuge bei 60°C eingedampft. Analog dieser Arbeitsvorschrift konnten folgende Verbindungen synthetisiert werden:

| Beispiel Methode | Struktur | Amin-Derivat [Menge Amin-Derivat] | Analytische Daten |
|---|---|---|---|
| Beispiel 31: AAV5 | 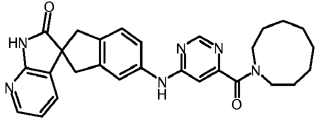 | Azacyclonan 1.53 mg (12 µmol) | ESI-MS: m/z = 483 [M+H]⁺ |
| | 5-(6-(Azonan-1-carbonyl)pyrimidin-4-ylamino)-1,3-dihydrospiro-[inden-2,3'-pyrrolo[2,3-b]pyridin]-2'(1'H)-on | | Rₜ: 1.66 min (Methode Q) |
| Beispiel 32: AAV5 | 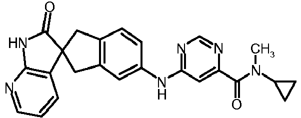 | N-Cyclopropylethylamin 0.85 mg (12µmol) | ESI-MS: m/z = 427 [M+H]⁺ |
| | N-Cyclopropyl-N-methyl-6-(2'-oxo-1,1',2',3-tetrahydrospiro-[inden-2,3'-pyrrolo[2,3-b]pyridin]-5-ylamino)pyrimidin-4-carbox-amid | | Rₜ: 1.42 min (Methode Q) |
| Beispiel 33: AAV5 | 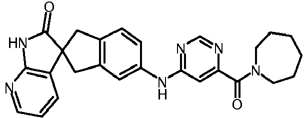 | Homopiperidin 1.19 mg (12 µmol) | ESI-MS: m/z = 455 [M+H]⁺ |
| | 5-(6-(Azepan-1-carbonyl)pyrimi-din-4-ylamino)-1,3-dihydrospiro-[inden-2,3'-pyrrolo[2,3-b]pyridin]-2'(1'H)-on | | Rₜ: 1.53 min (Methode Q) |

### Beispiel 34:

### 5-(6-(5-Hydroxyindolin-1-carbonyl)pyrimidin-4-ylamino)-1,3-dihydrospiro[inden-2,3'-pyrrolo[2,3-b]pyridin]-2'(1'H)-on

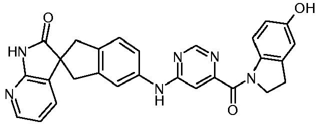

0.15 g (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 50 mg (0.37 mmol) 2,3-Dihydro-1H-indol-5-ol,150 µL (0.87 mmol) DIPEA und 0.13 g (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 44 mg (23% d.Th.) |
| ESI-MS: | m/z = 491 (M+H)⁺ |
| Rₜ (HPLC-MS): | 1.33 min (Methode C) |

### Beispiel 35:

### (R)-5-(6-(5-Fluorindolin-1-carbonyl)pyrimidin-4-ylamino)-1,3-dihydrospiro[inden-2,3'-pyrro-lo[2,3-b]pyridin]-2'(1'H)-on

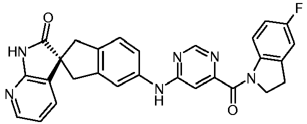

0.10 g (0.40 mmol) (R)-5-Amino-1,3-dihydrospiro[inden-2,3'-pyrrolo[2,3-b]pyridin]-2'(1'H)-on, 0.11 g (0.40 mmol) (6-Chlor-pyrimidin-4-yl)-(5-fluor-2,3-dihydro-indol-1-yl)-methanon und 13 µL einer 4 molaren wässrigen Salzsäurelösung wurden in 2.0 mL 2-Propanol zusammengegeben und 4 h unter Rückfluß gekocht. Dann wurde der Reaktionsansatz mit Diethylether verdünnt, abgesaugt und mit Diethylether nachgewaschen. Der Niederschlag wurde in DMF gelöst und mittels präparativer HPLC-MS gereinigt. Die Produkt enthaltenden Fraktionen wurden vereinigt und das organische Lösungsmittel wurde eingeengt. Der Rückstand wurde mit einer 1 N wässrigen Natriumhydroxid-Lösung alkalisch gestellt, der ausgefallene Niederschlag abgesaugt, mit Wasser nachgewaschen und im HV getrocknet.

| | |
|---|---|
| Ausbeute: | 35 mg (18% d.Th.) |
| ESI-MS: | m/z = 493 (M+H)⁺ |
| Rₜ (HPLC-MS): | 1.38 min (Methode C) |

### Beispiel 36:

### 5-(6-(6-Fluorindolin-1 -carbonyl)pyrimidin-4-ylamino)-1,3-dihydrospiro[inden-2,3'-pyrrolo-[2,3-b]pyridin]-2'(1'H)-on

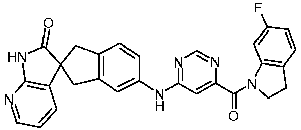

0.10 g (0.24 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 35 mg (0.26 mmol) 6-Fluor-2,3-dihydro-1 H-indol, 0.10 mL (0.72 mmol) TEA und 90 mg (0.28 mmol) TBTU in 1.8 mL DMF wurden 1 h bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 55 mg (44% d.Th.) |
| ESI-MS: | m/z = 493 (M+H)⁺ |
| Rₜ (HPLC-MS): | 1.53 min (Methode C) |

### Beispiel 37:

### 5-(6-(2-Methylindolin-1-carbonyl)pyrimidin-4-ylamino)-1,3-dihydrospiro[inden-2,3'-pyrro-lo[2,3-b]pyridin]-2'(1'H)-on

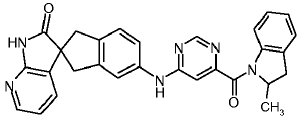

50 mg (0.12 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 16 µL (0.12 mmol) 2-Methylindolin, 0.10 mL (0.72 mmol) TEA und 45 mg (0.14 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 5.0 mg (8% d.Th.) |
| ESI-MS: | m/z = 489 (M+H)⁺ |
| Rₜ (HPLC-MS) | 1.59 min (Methode C) |

### Beispiel 38:

### (S)-5-(6-(5-Fluoroindolin-1-carbonyl)pyrimidin-4-ylamino)-1,3-dihydrospiro[inden-2,3'-pyrrolo[2,3-b]pyridin]-2'(1'H)-on

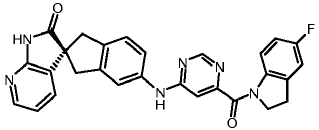

0.10 g (0.40 mmol) (S)-5-Amino-1,3-dihydrospiro[inden-2,3'-pyrrolo[2,3-b]pyridin]-2'(1'H)-on, 0.11 g (0.40 mmol) (6-Chlor-pyrimidin-4-yl)-(5-fluor-2,3-dihydro-indol-1-yl)-methanon und 13 µL einer 4 molaren wässrigen Salzsäurelösung wurden in 2.0 mL 2-Propanol zusammengegeben und 4 h unter Rückfluß gekocht. Dann wurde der Reaktionsansatz in DMF gelöst und mittels präparativer HPLC-MS gereinigt. Die Produkt enthaltenden Fraktionen wurden vereinigt und das organische Lösungsmittel wurde eingeengt. Der Rückstand wurde mit einer 1 N wässrigen Natriumhydroxid-Lösung alkalisch gestellt, der ausgefallene Niederschlag abgesaugt, mit Wasser nachgewaschen und im HV getrocknet.

| | |
|---|---|
| Ausbeute: | 45 mg (23 % d.Th.) |
| ESI-MS: | m/z = 493 (M+H)⁺ |
| Rₜ (HPLC-MS): | 1.41 min (Methode C) |

### Beispiel 39:

### 5-(6-(5,6-Difluorindolin-1-carbonyl)pyrimidin-4-ylamino)-1,3-dihydrospiro[inden-2,3'-pyrrolo[2,3-b]pyridin]-2'(1'H)-on

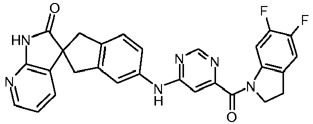

0.10 g (0.24 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 50 mg (0.26 mmol) 5,6-Difluor-2,3-di-hydro-1 H-indol, 0.10 mL (0.72 mmol) TEA und 80 mg (0.25 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 45 mg (36 % d.Th.) |
| ESI-MS: | m/z = 511 (M+H)⁺ |
| Rₜ (HPLC-MS): | 1.61 min (Methode C) |

### Beispiel 40:

### 5-(6-(3-Methylindolin-1-carbonyl)pyrimidin-4-ylamino)-1,3-dihydrospiro[inden-2,3'-pyrro-lo[2,3-b]pyridin]-2'(1'H)-on

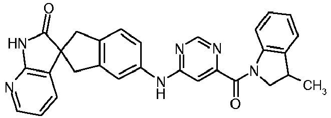

0.15 g (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 61 mg (0.37 mmol) 3-Methyl-2,3-di-hydro-1H-indol, 150 µL (0.87 mmol) DIPEA und 0.13 g (0.41 mmol) TBTU in 1.8 mL DMF wurden 1 h bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 100 mg (56 % d.Th.) |
| ESI-MS: | m/z = 489 (M+H)⁺ |
| Rₜ (HPLC-MS): | 1.58 min (Methode C) |

### Beispiel 41:

### 5-(6-(2,3-Dihydro-1H-pyrrolo[3,2-c]pyridin-1-carbonyl)pyrimidin-4-ylamino)-1,3-dihydro-spiro[inden-2,3'-pyrrolo[2,3-b]pyridin]-2'(1'H)-on

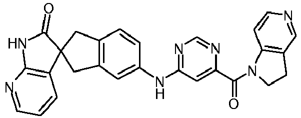

0.15 g (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 45 mg (0.38 mmol) 2,3-Dihydro-1H-pyrrolo[3,2-c]pyridin, 0.15 mL (0.87 mmol) DIPEA und 0.13 g (0.41 mmol) TBTU in 1.8 mL DMF wurden über Nacht bei RT gerührt. Dann wurde der Reaktionsansatz mit Wasser versetzt, der ausgefallene Niederschlag abgesaugt und in DMF/NMP gelöst. Die Reinigung erfolgte mittels präparativer HPLC-MS. Die Produkt enthaltenden Fraktionen wurden vereinigt und lyophilisiert. Es erfolgte noch eine zweite Reinigung mittels präparativer HPLC-MS.

| | |
|---|---|
| Ausbeute: | 9 mg (5 % d.Th.) |
| ESI-MS: | m/z = 476 (M+H)⁺ |

### Beispiel 42:

### 5-(6-(2-Ethylindolin-1-carbonyl)pyrimidin-4-ylamino)-1,3-dihydrospiro[inden-2,3'-pyrro-lo[2,3-b]pyridin]-2'(1'H)-on

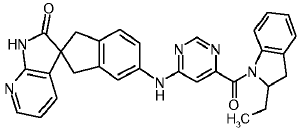

103 mg (0.40 mmol) 5-Amino-1,3-dihydrospiro[inden-2,3'-pyrrolo[2,3-b]pyridin]-2'(1'H)-on, 113 mg (0.40 mmol) (6-Chlor-pyrimidin-4-yl)-(2-ethyl-2,3-dihydro-indol-1-yl)-methanon und 13 µL einer 4 molaren wässrigen Salzsäurelösung wurden in 2.0 mL 2-Propanol zusammengegeben und 2 h unter Rückfluss gekocht. Dann wurde der Reaktionsansatz eingeengt und mittels präparativer HPLC-MS gereinigt. Die Produkt enthaltenden Fraktionen wurden vereinigt und das organische Lösungsmittel wurde eingeengt. Der Rückstand wurde mit einer 1 N wässrigen Natriumhydroxid-Lösung alkalisch gestellt, der ausgefallene Niederschlag abgesaugt, mit Wasser nachgewaschen und im HV getrocknet.

| | |
|---|---|
| Ausbeute: | 40 mg (20% d.Th.) |
| ESI-MS: | m/z = 503 (M+H)⁺ |
| Rₜ (HPLC-MS): | 1.56 min (Methode C) |

### Beispiel 43:

### 5-(6-(6-Chlorindolin-1-carbonyl)pyrimidin-4-ylamino)-1,3-dihydrospiro[inden-2,3'-pyrro-lo[2,3-b]pyridin]-2'(1'H)-on

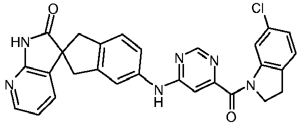

150 mg (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 63 mg (0.37 mmol) 6-Chlor-2,3-dihydro-1 H-indol, 0.15 mL (0.87 mmol) DIPEA und 130.00 mg (0.41 mmol) TBTU in 1.8 mL DMF wurden 1 h bei RT gerührt. Der Reaktionsansatz mittels präparativer HPLC-MS gereinigt. Die Produkt enthaltenden Fraktionen wurden vereinigt und abgesaugt. Der Niederschalg wurde im HV getrocknet.

| | |
|---|---|
| Ausbeute: | 70 mg (38% d.Th.) |
| ESI-MS: | m/z = 509 (M+H)⁺ |
| Rₜ (HPLC-MS): | 1.64 min (Methode C) |

### Beispiel 44:

### N-Ethyl-6-(2'-oxo-1,1',2',3-tetrahydrospiro[inden-2,3'-pyrrolo[2,3-b]pyridin]-5-ylamino)-N-phenylpyrimidin-4-carboxamid

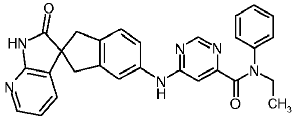

100 mg (0.24 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 34 µL (0.24 mmol) N-Ethylanilin, 0.10 mL (0.72 mmol) TEA und 85 mg (0.27 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 38 mg (33 % d.Th.) |
| ESI-MS: | m/z = 477 (M+H)⁺ |
| Rₜ (HPLC-MS): | 1.43 min (Methode C) |

### Beispiel 45:

### 5-(6-(1,2,3,4-Tetrahydrochinolin-1-carbonyl)pyrimidin-4-ylamino)-1,3-dihydrospiro[inden-2,3'-pyrrolo[2,3-b]pyridin]-2'(1'H)-on

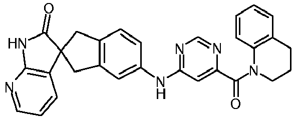

150 mg (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 50 µL (0.40 mmol) 1,2,3,4-Tetrahydro-chinolin, 0.15 mL (0.87 mmol) DIPEA und 130 mg (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 40 mg (20 % d.Th.) |
| ESI-MS: | m/z = 489 (M+H)⁺ |
| Rₜ (HPLC-MS): | 1.43 min (Methode C) |

### Beispiel 46:

### 5-(6-(6-Hydroxyindolin-1-carbonyl)pyrimidin-4-ylamino)-1,3-dihydrospiro[inden-2,3'-pyrro-lo[2,3-b]pyridin]-2'(1'H)-on

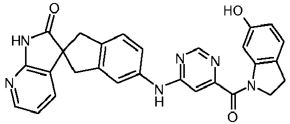

150 mg (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 50 mg (0.37 mmol) 2,3-Dihydro-1H-indol-6-ol, 0.15 mL (0.87 mmol) DIPEA und 130 mg (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 75 mg (42% d.Th.) |
| ESI-MS: | m/z = 491 (M+H)⁺ |
| Rₜ (HPLC-MS): | 1.36 min (Methode C) |

### Beispiel 47:

### N-Benzyl-N-ethyl-6-(2'-oxo-1,1',2',3-tetrahydrospiro[inden-2,3'-pyrrolo[2,3-b]pyridin]-5-yl-amino)pyrimidin-4-carboxamid

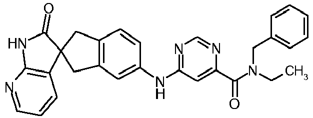

100 mg (0.24 mmol) 6-(2'-oxo-1,1',2',3-tetrahydrospiro[inden-2,3'-pyrrolo[2,3-b]pyridin]-5-ylamino)pyrimidin-4-carbonsäure Hydrochlorid, 50 µL (0.33 mmol) Benzyl-ethyl-amin, 0.10 mL (0.72 mmol) TEA und 85 mg (0.27 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 40 mg (32 % d.Th.) |
| ESI-MS: | m/z = 491 (M+H)⁺ |
| Rₜ (HPLC-MS): | 1.48 min (Methode C) |

### Beispiel 48:

### 5-(4-(2-Methylindolin-1-carbonyl)pyridin-2-ylamino)-1,3-dihydrospiro[inden-2,3'-pyrrolo-[2,3-b]pyridin]-2'(1'H)-on

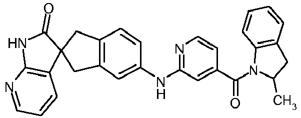

150 mg (0.40 mmol) 2-(2'-Oxo-1,1',2',3-tetrahydrospiro[inden-2,3'-pyrrolo[2,3-b]pyridin]-5-ylamino)isonicotinsäure, 55 µL (0.42 mmol) 2-Methylindolin, 0.15 mL (0.87 mmol) DIPEA und 130 mg (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 40 mg (20 % d.Th.) |
| ESI-MS: | m/z = 488 (M+H)⁺ |
| Rₜ (HPLC-MS): | 1.52 min (Methode C) |

### Beispiel 49:

### 5-(2-(2-Ethyl-6-fluorindolin-1-carbonyl)pyridin-4-ylamino)-1,3-dihydrospiro[inden-2,3'-pyrrolo[2,3-b]pyridin]-2'(1'H)-on

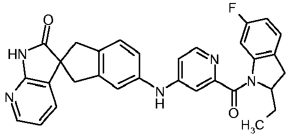

186 mg (0.50 mmol) 4-(2'-Oxo-1,1',2',3-tetrahydrospiro[inden-2,3'-pyrrolo[2,3-b]pyridin]-5-ylamino)picolinsäure, 220 mg (0.60 mmol) 2-Ethyl-6-fluor-2,3-dihydro-1H-indol, 0.26 mL (1.5 mmol) DIPEA und 0.18 g (0.55 mmol) TBTU in 5.0 mL DMF wurden über Nacht bei RT gerührt. Die Reinigung erfolgte mittels präparativer HPLC-MS. Die Produkt enthaltenden Fraktionen wurden vereinigt und das organische Lösungsmittel wurde eingeengt. Der wässrige Rückstand wurde mit 1 N wässriger Natriumhydroxid-Lösung alkalisch gestellt, der ausgefallene Niederschlag wurde abgesaugt, mit Wasser nachgewaschen und im HV getrocknet.

| | |
|---|---|
| Ausbeute: | 45 mg (17 % d.Th.) |
| ESI-MS: | m/z = 520 (M+H)⁺ |
| Rₜ (HPLC-MS): | 1.39 min (Methode C) |

### Beispiel 50:

### 5-(6-(2-(Hydroxymethyl)indolin-1-carbonyl)pyrimidin-4-ylamino)-1,3-dihydrospiro[inden-2,3'-pyrrolo[2,3-b]pyridin]-2'(1'H)-on

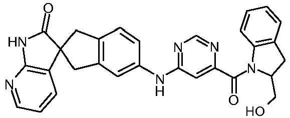

100 mg (0.24 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 45 mg (0.30 mmol) (2,3-Dihydro-1H-indol-2-yl)-methanol, 0.10 mL (0.71 mmol) TEA und 88 mg (0.27 mmol) TBTU in 1.0 mL DMF wurden über Nacht bei RT gerührt. Die Reinigung erfolgte mittels präparativer HPLC-MS. Die Produkt enthaltenden Fraktionen wurden vereinigt und das Lösungsmittel wurde auf ca. die Hälfte eingeengt. Der ausgefallene Niederschlag wurde abgesaugt und getrocknet.

| | |
|---|---|
| Ausbeute: | 39 mg (32% d.Th.) |
| ESI-MS: | m/z = 505 (M+H)⁺ |
| Rₜ (HPLC-MS): | 2.90 min (Methode E) |

### Beispiel 51:

### 5-(6-(3-(Hydroxymethyl)indolin-1-carbonyl)pyrimidin-4-ylamino)-1,3-dihydrospiro[inden-2,3'-pyrrolo[2,3-b]pyridin]-2'(1'H)-on

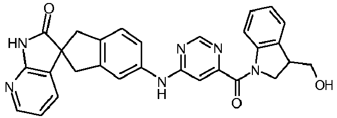

100 mg (0.24 mmol)_6-(2'-Oxo-1,1',2',3-tetrahydrospiro[inden-2,3'-pyrrolo[2,3-b]pyridin]-5-ylamino)pyrimidin-4-carbonsäure Hydrochlorid, 45 mg (0.30 mmol) (2,3-Dihydro-1H-indol-3-yl)-methanol, 0.10 mL (0.71 mmol) TEA und 88 mg (0.27 mmol) TBTU in 1.0 mL DMF wurden über Nacht bei RT gerührt. Die Reinigung erfolgte mittels präparativer HPLC-MS. Die Produkt enthaltenden Fraktionen wurden vereinigt und das Lösungsmittel wurde auf ca. die Hälfte eingeengt. Der ausgefallene Niederschlag wurde abgesaugt und getrocknet.

| | |
|---|---|
| Ausbeute: | 58 mg (45 % d.Th.) |
| ESI-MS: | m/z = 505 (M+H)⁺ |

### Beispiel 52:

### 5-(6-(2,3-Dihydro-1H-pyrrolo[2,3-b]pyridin-1-carbonyl)pyrimidin-4-ylamino)-1,3-dihydro-spiro[inden-2,3'-pyrrolo[2,3-b]pyridin]-2'(1'H)-on

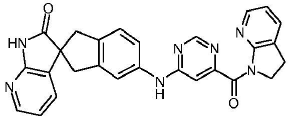

150 mg (0.37 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 50 mg (0.42 mmol) 2,3-Dihydro-1H-pyrrolo[2,3-b]pyridin, 0.15 mL (0.87 mmol) DIPEA und 130 mg (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und lyophilisiert. Dann wurde die Substanz in DMF gelöst und nochmals mittels präparativer HPLC-MS gereinigt. Die Produkt enthaltenden Fraktionen wurden vereinigt und lyophilisiert.

| | |
|---|---|
| Ausbeute: | 12 mg (6 % d.Th.) |
| ESI-MS: | m/z = 476 (M+H)⁺ |
| Rₜ (HPLC-MS): | 1.28 min (Methode C) |

### Beispiel 53:

### 5-(6-(6-Nitroindolin-1-carbonyl)pyrimidin-4-ylamino)-1,3-dihydrospiro[inden-2,3'-pyrrolo-[2,3-b]pyridin]-2'(1'H)-on

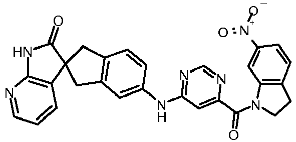

150 mg (0.37 mmol) 6-(2'-Oxo-1,1',2',3-tetrahydrospiro[inden-2,3'-pyrrolo[2,3-b]pyridin]-5-ylamino)pyrimidin-4-carbonsäure-Hydrochlorid, 60 mg (0.37mmol) 4-Nitroindol, 0.15 mL (0.87 mmol) DIPEA und 130 mg (0.41 mmol) TBTU in 1.8 mL DMF wurden über Nacht bei RT gerührt. Dann wurde der Reaktionsansatz mit Wasser/MeOH verdünnt, der ausgefallene Niederschlag abgesaugt und mit viel Wasser gewaschen. Der Niederschlag wurde getrocknet.

| | |
|---|---|
| Ausbeute: | 165 mg (74 % d.Th.) |
| ESI-MS: | m/z = 520 (M+H)⁺ |
| Rₜ (HPLC-MS): | 1.56 min (Methode C) |

### Beispiel 54:

### 5-(6-(6-Methylindolin-1-carbonyl)pyrimidin-4-ylamino)-1,3-dihydrospiro[inden-2,3'-pyrrolo-[2,3-b]pyridin]-2'(1'H)-on

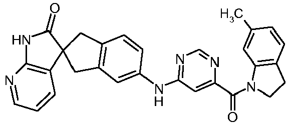

150 mg (0.37 mmol) 6-(2'-oxo-1,1',2',3-tetrahydrospiro[inden-2,3'-pyrrolo[2,3-b]pyridin]-5-ylamino)pyrimidin-4-carbonsäure Hydrochlorid, 62 mg (0.4 mmol) 6-Methyl-2,3-dihydro-1 H-indol-Hydrochlorid, 0.15 mL (0.87 mmol) DIPEA und 130 mg (0.41 mmol) TBTU in 1.8 mL DMF wurden über Nacht bei RT gerührt. Die Reinigung erfolgte mittels präparativer HPLC-MS. Die Produkt enthaltenden Fraktionen wurden vereinigt und das organische Lösungsmittel eingeengt. Der ausgefallene Niederschlag wurde abgesaugt und im HV getrocknet.

| | |
|---|---|
| Ausbeute: | 45 mg (25 % d.Th.) |
| ESI-MS: | m/z = 489 (M+H)⁺ |
| Rₜ (HPLC-MS): | 1.57 min (Methode C) |

### Beispiel 55:

### 5-(6-(6-Methoxyindolin-1-carbonyl)pyrimidin-4-ylamino)-1,3-dihydrospiro[inden-2,3'-pyrro-lo[2,3-b]pyridin]-2'(1'H)-on

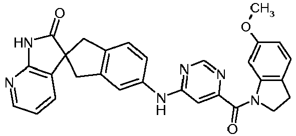

83 mg (0.20 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 30 mg (0.20 mmol) 6-Methoxy-2,3-di-hydro-1-H-indol, 51 µL (0.36 mmol) TEA und 70 mg (0.22 mmol) TBTU in 1.5 mL DMF wurden 1 h bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 53 mg (52 % d.Th.) |
| ESI-MS: | m/z = 505 (M+H)⁺ |
| Rₜ (HPLC-MS): | 1.52 min (Methode C) |

### Beispiel 56:

### 5-(6-(5,6,7,8-Tetrahydro-4H-thiazolo[5,4-d]azepin-6-carbonyl)pyrimidin-4-ylamino)-1,3-di-hydrospiro[inden-2,3'-pyrrolo[2,3-b]pyridin]-2'(1'H)-on

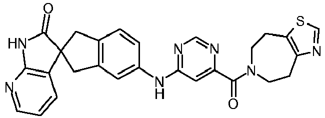

86 mg (0.21 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 37 mg (0.24 mmol) 5,6,7,8-Tetrahydro-4H-thiazolo[4,5-d]azepin, 0.13 mL (0.72 mmol) DIPEA und 77 mg (0.24 mmol) TBTU in 1.0 mL DMF wurden über Nacht bei RT gerührt. Die Reinigung erfolgte mittels präparativer HPLC-MS. Die Produkt enthaltenden Fraktionen wurden vereinigt und lyophilisiert.

| | |
|---|---|
| Ausbeute: | 16 mg (15 % d.Th.) |
| ESI-MS: | m/z = 510 (M+H)⁺ |
| Rₜ (HPLC-MS): | 1.25 min (Methode C) |

### Beispiel 57:

### 5-(6-(1,4,5,6,7,8-Hexahydroimidazo[4,5-d]azepin-6-carbonyl)pyrimidin-4-ylamino)-1,3-di-hydrospiro[inden-2,3'-pyrrolo[2,3-b]pyridin]-2'(1'H)-on

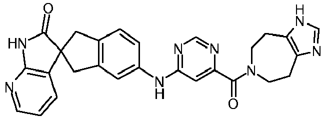

86 mg (0.21 mmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid, 33 mg (0.24 mmol) 1,4,5,6,7,8-Hexa-hydroimidazo[4,5-d]azepin, 0.13 mL (0.72 mmol) DIPEA und 77 mg (0.24 mmol) TBTU in 1.0 mL DMF wurden über Nacht bei RT gerührt. Die Reinigung erfolgte mittels präparativer HPLC-MS. Die Produkt enthaltenden Fraktionen wurden vereinigt und lyophilisiert. Dann wurde die Substanz nochmals mittels präparativer HPLC-MS gereinigt.

| | |
|---|---|
| Ausbeute: | 21 mg (19 % d.Th.) |
| ESI-MS: | m/z = 493 (M+H)⁺ |
| Rₜ (HPLC-MS): | 1.08 min (Methode C) |

### Allgemeine Arbeitsvorschrift 6 (AAV6) zur Umsetzung von 6-(2'-Oxo-1,1',2',3-tetrahydro-spiro[inden-2,3'-pyrrolo[2,3-b]pyridin]-5-ylamino)pyrimidin-4-carbonsäure Hydrochlorid mit Amin-Derivaten:

Zu 4.10 mg (10 µmol) 6-(2'-Oxo-1,1',2',3-tetrahydrospiro[inden-2,3'-pyrrolo[2,3-b]pyridin]-5-ylamino)pyrimidin-4-carbonsäure Hydrochlorid in 0.32 mL DMF wurden 1.2 Äquivalente (12 µmol) des Amin-Derivats, 3.4 Äquivalente DIPEA (34 µmol) und 1.2 Äquivalente (12 µmol) HATU gegeben und über Nacht bei 80°C geschüttelt. Der Reaktionsansatz wurde in der Vakuumzentrifuge bei 60°C eingedampft. Analog dieser Arbeitsvorschrift konnten folgende Verbindungen synthetisiert werden:

| Beispiel Methode | Struktur | Amin-Derivat [Menge Amin-Derivat] | Analytische Daten |
|---|---|---|---|
| Beispiel 58: AAV6 | 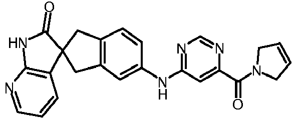 | 2,5-Dihydropyrrol | ESI-MS: m/z = 425 [M+H]⁺ |
| | 5-(6-(2,5-Dihydro-1H-pyrrol-1-carbonyl)pyrimidin-4-ylamino)-1,3-dihydrospiro[inden-2,3'-pyrrolo[2,3-b]pyridin]-2'(1'H)-on | 0.83 mg (12 µmol) | Rₜ: 1.45 min (Methode Q) |
| Beispiel 59: AAV6 | 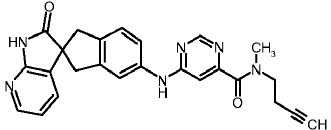 | But-3-ynyl-methylamin | ESI-MS: m/z = 439 [M+H]⁺ |
| | N-(But-3-ynyl)-N-methyl-6-(2'-oxo-1,1',2',3-tetrahydrospiro-[inden-2,3'-pyrrolo[2,3-b]pyridin]-5-ylamino)pyrimidin-4-carbox-amid | 1.00 mg (12 µmol) | Rₜ: 1.46 min (Methode Q) |
| Beispiel 60: AAV6 | 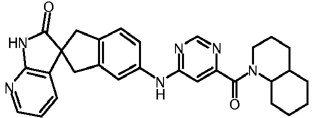 | Decahydrochinolin | ESI-MS: m/z = 495 [M+H]⁺ |
| | 5-(6-(Decahydrochinolin-1-carbonyl)pyrimidin-4-ylamino)-1,3-dihydrospiro[inden-2,3'-pyrrolo[2,3-b]pyridin]-2'(1'H)-on | 1.67 mg (12 µmol) | Rₜ: 1.68 min (Methode Q) |
| Beispiel 61: AAV6 | 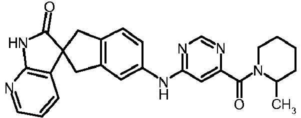 | 2-Methylpiperidin | ESI-MS: m/z = 455 [M+H]⁺ |
| | 5-(6-(2-Methylpiperidin-1-carbonyl)pyrimidin-4-ylamino)-1,3-dihydrospiro[inden-2,3'-pyrrolo[2,3-b]pyridin]-2'(1'H)-on | 1.19 mg (12 µmol) | Rₜ:1.53 min (Methode Q) |
| Beispiel 62: AAV6 | 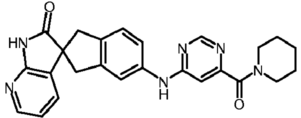 | Piperidin | ESI-MS: m/z =441 [M+H]⁺ |
| | 5-(6-(Piperidin-1-carbonyl)pyrimi-din-4-ylamino)-1,3-dihydrospiro-[inden-2,3'-pyrrolo[2,3-b]pyridin]-2'(1'H)-on | 1.02 mg (12 µmol) | Rₜ: 1.47 min (Methode Q) |
| Beispiel 63: AAV6 | 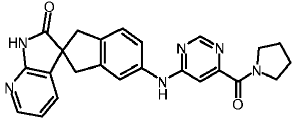 | Pyrrolidin | ESI-MS: m/z = 427 [M+H]⁺ |
| | 5-(6-(Pyrrolidin-1-carbonyl)pyrimidin-4-ylamino)-1,3-dihydro-spiro[inden-2,3'-pyrrool[2,3-b]-pyridin]-2'(1'H)-on | 0.85 mg (12 µmol) | Rₜ: 1.42 min (Methode Q) |
| | | | |
| Beispiel 64: AAV6 | 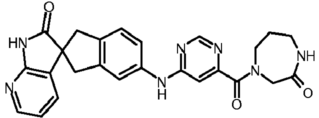 | 5-Homopiperazinon | ESI-MS: m/z = 470 [M+H]⁺ |
| | 5-(6-(3-Oxo-1,4-diazepan-1-carbonyl)pyrimidin-4-ylamino)-1,3-dihydrospiro[inden-2,3'-pyrrolo[2,3-b]pyridin]-2'(1'H)-on | 1.37 mg (12 µmol) | Rₜ: 1.33 min (Methode Q) |
| Beispiel 65: AAV6 | 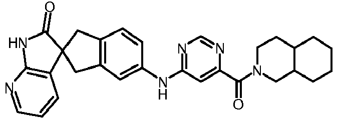 | Decahydroisochinolin | ESI-MS: m/z = 248 [M+2H]⁺⁺ |
| | 5-(6-(Decahydroisochinolin-2-carbonyl)pyrimidin-4-ylamino)-1,3-dihydrospiro[inden-2,3'-pyrrolo[2,3-b]pyridin]-2'(1'H)-on | 1.67 mg (12 µmol) | Rₜ: 1.72 min (Methode Q) |

Die nachfolgenden Beispiele beschreiben die Herstellung pharmazeutischer Anwendungsformen, die als Wirkstoff eine beliebige Verbindung der allgemeinen Formel **I** enthalten:

### Beispiel I

Kapseln zur Pulverinhalation mit 1 mg Wirkstoff

### Zusammensetzung:

**1 Kapsel zur Pulverinhalation enthält:**

| | |
|---|---|
| Wirkstoff | 1.0 mg |
| Milchzucker | 20.0 mg |
| Hartgelatinekapseln | 50.0 mg |
| | 71.0 mg |

### Herstellungsverfahren:

Der Wirkstoff wird auf die für Inhalativa erforderliche Korngröße gemahlen. Der gemahlene Wirkstoff wird mit dem Milchzucker homogen gemischt. Die Mischung wird in Hartgelatinekapseln abgefüllt.

### Beispiel II

Inhalationslösung für Respimat^{®} mit 1 mg Wirkstoff

### Zusammensetzung:

**1 Hub enthält:**

| | |
|---|---|
| Wirkstoff | 1.0 mg |
| Benzalkoniumchlorid | 0.002 mg |
| Dinatriumedetat | 0.0075 mg |
| Wasser gereinigt ad | 15.0 µl |

### Herstellungsverfahren:

Der Wirkstoff und Benzalkoniumchlorid werden in Wasser gelöst und in Respimat^{®}-Kartuschen abgefüllt.

### Beispiel III

Inhalationslösung für Vernebler mit 1 mg Wirkstoff

### Zusammensetzung:

**1 Fläschchen enthält:**

| | |
|---|---|
| Wirkstoff | 0.1 g |
| Natriumchlorid | 0.18 g |
| Benzalkoniumchlorid | 0.002 g |
| Wasser gereinigt ad | 20.0 ml |

### Herstellungsverfahren:

Wirkstoff, Natriumchlorid und Benzalkoniumchlorid werden in Wasser gelöst.

### Beispiel IV

Treibgas-Dosieraerosol mit 1 mg Wirkstoff

### Zusammensetzung:

**1 Hub enthält:**

| | |
|---|---|
| Wirkstoff | 1.0 mg |
| Lecithin | 0.1 % |
| Treibgas ad | 50.0 µl |

### Herstellungsverfahren:

Der mikronisierte Wirkstoff wird in dem Gemisch aus Lecithin und Treibgas homogen suspendiert. Die Suspension wird in einen Druckbehälter mit Dosierventil abgefüllt.

### Beispiel V

Nasalspray mit 1 mg Wirkstoff

### Zusammensetzung:

| | |
|---|---|
| Wirkstoff | 1.0 mg |
| Natriumchlorid | 0.9 mg |
| Benzalkoniumchlorid | 0.025 mg |
| Dinatriumedetat | 0.05 mg |
| Wasser gereinigt ad | 0.1 ml |

### Herstellungsverfahren:

Der Wirkstoff und die Hilfsstoffe werden in Wasser gelöst und in ein entsprechendes Behältnis abgefüllt.

### Beispiel VI

Injektionslösung mit 5 mg Wirksubstanz pro 5 ml

### Zusammensetzung:

| | |
|---|---|
| Wirksubstanz | 5 mg |
| Glucose | 250 mg |
| Human-Serum-Albumin | 10 mg |
| Glykofurol | 250 mg |
| Wasser für Injektionszwecke ad | 5 ml |

### Herstellung:

Glykofurol und Glucose in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

### Beispiel VII

Injektionslösung mit 100 mg Wirksubstanz pro 20 ml

### Zusammensetzung:

| | |
|---|---|
| Wirksubstanz | 100 mg |
| Monokaliumdihydrogenphosphat = KH₂PO₄ | 12 mg |
| Dinatriumhydrogenphosphat = Na₂HPO₄*2H₂O | 2 mg |
| Natriumchlorid | 180 mg |
| Human-Serum-Albumin | 50 mg |
| Polysorbat 80 | 20 mg |
| Wasser für Injektionszwecke ad | 10 ml |

### Herstellung:

Polysorbat 80, Natriumchlorid, Monokaliumdihydrogenphosphat und Dinatriumhydrogenphosphat in Wasser für Injektionszwecke (Wfl) auflösen; Human-Serum-Albumin zugeben; Wirkstoff unter Erwärmen auflösen; mit Wfl auf Ansatzvolumen auffüllen; in Ampullen abfüllen.

### Beispiel VIII

Lyophilisat mit 10 mg Wirksubstanz

### Zusammensetzung:

| | |
|---|---|
| Wirksubstanz | 10 mg |
| Mannit | 300 mg |
| Human-Serum-Albumin | 20 mg |
| Wasser für Injektionszwecke ad | 2 ml |

### Herstellung:

Mannit in Wasser für Injektionszwecke (Wfl) auflösen; Human-Serum-Albumin zugeben; Wirkstoff unter Erwärmen auflösen; mit Wfl auf Ansatzvolumen auffüllen; in Vials abfüllen; gefriertrocknen.

### Lösungsmittel für Lyophilisat:

| | |
|---|---|
| Polysorbat 80 = Tween 80 | 20 mg |
| Mannit | 200 mg |
| Wasser für Injektionszwecke ad | 10 ml |

### Herstellung:

Polysorbat 80 und Mannit in Wasser für Injektionszwecke (Wfl) auflösen; in Ampullen abfüllen.

### Beispiel IX

Tabletten mit 20 mg Wirksubstanz

### Zusammensetzung:

| | |
|---|---|
| Wirksubstanz | 20 mg |
| Lactose | 120 mg |
| Maisstärke | 40 mg |
| Magnesiumstearat | 2 mg |
| Povidon K 25 | 18 mg |

### Herstellung:

Wirksubstanz, Lactose und Maisstärke homogen mischen; mit einer wässrigen Lösung von Povidon granulieren; mit Magnesiumstearat mischen; auf einer Tablettenpresse abpressen; Tablettengewicht 200 mg.

### Beispiel X

Kapseln mit 20 mg Wirksubstanz

### Zusammensetzung:

| | |
|---|---|
| Wirksubstanz | 20 mg |
| Maisstärke | 80 mg |
| Kieselsäure, hochdispers | 5 mg |
| Magnesiumstearat | 2.5 mg |

### Herstellung:

Wirksubstanz, Maisstärke und Kieselsäure homogen mischen; mit Magnesiumstearat mischen; Mischung auf einer Kapselfüllmaschine in Hartgelatine-Kapseln Größe 3 abfüllen.

### Beispiel XI

Zäpfchen mit 50 mg Wirksubstanz

### Zusammensetzung:

| | |
|---|---|
| Wirksubstanz | 50 mg |
| Hartfett (Adeps solidus) q.s. ad | 1700 mg |

### Herstellung:

Hartfett bei ca. 38°C aufschmelzen; gemahlene Wirksubstanz im geschmolzenen Hartfett homogen dispergieren; nach Abkühlen auf ca. 35°C in vorgekühlte Formen ausgießen.

### Beispiel XII

Injektionslösung mit 10 mg Wirksubstanz pro 1 mL

### Zusammensetzung:

| | |
|---|---|
| Wirksubstanz | 10 mg |
| Mannitol | 50 mg |
| Human-Serum-Albumin | 10 mg |
| Wasser für Injektionszwecke ad | 1 ml |

### Herstellung:

Mannitol in Wasser für Injektionszwecke auflösen (Wfl); Human-Serum-Albumin zugeben; Wirkstoff unter Erwärmen auflösen; mit Wfl auf Ansatzvolumen auffüllen; unter Stickstoff-Begasung in Ampullen abfüllen.

## Patentansprüche

1. Verbindungen der allgemeinen Formel **I** 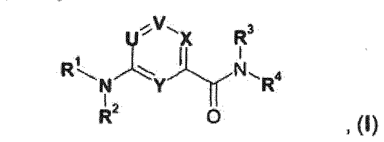 in der
**R¹** eine Gruppe der allgemeinen Formeln **IIa** oder **IIb** 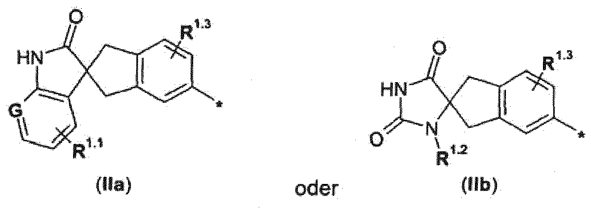 und
**R²** H oder C₁₋₃-Alkyl,
**G** C-**R^{1.1}** oder N,
**R^{1.1}** unabhängig voneinander
(a) H,
(b) Halogen, C₁₋₃-Alkyl, -OH, -CN, C₁₋₃-Alkyl-O-. -C(O)-O-C₁₋₃-Alkyl, C₂₋₄-Alkenyl,-C₂₋₄-Alkinyl, C₁₋₃-Alkyl-S-, Cyclopropyl, -NH₂, -COOH, -NH-C(O)-O-C₁₋₃-Alkyl, -NH-C(O)-C₁₋₃-Alkyl,
(c) eine C₁₋₃-Alkylgruppe oder C₁₋₃-Alkyl-O-gruppe, in der jede Methylengruppe mit bis zu zwei Fluoratomen und jede Methylgruppe mit bis zu drei Fluoratomen substituiert ist,
**R^{1.2}** unabhängig voneinander
(a) H oder
(b) C₁₋₃-Alkyl,
**R^{1.3}** (a) H,
(b) F, -CN, C₁₋₃-Alkyl, -CO₂-**R^{1.3.1}** oder
(c) eine C₁₋₃-Alkylgruppe. in der jede Methylengruppe mit bis zu zwei Fluoratomen und jede Methylgruppe mit bis zu drei Fluoratomen substituiert sein kann,
**R^{1.3.1}** (a) H,
(b) C₁₋₆-Alkyl,
**R³** (a) H,
(b) C₁₋₃-Alkyl oder
(c) eine C₁₋₃-Alkylgruppe, in der jede Methylengruppe mit bis zu zwei Fluoratomen und jede Methylgruppe mit bis zu drei Fluoratomen substituiert ist, und
**R⁴** H, Cyclopropyl oder eine Gruppe ausgewählt aus 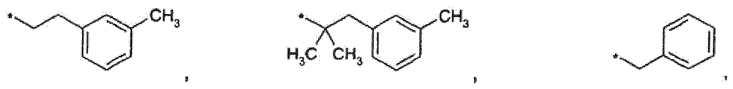 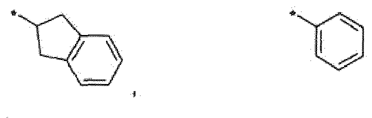 oder
**R³** und **R⁴** zusammen mit dem Stickstoffatom, an das sie gebunden sind, eine Gruppe ausgewählt aus 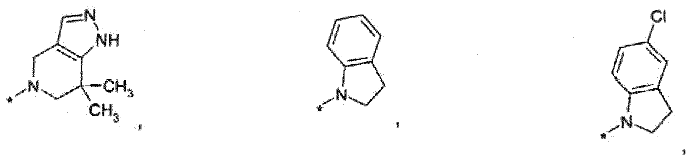 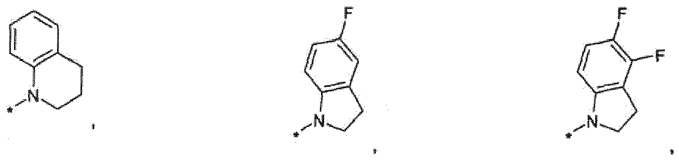 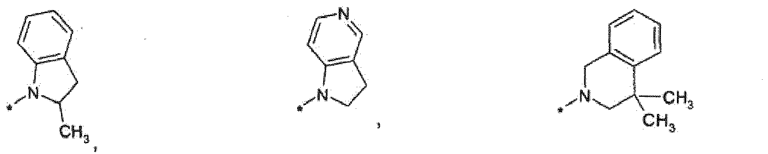 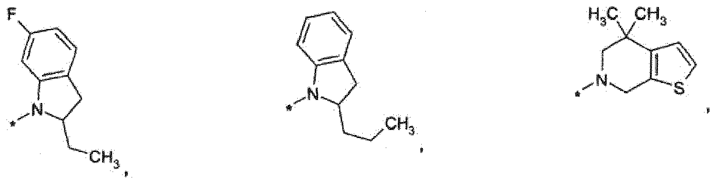 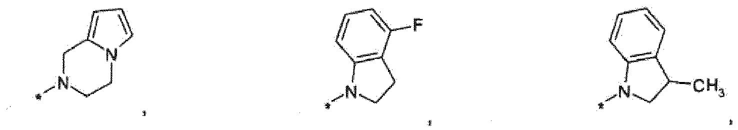 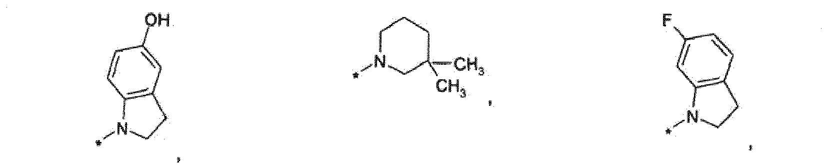 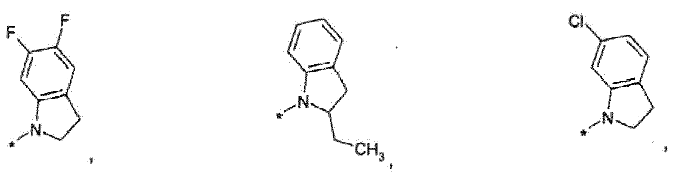 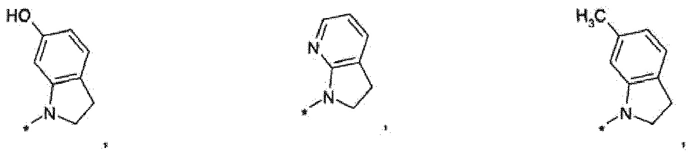 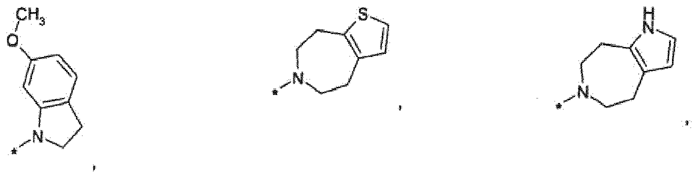 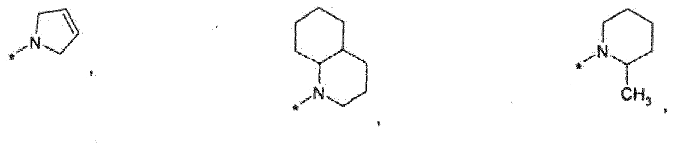 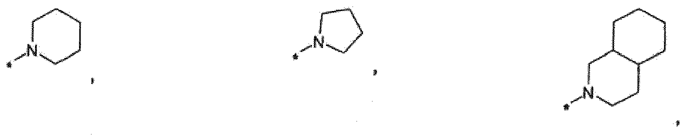 und
der Ring 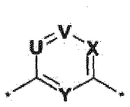 eine Gruppe ausgewählt aus 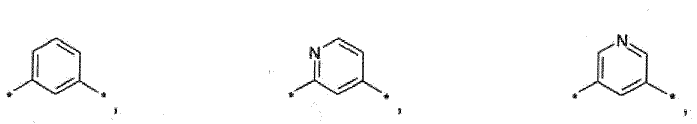 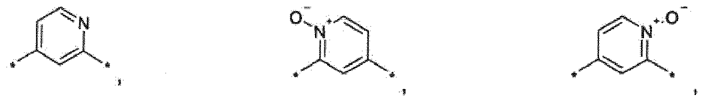 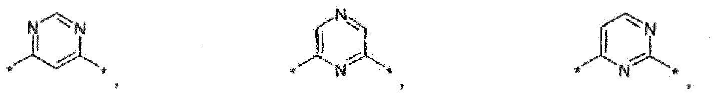 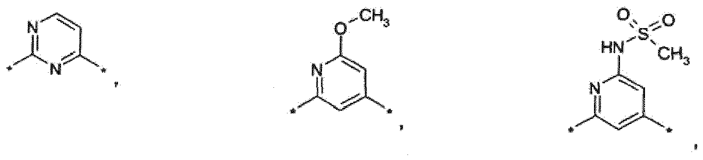 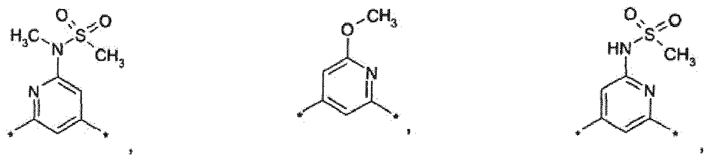 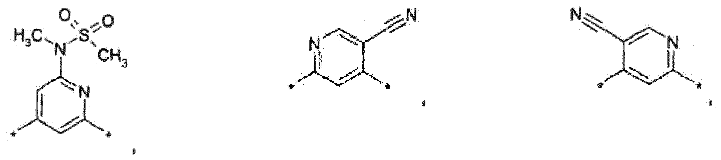 deren Tautomere, deren Diastereomere, deren Enantiomere, deren Hydrate, deren Gemische und deren Salze sowie die Hydrate der Salze, insbesondere deren physiologisch verträglichen Salze mit anorganischen oder organischen Säuren oder Basen.

2. Verbindungen der allgemeinen Formel **I** gemäß Anspruch 1, in denen **U, V, X, Y, R³** und **R⁴** wie in Anspruch 1 definiert sind und
**R¹** eine Gruppe ausgewählt aus 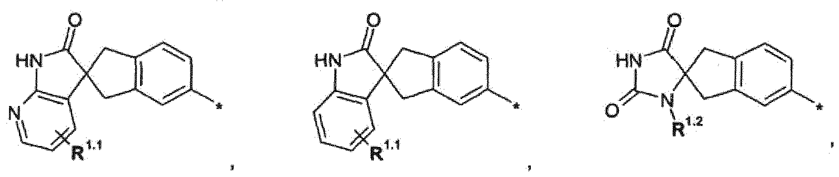
**R^{1.1}** (a) H,
(b) Halogen, C₁₋₃-Alkyl, -OH, -CN, -O-C₁₋₃-Alkyl, -C(O)-O-C₁₋₃-Alkyl, C₂₋₄-Alkenyl. C₂₋₄-Alkinyl, C₁₋₃-Alkyl-S-, -NH₂,
(c) eine C₁₋₃-Alkylgruppe oder C₁₋₃-Alkyl-O-gruppe, in der jede Methylengruppe mit bis zu zwei Fluoratomen und jede Methylgruppe mit bis zu drei Fluoratomen substituiert ist,
**R^{1.2}** H oder CH₃ und
**R²** H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

3. Verbindungen der allgemeinen Formel **I** gemäß Anspruch 1, in denen **U**, **V**, **X**, **Y**,
**R³** und **R⁴** wie in Anspruch 1 definiert sind und
**R¹** eine Gruppe ausgewählt aus 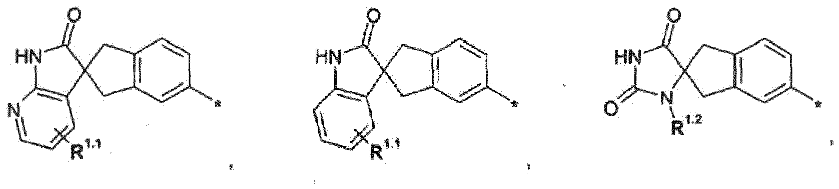
**R^{1.1}** (a) F, CH₃, -OH, -O-CH₃ oder
(b) CF₃ und
**R²** H oder C₁₋₃-Alkyl bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

4. Verbindungen der allgemeinen Formel **I** gemäß Anspruch 1, in denen **U, V**, **X, Y**, **R³** und **R⁴** wie in Anspruch 1 definiert sind und
**R¹** eine Gruppe ausgewählt aus 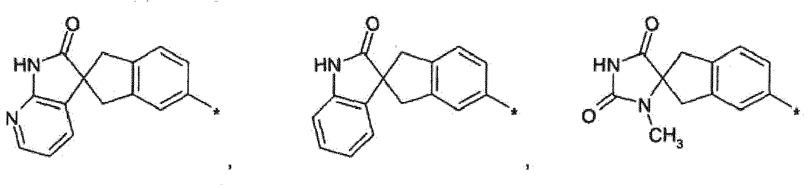 und
**R²** H bedeutet,
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

5. Verbindungen der allgemeinen Formel **I** gemäß Anspruch 1, in der
**R¹** eine Gruppe ausgewählt aus 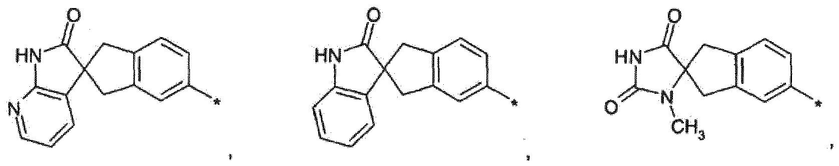
**R²** H,
**R³** (a) H,
(b) C₁₋₃-Alkyl oder
(c) eine C₁₋₃-Alkylgruppe, in der jede Methylengruppe mit bis zu zwei Fluoratomen und jede Methylgruppe mit bis zu drei Fluoratomen substituiert ist, und
**R⁴** H, Cyclopropyl oder eine Gruppe ausgewählt aus 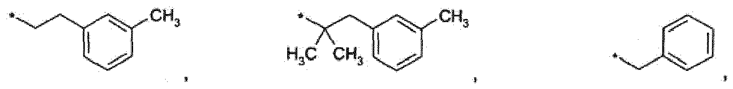 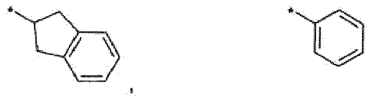
oder
**R³** und **R⁴** zusammen mit dem Stickstoffatom, an das sie gebunden sind, eine Gruppe ausgewählt aus 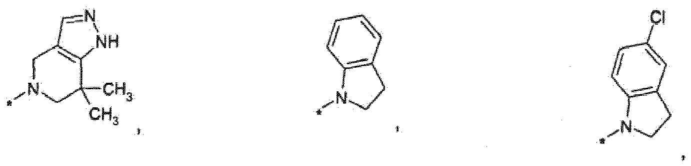 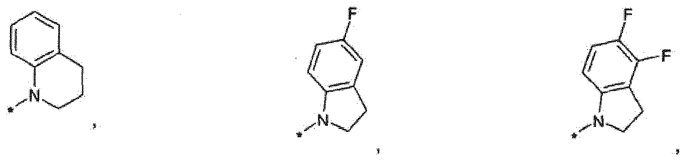 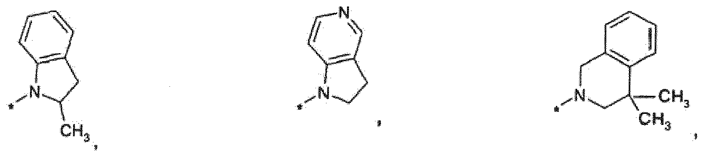 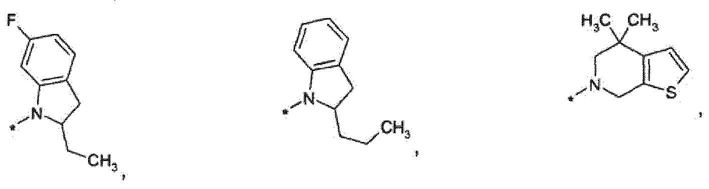 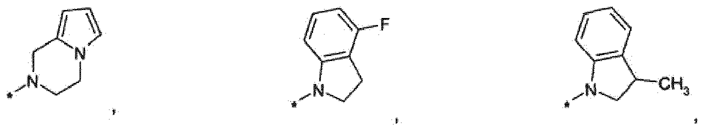 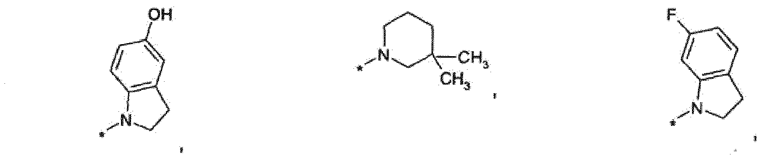 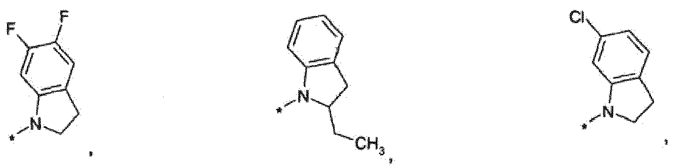 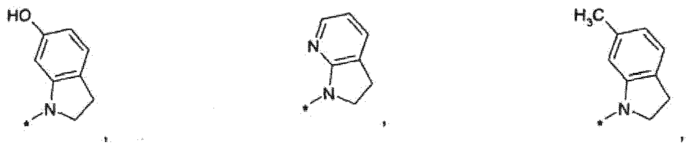 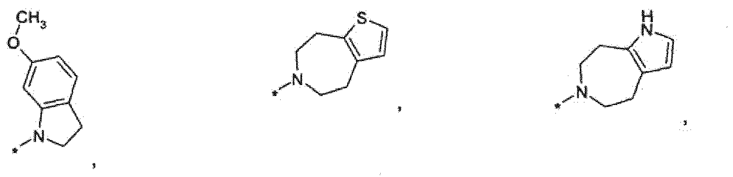 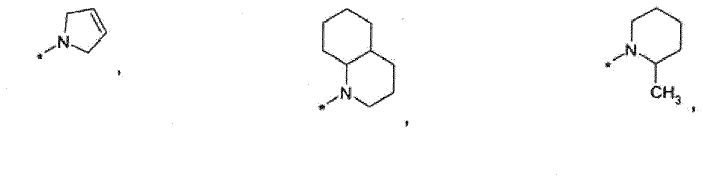 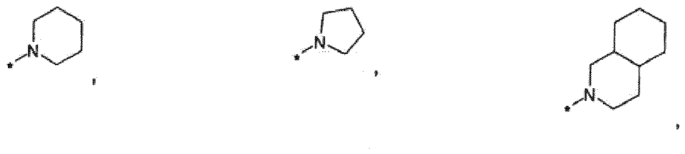 und der Ring 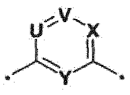 eine Gruppe ausgewählt aus 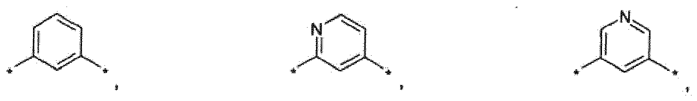 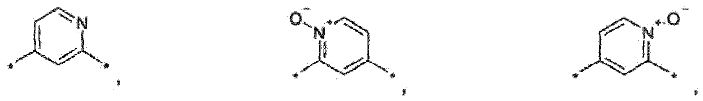 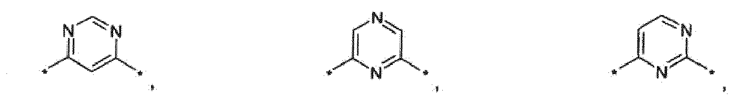 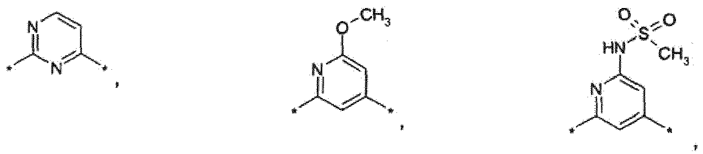 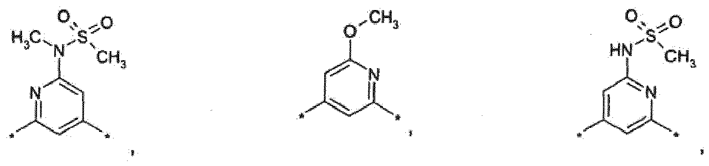 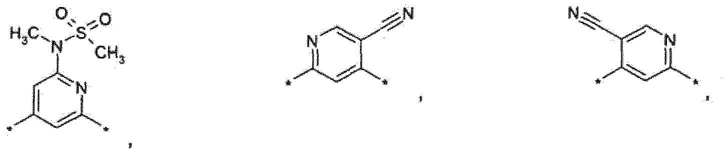 bedeutet.
deren Tautomere, deren Diastereomere, deren Enantiomere, deren Hydrate, deren Gemische und deren Salze sowie die Hydrate der Salze, insbesondere deren physiologisch verträgliche Salze mit anorganischen oder organischen Säuren oder Basen.

6. Folgende Verbindungen der allgemeinen Formel **I** gemäß Anspruch 1:
| **Nr.** | **Struktur** |
|---|---|
| (1) | 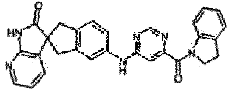 |
| (2) | 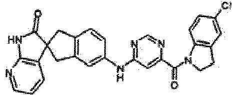 |
| (3) | 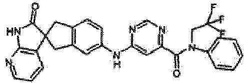 |
| (4) | 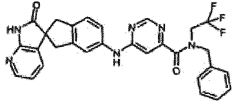 |
| (5) | 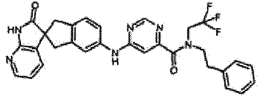 |
| (6) | 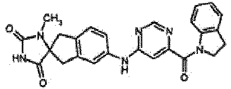 |
| (7) | 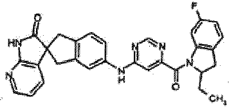 |
| (8) | 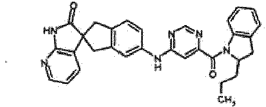 |
| (9) | 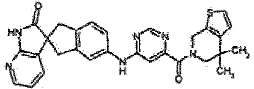 |
| (10) | 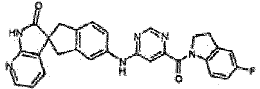 |
| (11) | 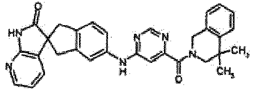 |
| (12) | 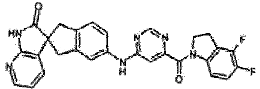 |
| (13) | 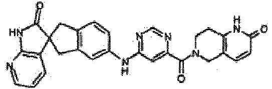 |
| (14) | 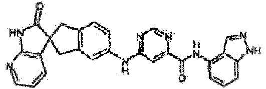 |
| (15) | 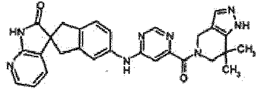 |
| (16) | 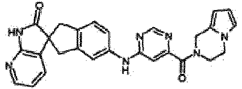 |
| (17) | 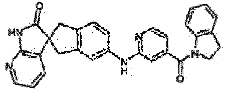 |
| (18) | 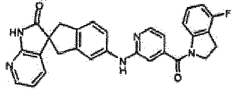 |
| (19) | 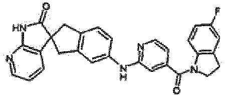 |
| (20) | 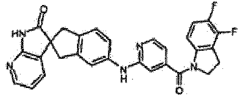 |
| (21) | 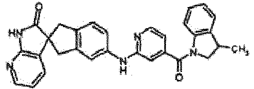 |
| (22) | 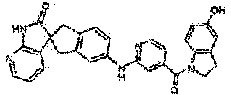 |
| (23) | 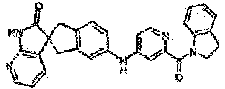 |
| (24) | 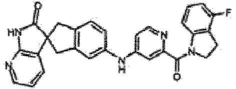 |
| (25) | 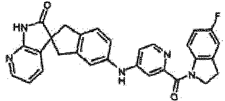 |
| (26) | 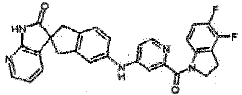 |
| (27) | 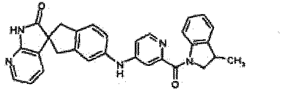 |
| (28) | 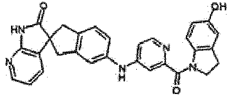 |
| (29) | 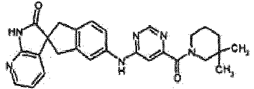 |
| (30) | 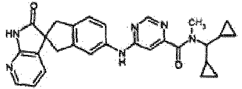 |
| (31) | 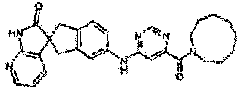 |
| (32) | 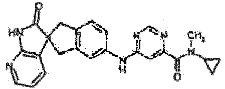 |
| (33) | 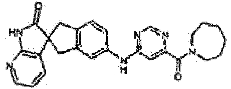 |
| (34) | 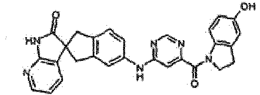 |
| (35) | 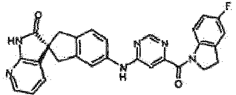 |
| (36) | 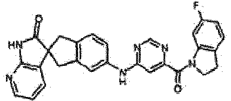 |
| (37) | 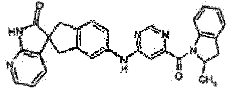 |
| (38) | 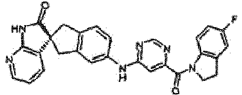 |
| (39) | 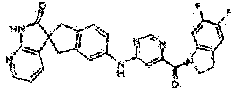 |
| (40) | 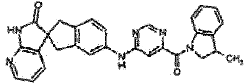 |
| (41) | 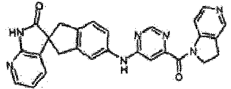 |
| (42) | 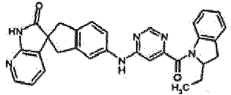 |
| (43) | 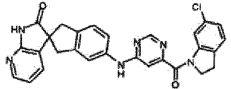 |
| (44) | 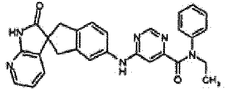 |
| (45) | 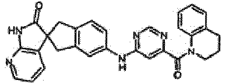 |
| (46) | 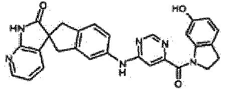 |
| (47) | 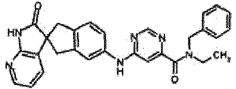 |
| (48) | 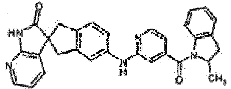 |
| (49) | 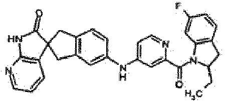 |
| (50) | 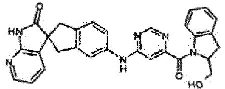 |
| (51) | 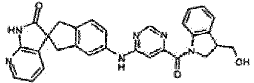 |
| (52) | 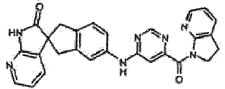 |
| (53) | 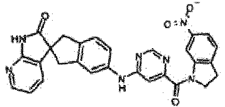 |
| (54) | 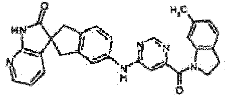 |
| (55) | 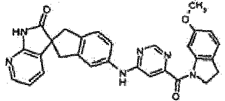 |
| (56) | 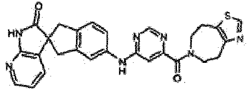 |
| (57) | 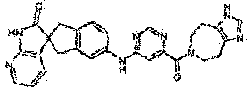 |
| (58) | 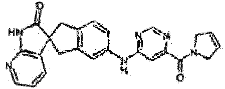 |
| (59) | 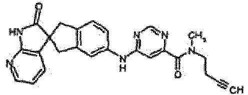 |
| (60) | 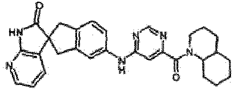 |
| (61) | 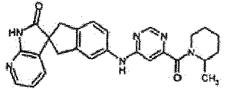 |
| (62) | 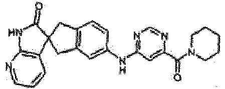 |
| (63) | 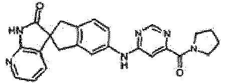 |
| (64) | 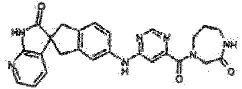 |
| (65) | 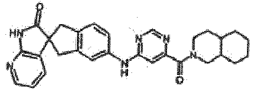 |
deren Enantiomere, deren Diastereomere, deren Hydrate, deren Gemische und deren Salze sowie die Hydrate der Salze, insbesondere deren physiologisch verträgliche Salze mit anorganischen oder organischen Säuren oder Basen.

7. Physiologisch verträgliche Salze der Verbindungen nach einem der Ansprüche 1 bis 6 mit anorganischen oder organischen Säuren oder Basen.

8. Arzneimittel, enthaltend eine Verbindung nach einem der Ansprüche 1 bis 6 oder ein physiologisch verträgliches Satz gemäß Anspruch 7 neben gegebenenfalls einem oder mehreren inerten Trägerstoffen und/oder Verdünnungsmitteln.

9. Verwendung einer Verbindung nach einem der Ansprüche 1 bis 7 zur Herstellung eines Arzneimittels zur akuten und prophylaktischen Behandlung von Kopfschmerzen, insbesondere Migräne- und Cluster-Kopfschmerz sowie Spannungskopfschmerzen.

10. Verwendung einer Verbindung nach einem der Ansprüche 1 bis 7 zur Herstellung eines Arzneimittels zur Behandlung von nicht-insulinabhängigem Diabetes mellitus ("NIDDM"), cardiovaskulärer Erkrankungen, Morphintoleranz, Clostridiumtoxin-bedingter Durchfallerkrankungen, Erkrankungen der Haut, insbesondere thermischer und strahlenbedingter Hautschäden inklusive Sonnenbrand, Lichen, Prurigo, pruriginöse Toxidermien sowie schwerer Juckreiz, entzündlicher Erkrankungen, z.B. entzündlicher Gelenkerkrankungen (Osteoarthritis, rheumatoide Arthritis, neurogene Arthritis), generalisierter Weichteilrheumatismus (Fibromyalgie), neurogener Entzündungen der oralen Mucosa, entzündlicher Lungenerkrankungen, allergischer Rhinitis, Asthma, COPD, Erkrankungen, die mit einer überschießenden Gefäßerweiterung und dadurch bedingter verringerter Gewebedurchblutung einhergehen, z.B. Schock und Sepsis, chronischer Schmerzerkrankungen, wie z.B. diabetische Neuropathien, durch Chemotherapien induzierter Neuropathien, HIV-induzierter Neuropathien, postherpetischer Neuropathien durch Gewebetrauma induzierter Neuropathien, trigeminaler Neuralgien, temporomandibulärer Dysfunktionen, CRPS (complex regional pain syndrome), Rückenschmerzen, und viszeraler Erkrankungen, wie z.B. irritable bowel syndrome (IBS), inflammatory bowel syndrome, zur lindernden Wirkung auf Schmerzzustände im allgemeinen oder zur präventiven oder akut-therapeutischen Behandlung der Symptomatik menopausaler, durch Gefäßerweiterung und erhöhten Blutfluss verursachter Hitzewallungen östrogendefizienter Frauen sowie hormonbehandelter Prostatakarzinompatienten und Kastraten.

11. Verwendung einer Verbindung nach einem der Ansprüche 1 bis 7 zur Herstellung eines Arzneimittels zur akuten und prophylaktischen Behandlung von Migräne- und Cluster-Kopfschmerz, zur Behandlung des irritable bowel syndroms (IBS) und zur präventiven und akut-therapeutischen Behandlung von Hitzewallungen östrogendefizienter Frauen.

12. Verfahren zur Herstellung eines Arzneimittels gemäß Anspruch 8, **dadurch gekennzeichnet, dass** auf nichtchemischem Weg eine Verbindung nach einem der Ansprüche 1 bis 7 in einen oder mehrere inerte Trägerstoffe und/oder Verdünnungsmittel eingearbeitet wird.

## Claims

1. Compounds of general formula I 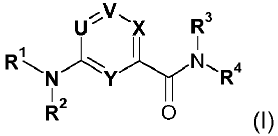 wherein
**R¹** denotes a group of general formulae **IIa** or **IIb** 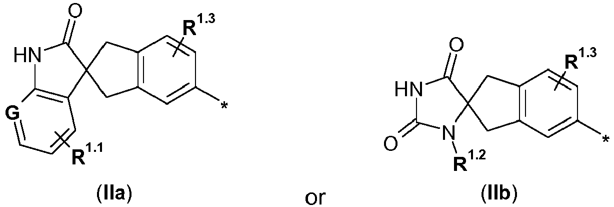 and
**R²** denotes H or C₁₋₃-alkyl,
**G** denotes C-**R^{1.1}** or N,
**R^{1.1}** independently of one another denote
(a) H,
(b) halogen, C₁₋₃-alkyl, -OH, -CN, C₁₋₃-alkyl-O-, -C(O)-O-C₁₋₃-alkyl, -C₂₋₄-alkenyl, -C₂₋₄-alkynyl, C₁₋₃-alkyl-S-, cyclopropyl, -NH₂, -COOH, -NH-C(O)-O-C₁₋₃-alkyl, -NH-C(O)-C₁₋₃-alkyl,
(c) a C₁₋₃-alkyl group or C₁₋₃-alkyl-O-group, wherein each methylene group is substituted by up to two fluorine atoms and each methyl group is substituted by up to three fluorine atoms,
**R^{1.2}** independently of one another denote
(a) H or
(b) C₁₋₃-alkyl,
**R^{1.3}** denotes
(a) H,
(b) F, -CN, C₁₋₃-alkyl, -CO₂-**R^{1.3.1}** or
(c) a C₁₋₃-alkyl group, wherein each methylene group may be substituted by up to two fluorine atoms and each methyl group may be substituted by up to three fluorine atoms,
**R^{1.3.1}** denotes
(a) H,
(b) C₁₋₆-alkyl,
**R³** denotes
(a) H,
(b) C₁₋₃-alkyl or
(c) a C₁₋₃-alkyl group, wherein each methylene group is substituted by up to two fluorine atoms and each methyl group is substituted by up to three fluorine atoms, and
**R⁴** denotes H, cyclopropyl or a group selected from 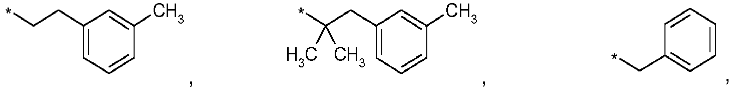 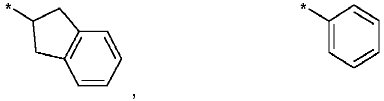 or
**R³** and **R⁴** together with the nitrogen atom to which they are bound denote a group selected from 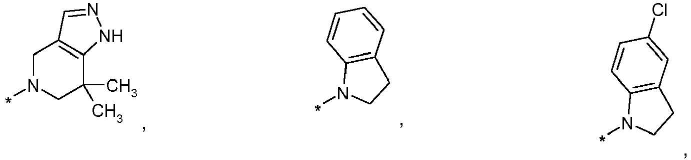 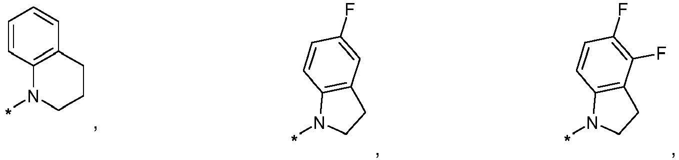 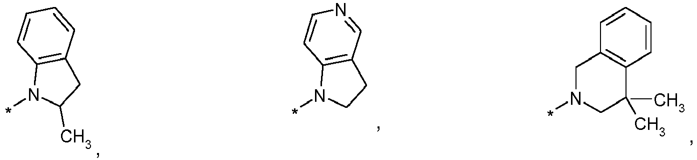 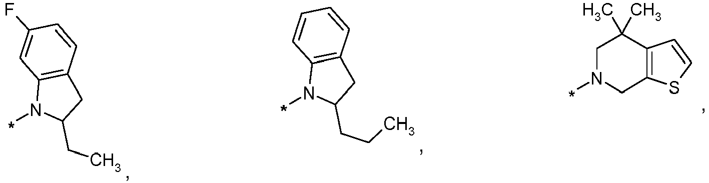 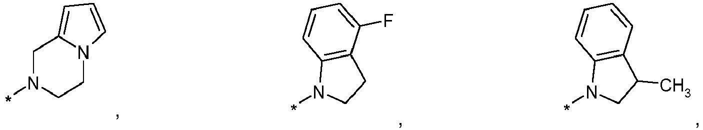 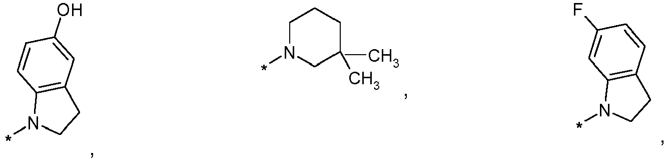 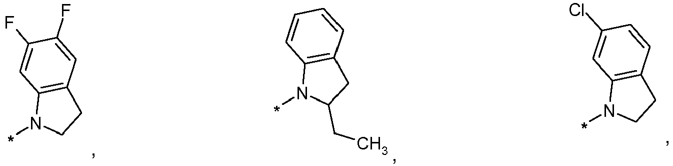 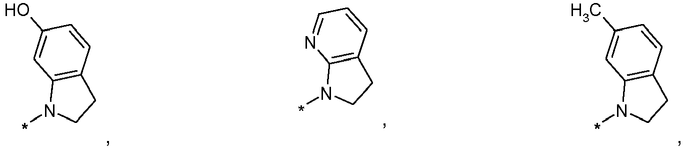 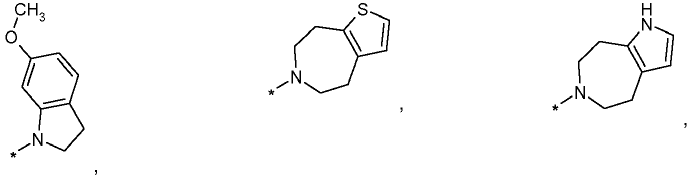 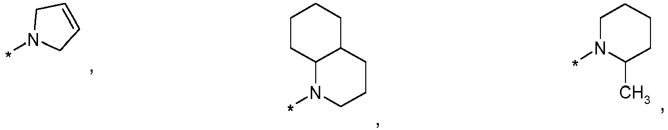 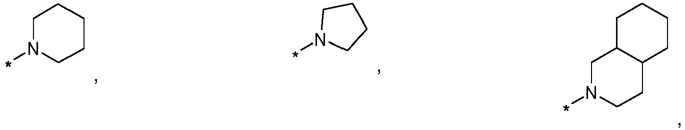 and
the ring 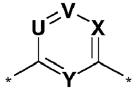 denotes a group selected from 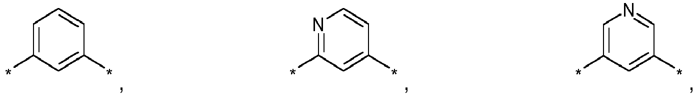 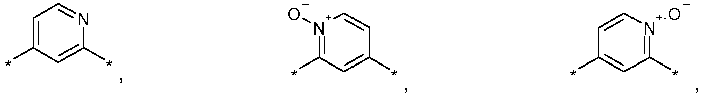 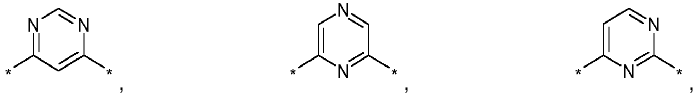 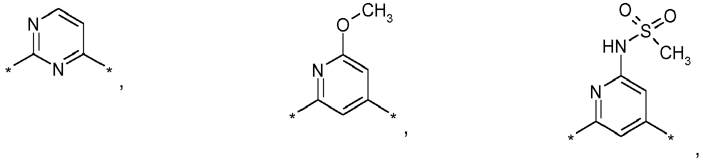 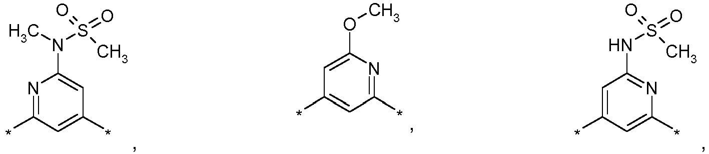 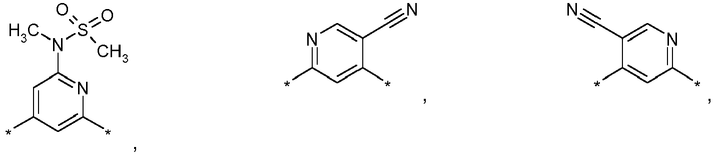 the tautomers, the diastereomers, the enantiomers, the hydrates, the mixtures thereof and the salts thereof and the hydrates of the salts, particularly the physiologically acceptable salts thereof with inorganic or organic acids or bases.

2. Compounds of general formula **I** according to claim 1, wherein **U**, **V**, **X**, **Y**, **R³** and **R⁴** are defined as in claim 1 and
**R¹** denotes a group selected from 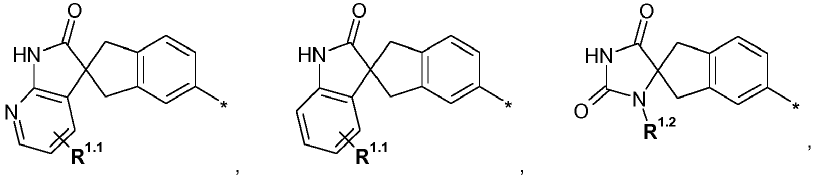
**R^{1.1}** denotes
(a) H,
(b) halogen, C₁₋₃-alkyl, -OH, -CN, -O-C₁₋₃-alkyl, -C(O)-O-C₁₋₃-alkyl, C₂₋₄-alkenyl, C₂₋₄-alkynyl, C₁₋₃-alkyl-S-, -NH₂,
(c) a C₁₋₃-alkyl group or C₁₋₃-alkyl-O-group, wherein each methylene group is substituted by up to two fluorine atoms and each methyl group is substituted by up to three fluorine atoms,
**R^{1.2}** denotes H or CH₃ and
**R²** denotes H or C₁₋₃-alkyl,
the tautomers, the diastereomers, the enantiomers, the hydrates, the mixtures thereof and the salts thereof and the hydrates of the salts, particularly the physiologically acceptable salts thereof with inorganic or organic acids or bases.

3. Compounds of general formula **I** according to claim 1, wherein **U**, **V**, **X**, **Y**, **R³** and **R⁴** are defined as in claim 1 and
**R¹** denotes a group selected from 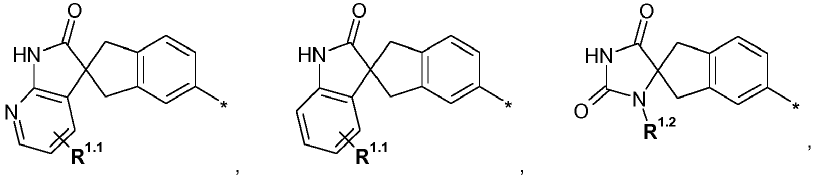
**R^{1.1}** denotes
(a) F, CH₃, -OH, -O-CH₃ or
(b) CF₃ and
**R²** denotes H or C₁₋₃-alkyl,
the tautomers, the diastereomers, the enantiomers, the hydrates, the mixtures thereof and the salts thereof as well as the hydrates of the salts, particularly the physiologically acceptable salts thereof with inorganic or organic acids or bases.

4. Compounds of general formula **I** according to claim 1, wherein **U**, **V**, **X**, **Y**, **R³** and **R⁴** are defined as in claim 1 and
**R¹** denotes a group selected from 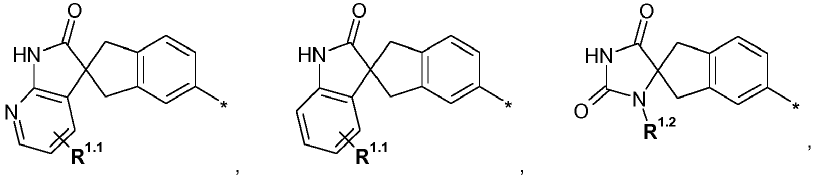 and
R² denotes H,
the tautomers, the diastereomers, the enantiomers, the hydrates, the mixtures thereof and the salts thereof as well as the hydrates of the salts, particularly the physiologically acceptable salts thereof with inorganic or organic acids or bases.

5. Compounds of general formula **I** according to claim 1, wherein
**R¹** denotes a group selected from 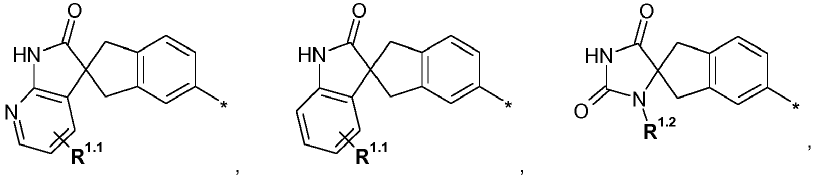
R² denotes H,
**R³** denotes
(a) H,
(b) C₁₋₃-alkyl or
(c) a C₁₋₃-alkyl group, wherein each methylene group is substituted by up to two fluorine atoms and each methyl group is substituted by up to three fluorine atoms, and
**R⁴** denotes H, cyclopropyl or a group selected from 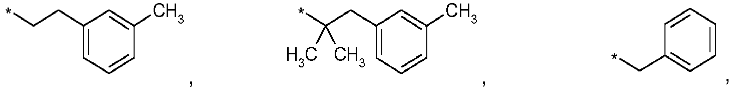 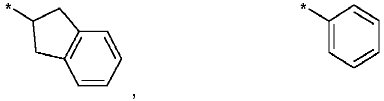 or
**R³** and **R⁴** together with the nitrogen atom to which they are attached denote a group selected from 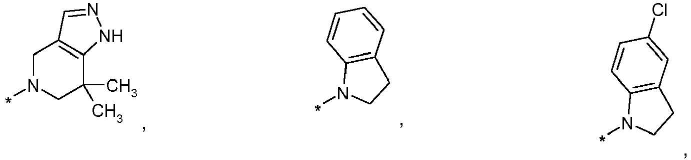 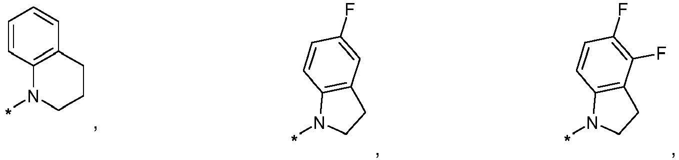 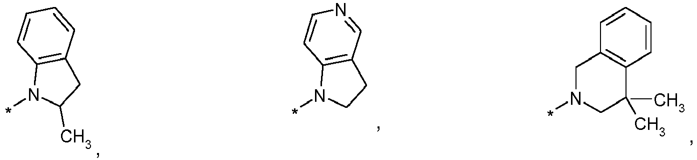 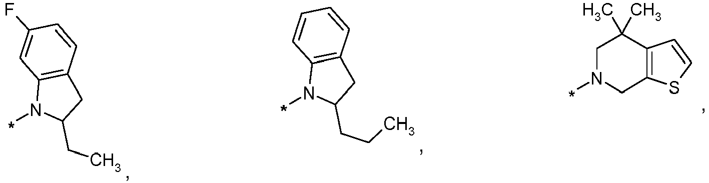 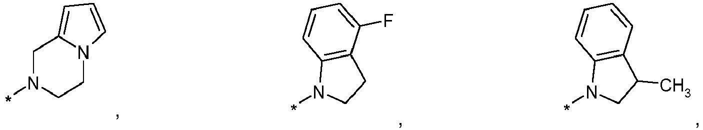 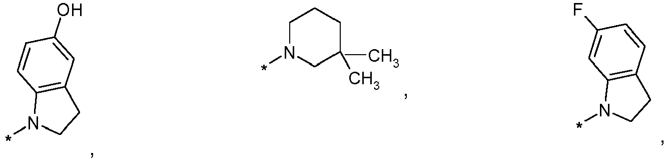 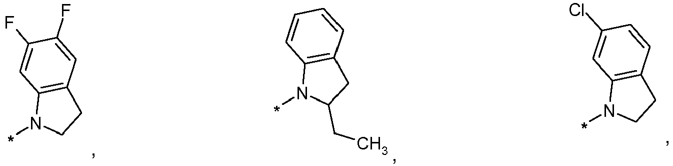 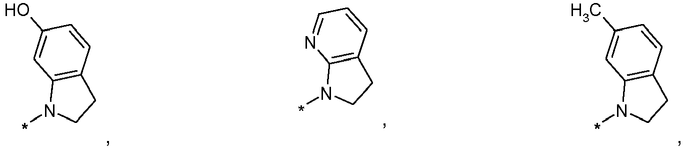 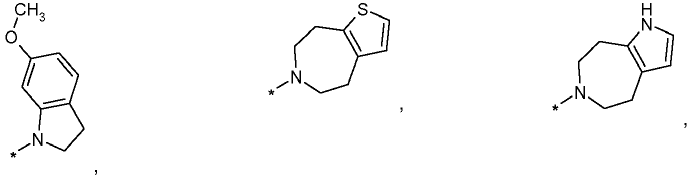 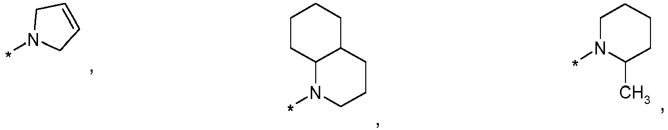 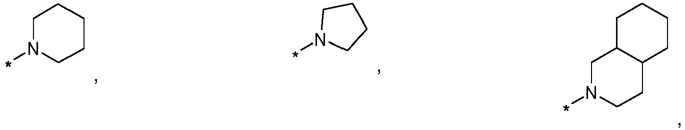 and the ring 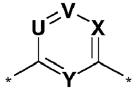 denotes a group selected from 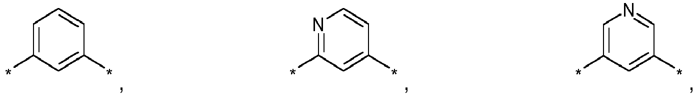 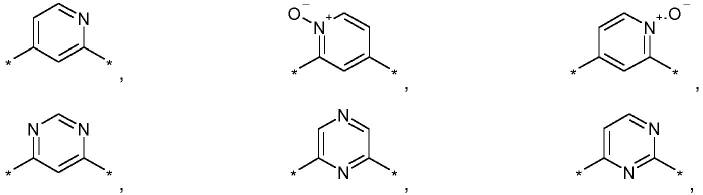 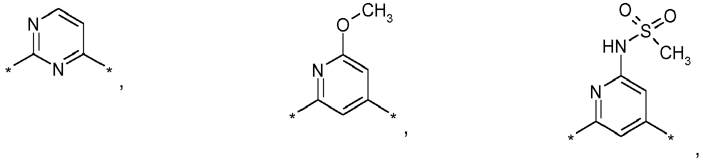 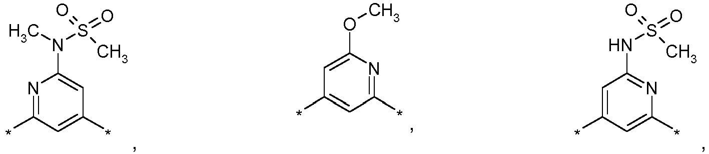 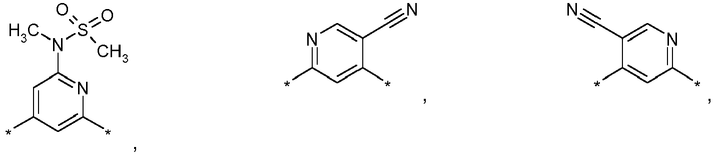 the tautomers, the diastereomers, the enantiomers, the hydrates, the mixtures thereof and the salts thereof as well as the hydrates of the salts, particularly the physiologically acceptable salts thereof with inorganic or organic acids or bases.

6. The following compounds of general formula **I** according to claim 1 :
| **No.** | **Structure** |
|---|---|
| (1) | 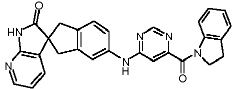 |
| (2) | 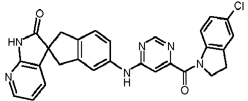 |
| (3) | 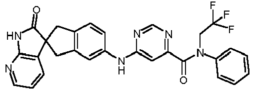 |
| (4) | 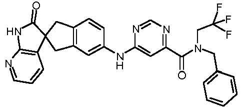 |
| (5) | 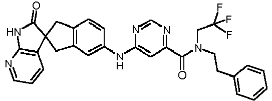 |
| (6) | 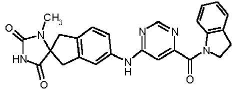 |
| (7) | 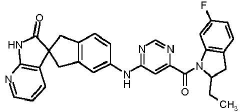 |
| (8) | 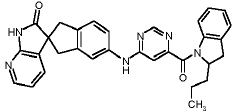 |
| (9) | 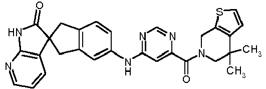 |
| (10) | 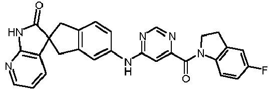 |
| (11) | 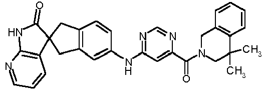 |
| (12) | 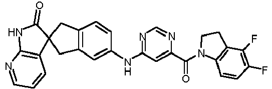 |
| (13) | 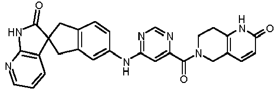 |
| (14) | 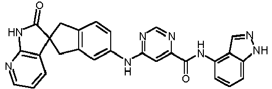 |
| (15) | 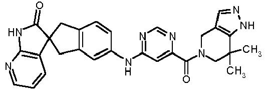 |
| (16) | 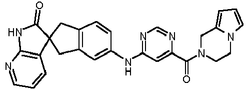 |
| (17) | 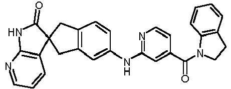 |
| (18) | 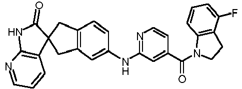 |
| (19) | 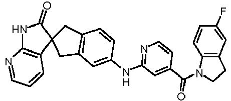 |
| (20) | 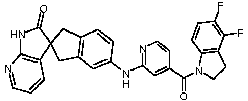 |
| (21) | 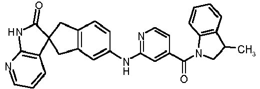 |
| (22) | 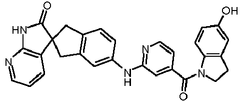 |
| (23) | 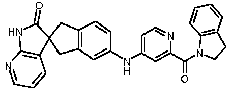 |
| (24) | 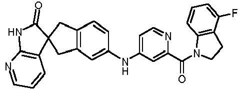 |
| (25) | 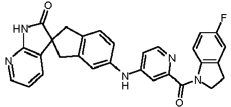 |
| (26) | 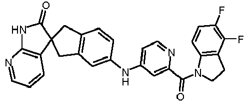 |
| (27) | 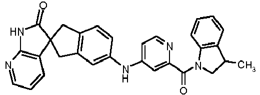 |
| (28) | 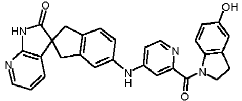 |
| (29) | 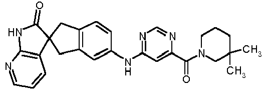 |
| (30) | 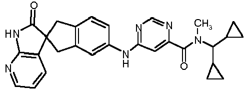 |
| (31) | 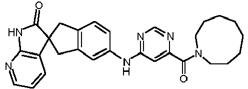 |
| (32) | 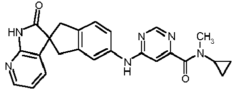 |
| (33) | 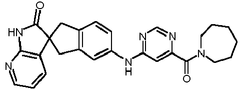 |
| (34) | 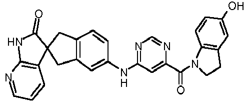 |
| (35) | 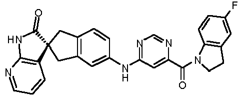 |
| (36) | 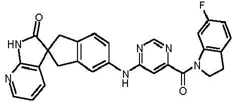 |
| (37) | 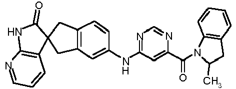 |
| (38) | 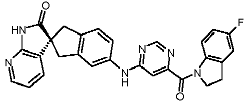 |
| (39) | 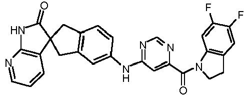 |
| (40) | 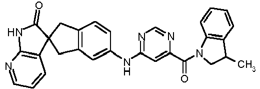 |
| (41) | 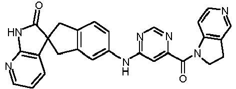 |
| (42) | 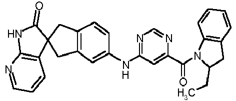 |
| (43) | 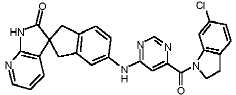 |
| (44) | 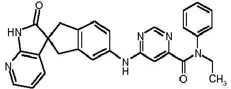 |
| (45) | 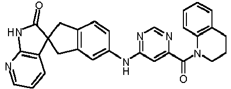 |
| (46) | 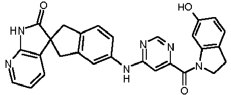 |
| (47) | 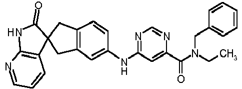 |
| (48) | 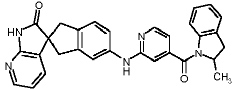 |
| (49) | 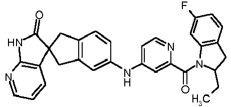 |
| (50) | 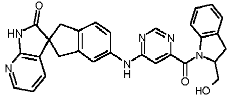 |
| (51) | 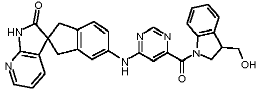 |
| (52) | 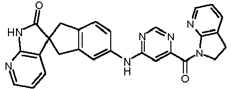 |
| (53) | 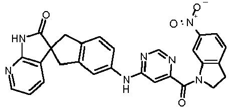 |
| (54) | 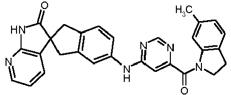 |
| (55) | 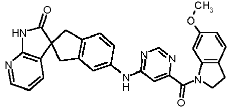 |
| (56) | 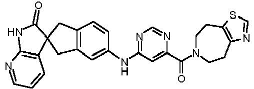 |
| (57) | 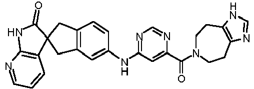 |
| (58) | 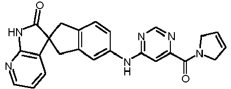 |
| (59) | 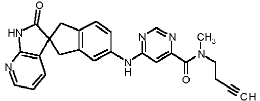 |
| (60) | 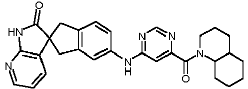 |
| (61) | 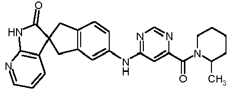 |
| (62) | 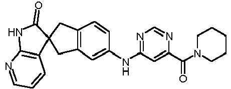 |
| (63) | 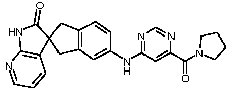 |
| (64) | 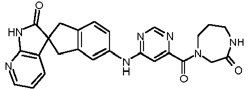 |
| (65) | 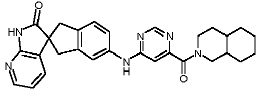 |
the enantiomers, the diastereomers, the hydrates, the mixtures thereof and the salts thereof and the hydrates of the salts, particularly the physiologically acceptable salts thereof with inorganic or organic acids or bases.

7. Physiologically acceptable salts of the compounds according to one of claims 1 to 6 with inorganic or organic acids or bases.

8. Medicaments, containing a compound according to one of claims 1 to 6 or a physiologically acceptable salt according to claim 7 optionally together with one or more inert carriers and/or diluents.

9. Use of a compound according to one of claims 1 to 7 for preparing a medicament for the acute and prophylactic treatment of headaches, particularly migraine and cluster headaches and tension headaches.

10. Use of a compound according to one of claims 1 to 7 for preparing a medicament for treating non-insulin-dependent diabetes mellitus ("NIDDM"), cardiovascular diseases, morphine tolerance, diarrhoea caused by clostridium toxin, skin diseases, particularly thermal and radiation-induced skin damage including sunburn, lichen, pruritis, pruritic toxidermies and severe itching, inflammatory diseases, e.g. inflammatory diseases of the joints (osteoarthritis, rheumatoid arthritis, neurogenic arthritis), generalised soft-tissue rheumatism (fibromyalgia), neurogenic inflammation of the oral mucosa, inflammatory lung diseases, allergic rhinitis, asthma, COPD, diseases accompanied by excessive vasodilatation and resultant reduced blood supply to the tissues, e.g. shock and sepsis, chronic pain, e.g. diabetic neuropathies, neuropathies induced by chemotherapy, HIV-induced neuropathies, postherpetic neuropathies, neuropathies induced by tissue trauma, trigeminal neuralgias, temporomandibular dysfunctions, CRPS (complex regional pain syndrome), back pain, and visceral complaints, such as e.g. irritable bowel syndrome (IBS), inflammatory bowel syndrome, for relieving pain in general, or for preventive or acute therapeutic treatment of the symptoms of menopausal hot flushes caused by vasodilatation and increased blood flow in oestrogen-deficient women and hormone-treated patients with prostate carcinoma and castrated men.

11. Use of a compound according to one of claims 1 to 7 for preparing a medicament for the acute and prophylactic treatment of migraine and cluster headaches, for the treatment of irritable bowel syndrome (IBS) and for the preventive and acute therapeutic treatment of hot flushes in oestrogen-deficient women.

12. Method of preparing a medicament according to claim 8, **characterised in that** a compound according to one of claims 1 to 7 is incorporated in one or more inert carriers and/or diluents by a non-chemical method.

## Revendications

1. Composés de formule générale I 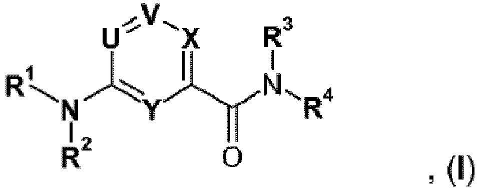 dans laquelle
R¹ représente un groupe de formules générales IIa ou IIb 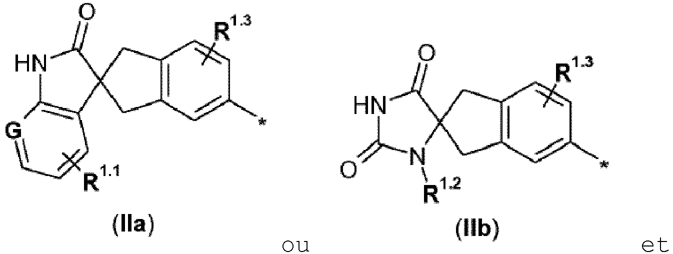
R² représente H ou un groupe C₁₋₃-alkyle,
G représente C-R^{1.1} ou N,
R^{1.1} représente, indépendamment les uns des autres
(a) H,
(b) un atome d'halogène, un groupe C₁₋₃-alkyle, - OH, -CN, C₁₋₃-alkyl-O-, -C(O)-O-C₁₋₃-alkyle, C₂₋₄-alcényle, C₂₋₄-alcinyle, C₁₋₃-alkyl-S-, cyclopropyle, -NH₂, -COOH, -NH-C(O)-O-C-₁₋₃-alkyle, -NH-C(O)-C₁₋₃-alkyle,
(c) un groupe C₁₋₃-alkyle ou C₁₋₃-alkyl-O-, dans lequel chaque groupe méthylène est substitué par jusqu'à deux atomes de fluor et chaque groupe méthyle est substitué par jusqu'à trois atomes de fluor,
R^{1.2} représente, indépendamment les uns des autres
(a) H ou
(b) un groupe C₁₋₃-alkyle,
R^{1.3} représente
(a) H
(b) F, -CN, C₁₋₃-alkyle, -CO₂-R^{1.3.1} ou
(c) un groupe C₁₋₃-alkyle dans lequel chaque groupe méthylène peut être substitué par jusqu'à deux atomes de fluor et chaque groupe méthyle peut être substitué par jusqu'à trois atomes de fluor,
R^{1.3.1} représente
(a) H,
(b) un groupe C₁₋₆-alkyle,
R³ représente
(a) H,
(b) un groupe C₁₋₃-alkyle ou
(c) un groupe C₁₋₃-alkyle dans lequel chaque groupe méthylène est substitué par jusqu'à deux atomes de fluor et chaque groupe méthyle est substitué par jusqu'à trois atomes de fluor, et
R⁴ représente H, un groupe cyclopropyle ou un groupe choisi parmi 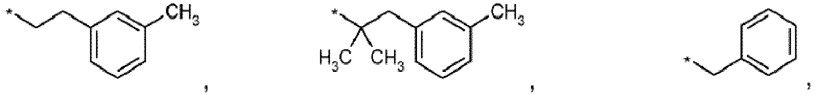 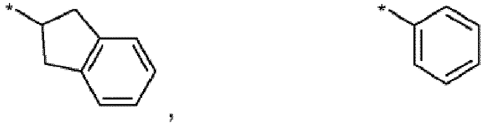 ou
R³ et R⁴, conjointement avec l'atome d'azote auquel ils sont liés, représentent un groupe choisi parmi 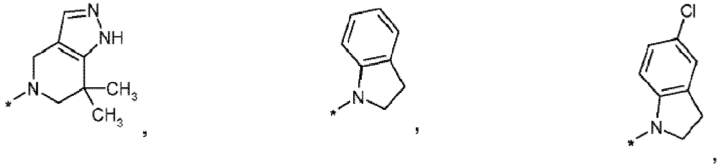 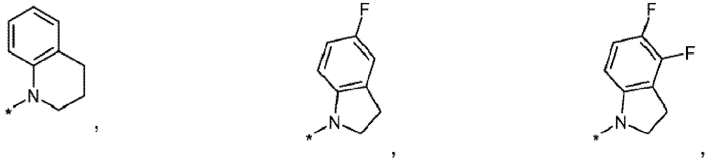 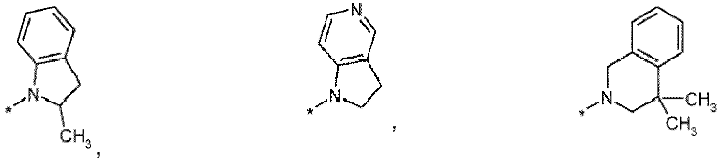 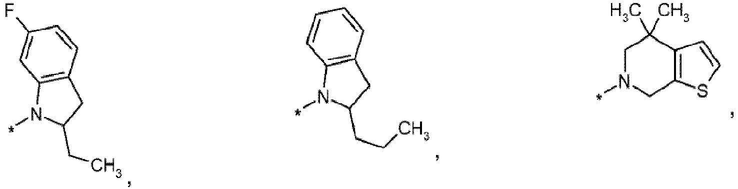 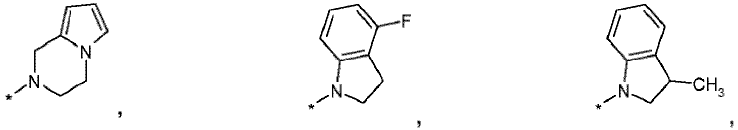 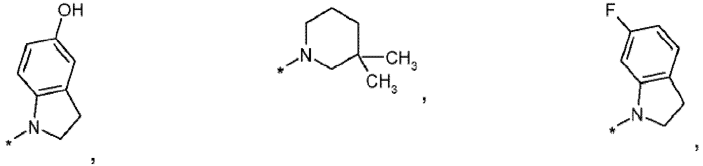 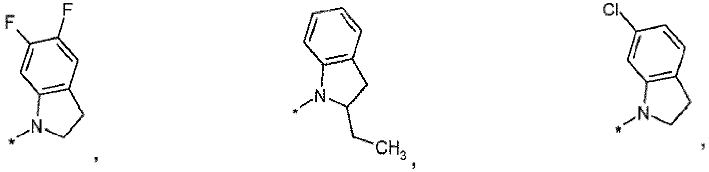 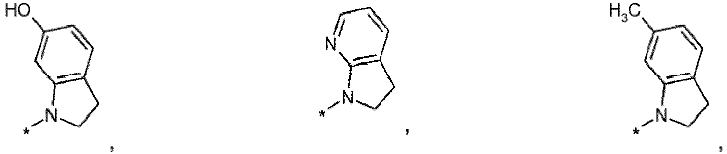 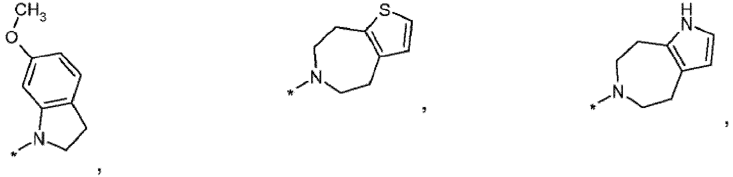 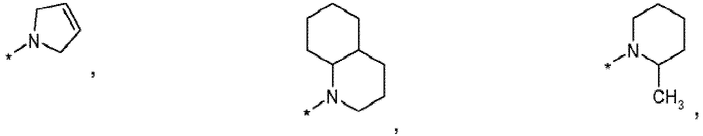 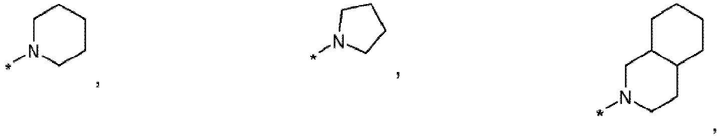 et
le cycle 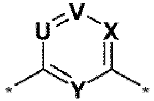 représente un groupe choisi parmi 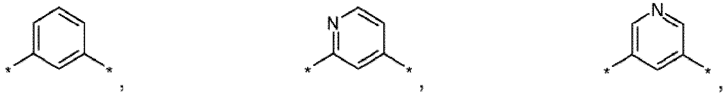 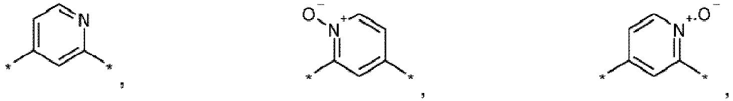 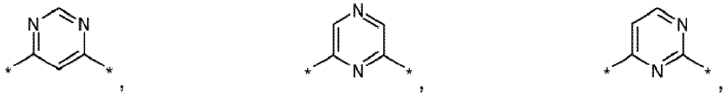 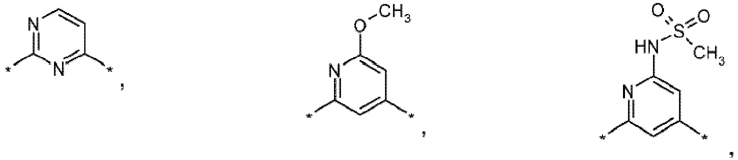 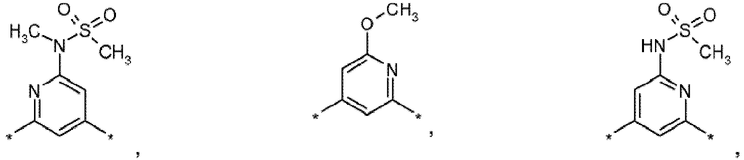 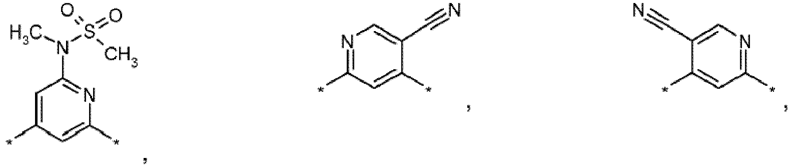 leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

2. Composés de formule générale I selon la revendication 1, dans laquelle U, V, X, Y, R³ et R⁴ sont tels que définis dans la revendication 1 et
R¹ représente un groupe choisi parmi 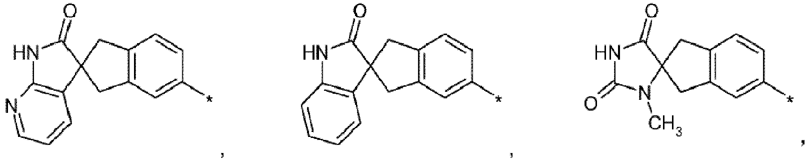
R^{1.1} représente
(a) H,
(b) un atome d'halogène, un groupe C₁₋₃-alkyle, - OH, -CN, -O-C₁₋₃-alkyle, -C(O)-O-C₁₋₃-alkyle, C₂₋₄-alcényle, C₂₋₄-alcinyle, C₁₋₃-alkyl-S-, - NH₂,
(c) un groupe C₁₋₃-alkyle ou un groupe C₁₋₃-alkyl-0-, dans lequel chaque groupe méthylène est substitué par jusqu'à deux atomes de fluor et chaque groupe méthyle est substitué par jusqu'à trois atomes de fluor,
R^{1.2} représente H ou CH₃ et
R² représente H ou C₁₋₃-alkyle,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

3. Composés de formule générale I selon la revendication 1, dans laquelle U, V, X, Y, R³ et R⁴ sont tels que définis dans la revendication 1 et
R¹ représente un groupe choisi parmi 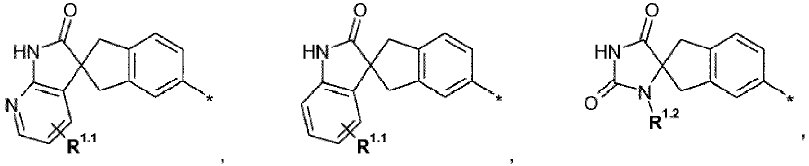
R^{1.1} représente
(a) F, CH₃, -OH, -O-CH₃ ou
(b) CF₃ et
R² représente
H ou un groupe C₁₋₃-alkyle,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

4. Composés de formule générale I selon la revendication 1, dans laquelle U, V, X, Y, R³ et R⁴ sont tels que définis dans la revendication 1 et
R¹ représente un groupe choisi parmi 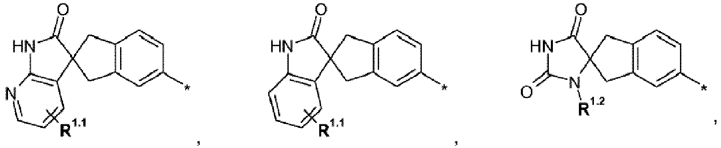 et
R² représente H,
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

5. Composés de formule générale I selon la revendication 1, dans laquelle
R¹ représente un groupe choisi parmi 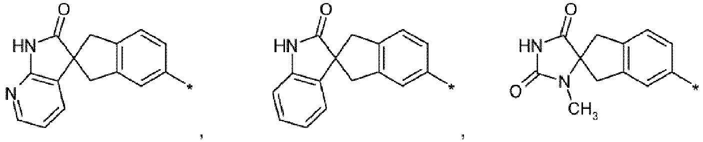 et
R² représente H,
R³ représente
(a) H,
(b) un groupe C₁₋₃-alkyle ou
(c) un groupe C₁₋₃-alkyle dans lequel chaque groupe méthylène est substitué par jusqu'à deux atomes de fluor et chaque groupe méthyle est substitué par jusqu'à trois atomes de fluor, et
R⁴ représente H, un groupe cyclopropyle ou un groupe choisi parmi 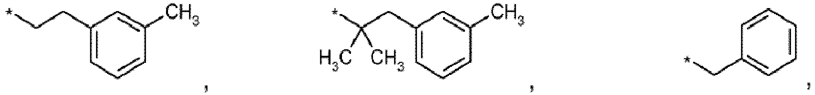 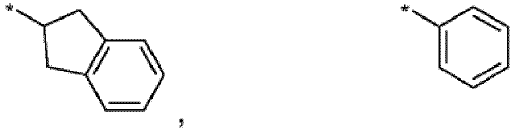 ou
R³ et R⁴ représentent, conjointement avec l'atome d'azote auquel ils sont liés, un groupe choisi parmi 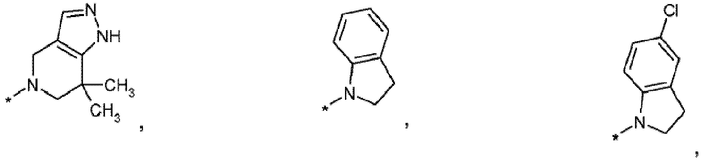 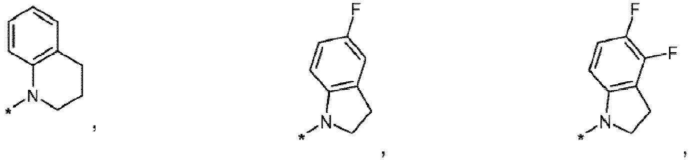 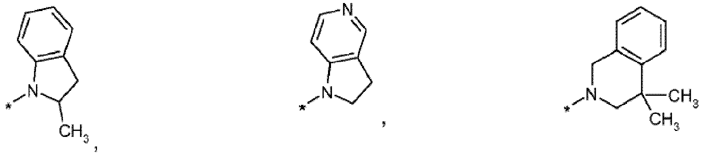 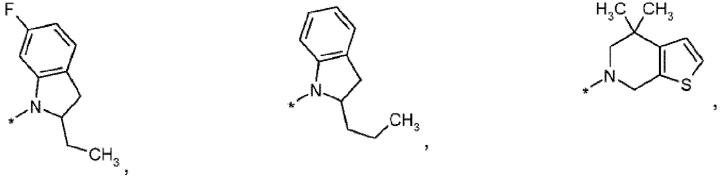 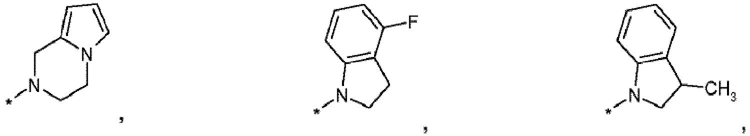 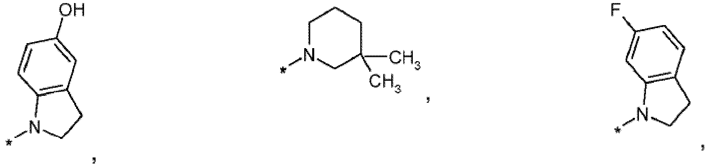 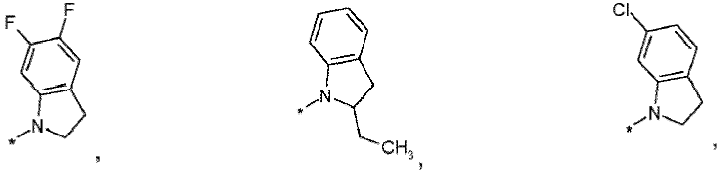 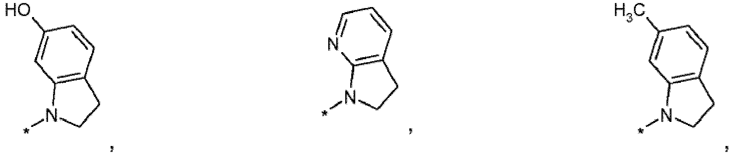 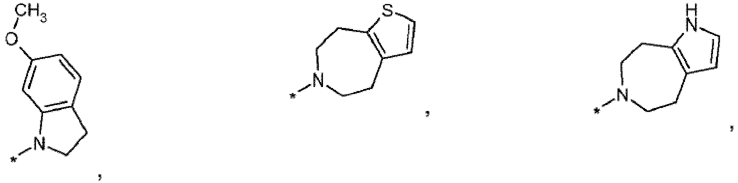 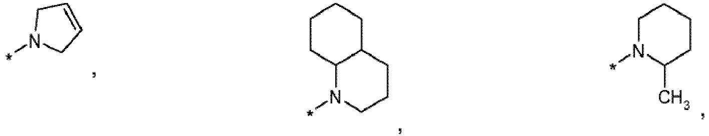 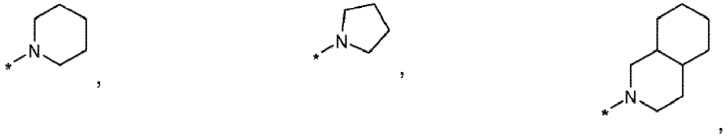 et le cycle 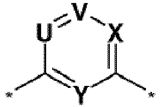 représente un groupe choisi parmi 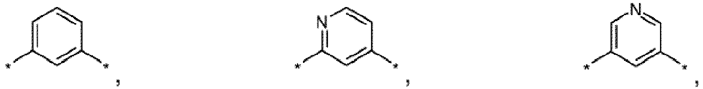 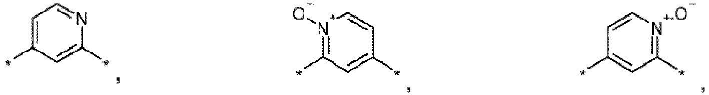 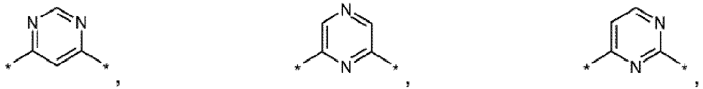 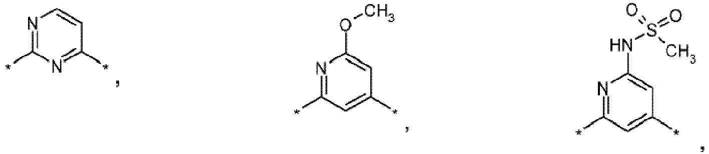 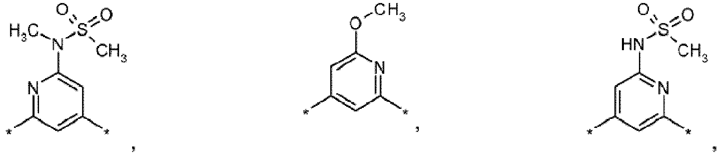 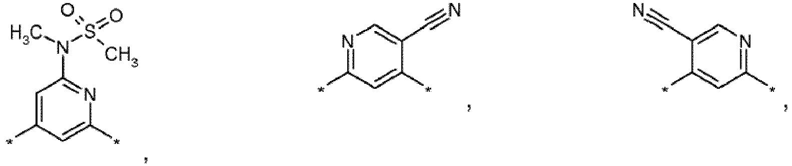 leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

6. Composés suivants de formule générale I selon la revendication 1 :
| N° | Structure |
|---|---|
| (1) | 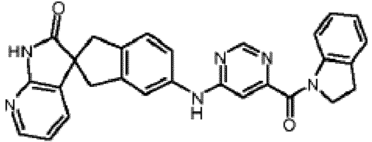 |
| (2) | 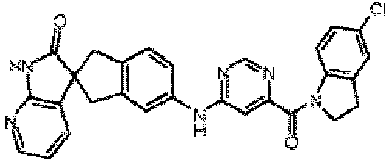 |
| (3) | 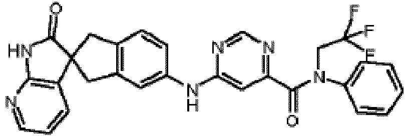 |
| (4) | 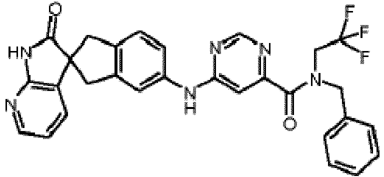 |
| (5) | 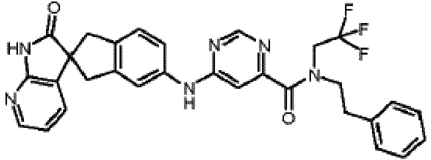 |
| (6) | 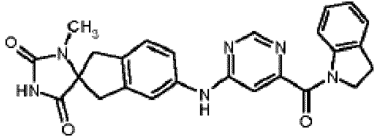 |
| (7) | 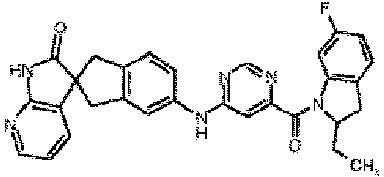 |
| (8) | 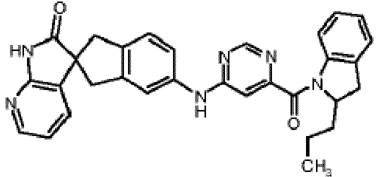 |
| (9) | 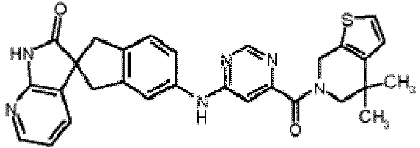 |
| (10) | 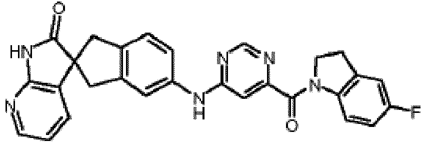 |
| (11) | 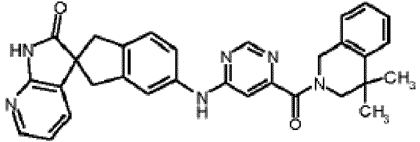 |
| (12) | 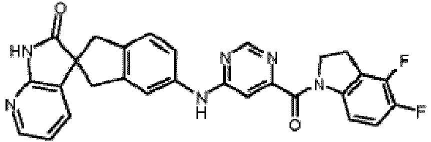 |
| (13) | 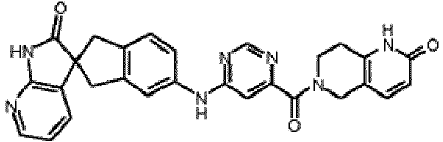 |
| (14) | 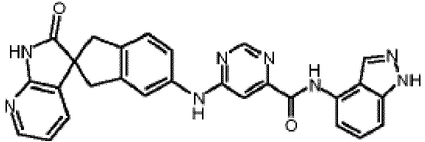 |
| (15) | 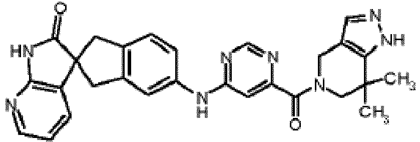 |
| (16) | 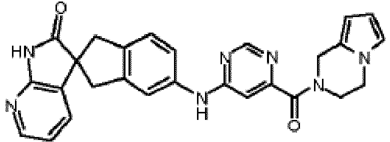 |
| (17) | 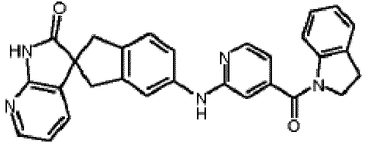 |
| (18) | 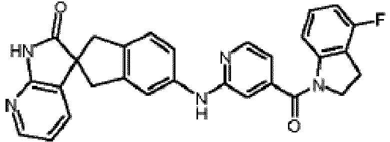 |
| (19) | 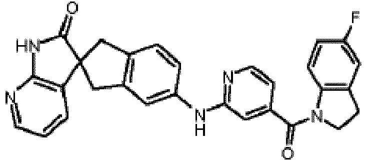 |
| (20) | 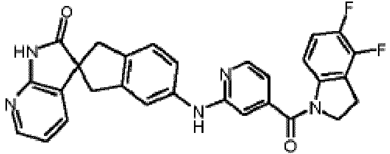 |
| (21) | 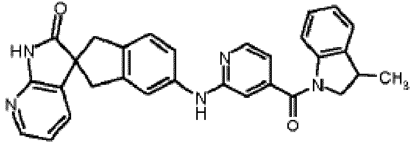 |
| (22) | 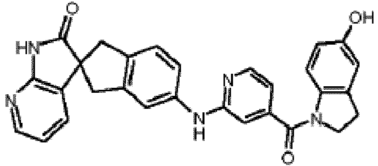 |
| (23) | 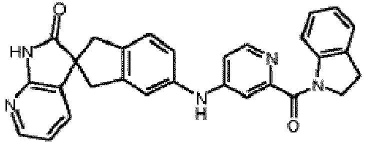 |
| (24) | 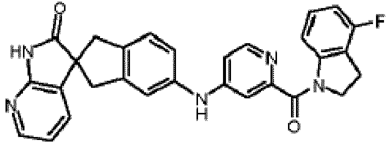 |
| (25) | 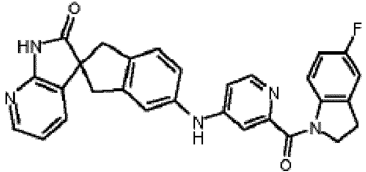 |
| (26) | 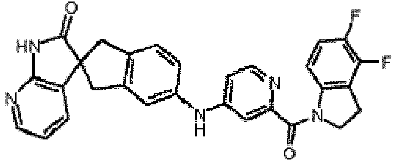 |
| (27) | 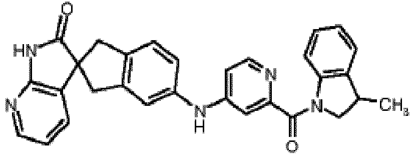 |
| (28) | 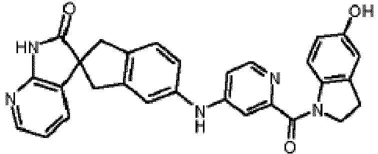 |
| (29) | 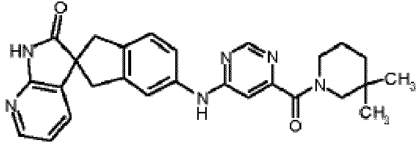 |
| (30) | 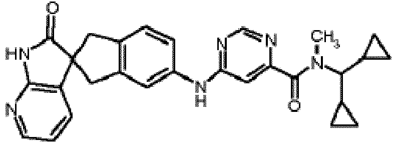 |
| (31) | 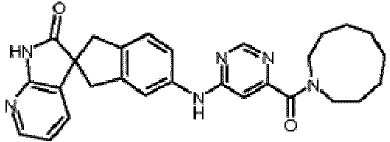 |
| (32) | 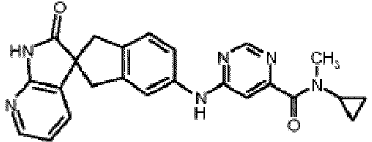 |
| (33) | 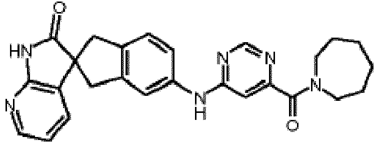 |
| (34) | 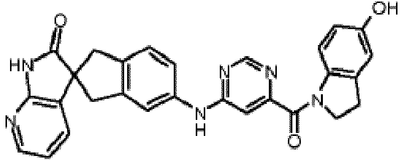 |
| (35) | 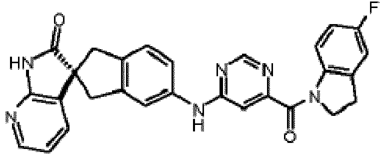 |
| (36) | 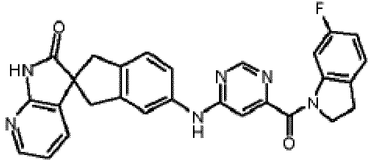 |
| (37) | 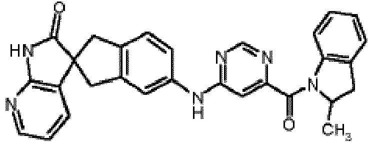 |
| (38) | 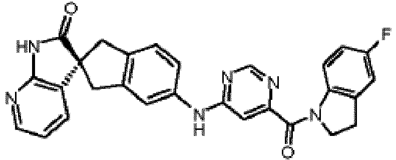 |
| (39) | 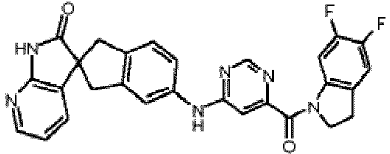 |
| (40) | 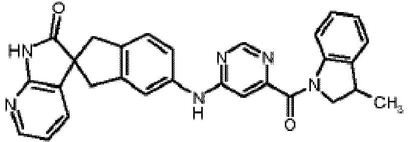 |
| (41) | 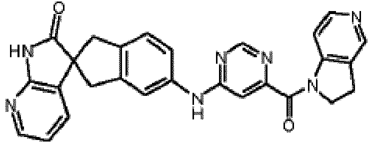 |
| (42) | 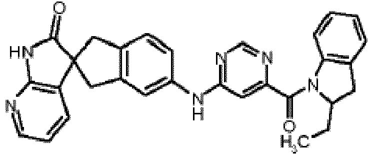 |
| (43) | 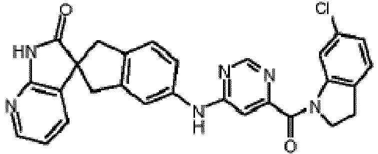 |
| (44) | 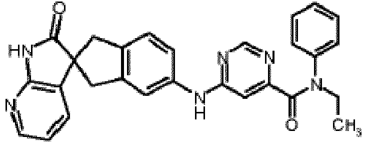 |
| (45) | 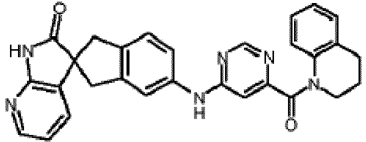 |
| (46) | 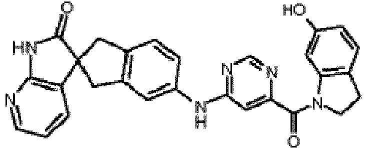 |
| (47) | 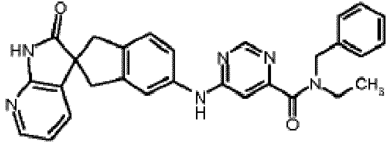 |
| (48) | 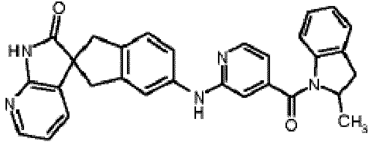 |
| (49) | 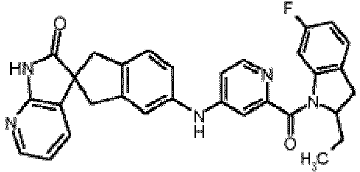 |
| (50) | 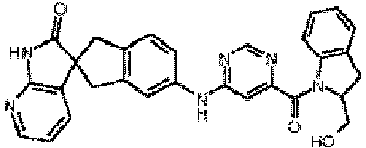 |
| (51) | 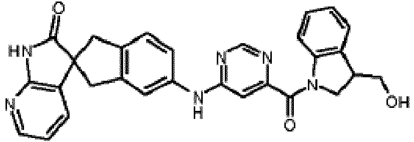 |
| (52) | 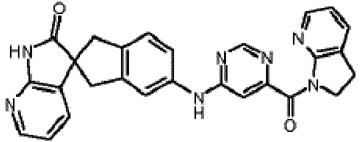 |
| (53) | 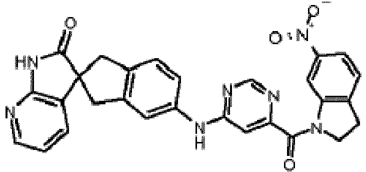 |
| (54) | 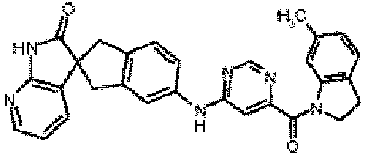 |
| (55) | 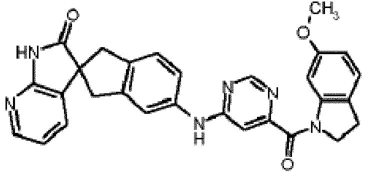 |
| (56) | 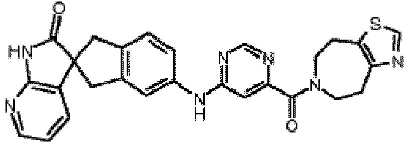 |
| (57) | 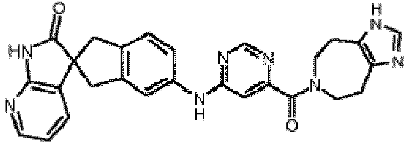 |
| (58) | 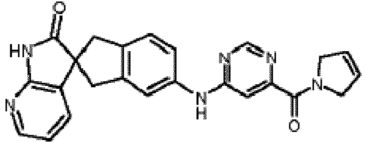 |
| (59) | 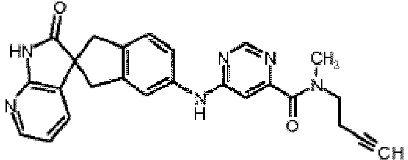 |
| (60) | 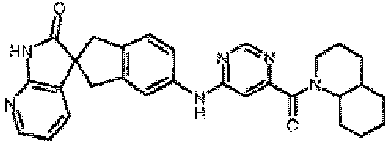 |
| (61) | 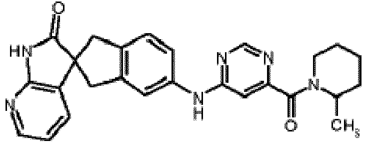 |
| (62) | 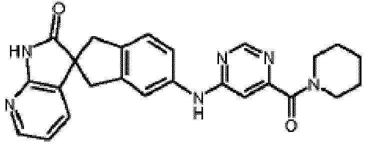 |
| (63) | 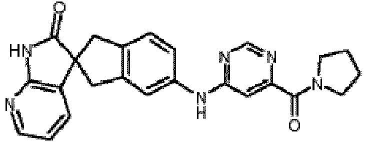 |
| (64) | 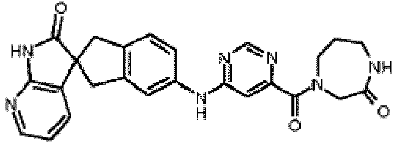 |
| (65) | 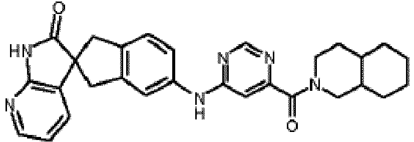 |
leurs tautomères, leurs diastéréomères, leurs énantiomères, leurs hydrates, leurs mélanges et leurs sels et les hydrates des sels, en particulier leurs sels physiologiquement acceptables avec des acides ou des bases inorganiques ou organiques.

7. Sels physiologiquement acceptables des composés selon l'une des revendications 1 à 6, avec des acides ou des bases inorganiques ou organiques.

8. Médicament contenant un composé selon l'une des revendications 1 à 6 ou un sel physiologiquement acceptable de celui-ci selon la revendication 7, outre éventuellement un ou plusieurs véhicules et/ou diluants inertes.

9. Utilisation d'un composé selon l'une des revendications 1 à 7, pour la production d'un médicament pour le traitement aigu et prophylactique des céphalées, en particulier des migraines et de l'algie vasculaire de la face et des céphalées de tension.

10. Utilisation d'un composé selon l'une des revendications 1 à 7, pour la production d'un médicament destiné au traitement du diabète sucré non insulino-dépendant (« NIDDM »), de maladies cardiovasculaires, de la tolérance à la morphine, des diarrhées dues à *Clostridium,* des maladies de la peau, en particulier, des lésions cutanées thermiques et dues à un rayonnement comprenant les coups de soleil, le lichen, le prurit, les toxidermies prurigineuses et les démangeaisons sévères, des maladies inflammatoires, par exemple, des affections articulaires inflammatoires (arthrose, arthrite rhumatoïde, arthrite neurogène), des rhumatismes généralisés des tissus mous (fibromyalgie), des inflammations neurogènes de la muqueuse buccale, des maladies pulmonaires inflammatoires, la rhinite allergique, l'asthme, la BPCO, des maladies qui s'accompagnent d'une vasodilatation excessive et ainsi, d'une réduction de l'irrigation tissulaire, par exemple choc et septicémie, des maladies douloureuses chroniques telles que par exemple, les neuropathies diabétiques, les neuropathies induites par des chimiothérapies, des neuropathies induites par le VIH, des neuropathies post-herpétiques, des neuropathies induites par un traumatisme tissulaire, des névralgies du trijumeau, des dysfonctionnements temporo-mandibulaires, du CRPS (syndrome de douleur régionale complexe), des douleurs dorsales et des douleurs viscérales, par exemple le syndrome du côlon irritable (SCI), le syndrome intestinal inflammatoire, à titre d'effet calmant des états douloureux en traitement général ou pour la prévention ou le traitement thérapeutique aigu des symptômes de bouffées de chaleur, induites par une vasodilatation et une augmentation du débit sanguin, chez les femmes ménopausées présentant un déficit en oestrogène et les patients souffrant d'un cancer de la prostate ou castrés traités par hormonothérapie.

11. Utilisation d'un composé selon l'une des revendications 1 à 7, pour la production d'un médicament pour le traitement aigu et prophylactique de la migraine et de l'algie vasculaire de la face, pour le traitement du côlon irritable (SCI) et pour le traitement préventif et thérapeutique aigu des bouffées de chaleur des femmes présentant un déficit en oestrogènes.

12. Procédé de production d'un médicament selon la revendication 8, **caractérisé en ce que**, de façon non chimique, un composé selon l'une des revendications 1 à 7, est incorporé dans un ou plusieurs véhicules et/ou diluants inertes.
